# EUROPEAN PATENT APPLICATION

(11) **EP 4 545 138 A1**
(43) Date of publication of application: **30.04.2025**
(21) Application number: 23205641.6
(22) Date of filing: 24.10.2023
(51) Int. Cl.: A61P 17/00, A61P 29/00, C07D 209/44, C07D 403/10, C07D 403/14, C07D 413/14, C07D 417/14, C07D 471/10, C07D 487/10, A61K 31/4045

(54) **HETEROCYCLIC COMPOUNDS AS BLT2 AGONISTS AND THEIR MEDICAL USE**

(71) Applicant: Fraunhofer-Gesellschaft zur Förderung der angewandten Forschung e.V., 80686 München (DE); Johann-Wolfgang-Goethe-Universität Frankfurt am Main, 60323 Frankfurt am Main (DE)
(72) Inventor: Hernandez-Olmos, Victor, 80686 München (DE); Heering, Jan, 80686 München (DE); Proschak, Eugen, 80686 München (DE); Steinhilber, Dieter, 80686 München (DE); Weigert, Andreas, 60323 Frankfurt am Main (DE)
(74) Representative: Kuttenkeuler, David

(57) **Abstract**

Herein, compounds of Formulae (I-a) and (II) that act as selective agonists of the leukotriene B4 receptor 2 (BLT2) receptor are presented. The invention pertains to these compounds, their synthesis, as well as their medical use, amongst others in the treatment of pain and inflammatory skin diseases such as psoriasis.

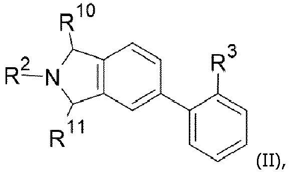

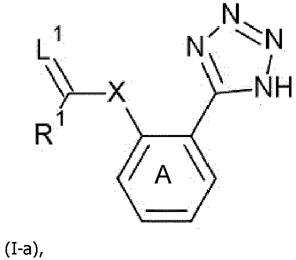

## Description

### FIELD OF THE INVENTION

Herein, novel compounds that act as selective agonists of the leukotriene B4 receptor 2 (BLT2) receptor are presented. The invention pertains to these compounds, their synthesis, as well as their medical use, amongst others in the treatment of pain and inflammatory skin diseases such as psoriasis.

### DESCRIPTION

An estimated 20-25% of the population is affected by chronic, non-communicable inflammatory skin diseases. These are usually treated with anti-inflammatory and immune-suppressive drugs. However, such a treatment is accompanied by adverse side effects and a slow-down in wound-healing.

Leukotriene B4 receptor 2 (BLT2) is a G-protein coupled receptor (GPCR) that is currently gaining increasing attention as a potential therapeutic target. It is involved in various pathologies including diabetic wound healing, ophthalmic diseases, and colitis. Notably, BLT2 activation was first associated with a reduction of neuropathic pain. Potential therapeutic applications of BLT2 activation additionally encompass pathologies of impaired wound closure like diabetic wound healing, corneal wound healing, and healing of intestinal lesions.

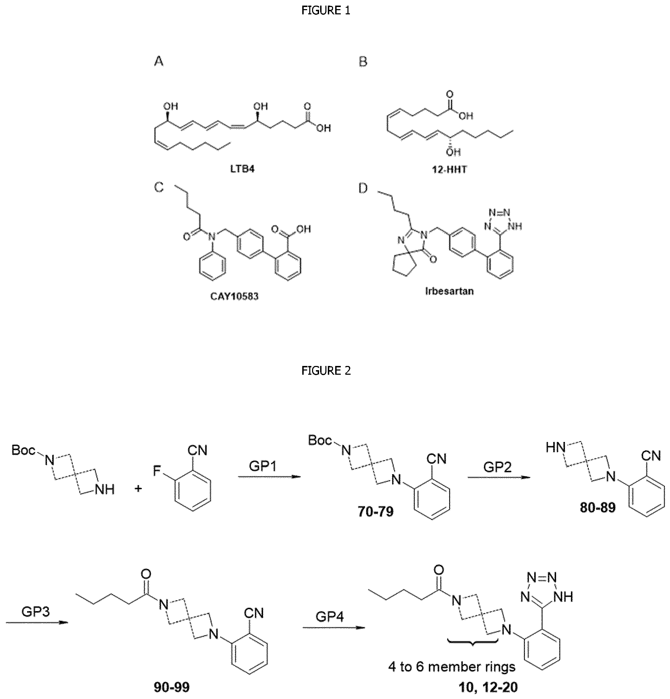

BLT2, which was initially described by Yokomizo et al. in 2000¹, is structurally related to the leukotriene B4 type 1 receptor (BLT1), however both GPCR receptors differ in their affinity and specificity for leukotriene B4 **(****FIG. 1A****,** LTB4). BLT1 is the high affinity receptor for LTB4 (pKd = 8.5) and is mainly found in human leukocytes. BLT2 displays lower affinity for LTB4 (pKd = 7.64) and is expressed ubiquitously in humans, with highest mRNA expression levels in the spleen, liver, ovaries, and leucocytes. Furthermore, BLT2 is in particular expressed in skin fibroblasts.

BLT2 is a G-protein coupled receptor, which is endogenously activated by 12(*S*)-hydroxyheptadeca-5*Z*,8*E*,10*E-*trienoic acid **(****FIG. 1B****,** 12-HHT). BLT2 modulation constitutes an attractive strategy for the development of new therapeutics. The first synthetic potent and selective BLT2 agonistCAY10583 (4'-((N-phenylpentanamido)methyl)-[1,1'-biphenyl]-2-carboxylic acid, also referred to as CAY, FIG. 1C) was described by Iizuka et al. ² Furthermore, in a previous study the inventors have discovered Irbesartan (FIG. 1D) and derivatives as BLT2 agonists³.

The usage of selective BLT2 agonists seems is a potent new strategy to improve healing of wounds in inflammatory skin diseases, however the selection of drugs which allows for its implementation *in vivo* is still limited. There is still a need for new lead structures which exhibit high potency and a favourable pharmacokinetics (PK) profile. Thus, it is an object of the invention to provide novel compounds that expand and improve the pool of available BLT2 agonists.

The inventors designed and synthesized a series of structurally related compounds based on two new potent scaffolds for BLT2 agonism. These feature an excellent solubility, metabolic stability, PK profile and can therefore serve as valuable therapeutics for the treatment of diseases associated with BLT2 in vivo. In particular, the inventors demonstrate this in a mouse model of psoriasis.

### BRIEF DESCRIPTION OF THE INVENTION

Generally, and by way of brief description, the main aspects of the present invention can be described as follows:

In **a first aspect,** the invention pertains to a compound, that comprises the formula (I) 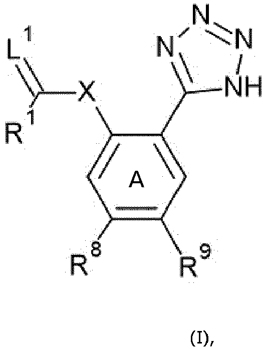
wherein the compound comprises a phenyl group A, wherein the phenyl group A is substituted with a tetrazole group and with a spiro group X,
wherein X comprises two aliphatic heterocycles B¹ and B² that are connected by a carbon spiro atom and each of B¹ and B² comprises a nitrogen heteroatom,
wherein B¹ is connected to A via a nitrogen heteroatom of B¹,
wherein B² is connected to an R¹(C=L¹) group via a nitrogen heteroatom of B²,
wherein the amount of ring atoms m¹ in B¹ is 6 or fewer,
wherein the amount of ring atoms m² in B² is 6 or fewer,
wherein R¹ is a Cₙ alkyl group with n = 1-5, a Cⱼ alkenyl group with j = 2-5, a C_{d} alkyne group with d = 2-5, or a C_{b} cycloalkyl group with b = 3-5, wherein optionally a hydrogen atom in R¹ is replaced with a fluor atom, a deuterium atom or a trifluormethyl group,
wherein L¹ is =O, =S, =C(CN)₂, =N-OH, or =N-OMe,
wherein R⁸ is selected from the group of -H, -Cl, -Br, -CH₃₋ᵣFᵣ, and-OCH₃, wherein r = 0-3.
wherein R⁹ is selected from -H, -halogen and -CH₃₋ᵥFᵥ, wherein v = 0-3,
or a tautomer, isomer, diastereomer, enantiomer, hydrate, mixture or salt thereof.

**In a second aspect, the invention pertains to a compound** that comprises a structure with the formula (II) 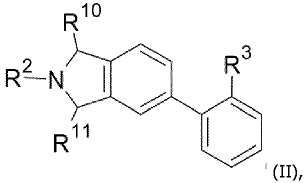
wherein R³ is a 1H-Tetrazol-5-yl group 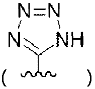 or a carboxyl group,
wherein R¹⁰ and R¹¹ are independently selected from -H, phenyl and a C₁₋₃ alkyl, preferably wherein at least one of R¹⁰ and R¹¹ is -H,
wherein R² is R⁴-(C=L²)-, wherein R⁴ is a Cₚ alkyl group with p = 1-5, a Cₒ alkenyl group with o = 2-5, or a Cₜ alkyne group with t = 2-5, wherein optionally R⁴ is substituted with a substituent selected from fluor and R⁵,
wherein R⁵ is a methyl group, ethyl group or phenyl group,
wherein L² is =O, =S, =C(CN)₂, =N-OH, =N-OMe, preferably =O or =S,
or wherein R² is a 5-membered heteroaryl with at least one nitrogen heteroatom, wherein the 5-membered heteroaryl with at least one nitrogen heteroatom comprises one substituent R⁶ and/or one substituent R⁷,
wherein R⁶ and R⁷ are independently selected from -H and a C_{q} alkyl-group with q = 1 to 3, or wherein R⁶ and R⁷ form a 6-membered ring together with the atoms that they are attached to that comprises at least 5 carbon ring atoms,
or a tautomer, isomer, diastereomer, enantiomer, hydrate, mixture or salt thereof.

**In a third aspect, the invention pertains to a method of synthesizing a compound** according to the first aspect of the invention.

**In a fourth aspect, the invention pertains to an intermediate,** wherein the intermediate is selected from

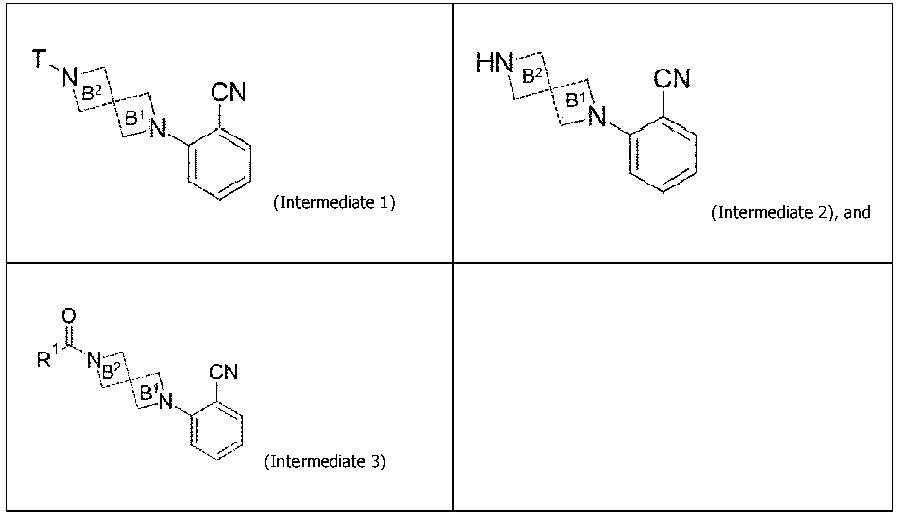

wherein, 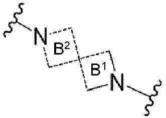 refers to a spirocyclic group that comprises two rings, namely B² and B¹, as defined according to the first aspect of the invention, R¹ is defined according to the first aspect of the invention and T is defined as in the third aspect of the invention.

**In a fifth aspect, the invention pertains to a method of synthesizing a compound** according to the second aspect of the invention.

**In a sixth aspect, the invention pertains to an intermediate,** wherein the intermediate is selected from

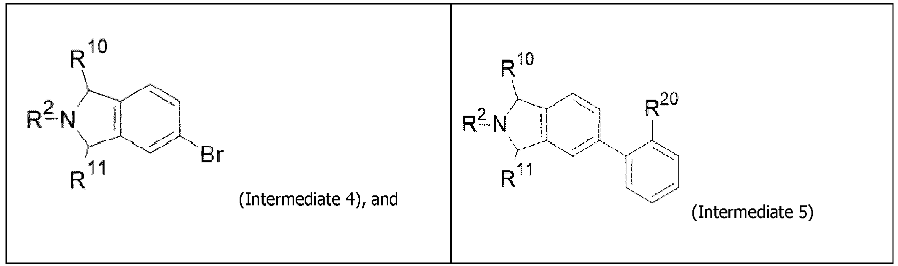

wherein R², R¹⁰, and R¹¹ are defined as in the second aspect of the invention and R²⁰ is -CN or -CO₂Me.

**In a seventh aspect, the invention pertains to a compound for use** in the treatment of a disease or condition in a subject, wherein the compound is a compound according to any other aspect of the invention.

**In an eighth aspect, the invention pertains to a therapeutic composition** comprising a compound of the invention and a pharmaceutically acceptable carrier and/or excipient.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the invention pertains to a compound, that comprises the formula (I) 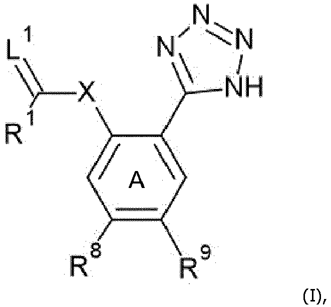
wherein the compound comprises a phenyl group A, wherein the phenyl group A is substituted with a tetrazole group and with a spiro group X,
wherein X comprises two aliphatic heterocycles B¹ and B² that are connected by a carbon spiro atom and each of B¹ and B² comprises a nitrogen heteroatom,
wherein B¹ is connected to A via a nitrogen heteroatom of B¹,
wherein B² is connected to an R¹(C=L¹) group via a nitrogen heteroatom of B²,
wherein the amount of ring atoms m¹ in B¹ is 6 or fewer,
wherein the amount of ring atoms m² in B² is 6 or fewer,
wherein R¹ is a Cₙ alkyl group with n = 1-5, a Cⱼ alkenyl group with j = 2-5, a C_{d} alkyne group with d = 2-5, or a C_{b} cycloalkyl group with b = 3-5, wherein optionally a hydrogen atom in R¹ is replaced with a fluor atom, a deuterium atom or a trifluormethyl group,
wherein L¹ is =O, =S, =C(CN)₂, =N-OH, or =N-OMe,
wherein R⁸ is selected from the group of -H, -Cl, -Br, -CH₃₋ᵣFᵣ, and -OCH₃, wherein r = 0-3.
wherein R⁹ is selected from -H, halogen and -CH₃₋ᵥFᵥ, wherein v = 0-3,
or a tautomer, isomer, diastereomer, enantiomer, hydrate, mixture or salt thereof.

In the context of the invention, term "halogen" means a fluor atom, chlorine atom, bromine atom, or iodine atom.

In the context of the invention, the term "alkyl group" refers to a saturated linear or branched hydrocarbon group. Preferably, the alkyl group comprises from 1 to 8, preferably 1 to 5, carbon atoms, i.e., 1, 2, 3, 4, or 5 carbon atoms. Exemplary linear or branched alkyl groups include methyl (Me), ethyl (Et), propyl, iso-propyl (also called 2-propyl or 1-methylethyl), butyl, iso-butyl, tert-butyl, n-pentyl, iso-pentyl, sec-pentyl, neo-pentyl, 1,2-dimethyl-propyl, iso-amyl, n-hexyl, iso-hexyl, sec-hexyl, n-heptyl, iso-heptyl, n-octyl and 2-ethyl-hexyl.

The term "alkenyl group" refers an unsaturated linear or branched hydrocarbon group having at least one carbon-carbon double bond. Preferably, such an alkenyl group comprises 2 to 8, more preferably 2 to 5, carbon atoms, i.e., 2, 3, 4, 5 carbon atoms. The carbon-carbon double bond(s) may be in cis (Z) or trans (E) configuration. Preferably, if an alkenyl group is attached to a nitrogen atom, the double bond cannot be alpha to the nitrogen atom.

The term "alkynyl" refers to a linear or branched hydrocarbon having at least one carbon-carbon triple bond. Preferably, the alkynyl group comprises from 2 to 8, preferably 2 to 5, carbon atoms, such as 2, 3, 4, 5 carbon atoms.

The term "cycloalkyl" or "cycloaliphatic" indicates a cyclic non-aromatic "alkyl" or "alkenyl" group. Preferably, such a cycloalkyl group contains 3 to 14 ring carbon atoms, preferably 3 to 10 ring carbon atoms, more preferably 3 to 7 ring carbon atoms, most preferably 3 to 5 ring carbon atoms. Exemplary cycloalkyl groups include cyclopropyl, cyclopropenyl, cyclobutyl, cyclobutenyl, cyclopentyl, cyclopentenyl, cyclohexyl, cyclohexenyl, cycloheptyl, cycloheptenyl, cyclooctyl, cyclooctenyl, cyclononyl, cyclononenyl, cylcodecyl, cylcodecenyl, and adamantyl. The term "cycloalkyl" includes bicyclic and tricyclic rings. In the case of a bicyclic ring, the respective rings can be connected to each other at two adjacent carbon atoms, or alternatively the two rings can be connected via the same carbon atom, thus forming a spiro ring system, or they may form "bridged" ring systems. A cycloalkyl group does not encompass two or more cycles that are linked via a single bond. The term "cycloalkyl" does not encompass fullerenes.

The term "aryl" or "aromatic ring" refers to an aromatic cyclic hydrocarbon. Preferably, such an aryl group contains 3 to 14 (e.g., 5, 6, 7, 8, 9, or 10, such as 5, 6, or 10) carbon atoms which can be arranged in one ring (e.g., phenyl) or two or more condensed rings (e.g., naphthyl). Preferably, "aryl" refers to a monocyclic ring containing 6 carbon atoms or an aromatic bicyclic ring system containing 10 carbon atoms. The term "aryl" does not encompass fullerenes.

In the context of the invention, if a group is "substituted", this term indicates that a hydrogen atom that is attached to said "substituted group" is replaced by another atom or group. In this context, one, two, three or more, preferably all, hydrogen atoms can be replaced with a substituent other than hydrogen. The term "substituted" in this context does not indicate a chemical mechanism, by which the substitution takes place. In the context of the invention, the term "substituted with a tetrazole group" refers to the replacement of a hydrogen atom with 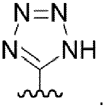 If not indicated otherwise, a moiety in the context of the invention is considered un-substituted.

In the context of the invention, if a group is "replaced", this term indicates that said group is exchanged by another group. The terms "replaced" or "exchanged" in this context do not indicate a chemical mechanism, by which the replacement or exchange is conducted.

The term "heteroaryl" or "heteroaromatic ring" means an aryl group as defined above in which one or more carbon atoms in the aryl group are replaced by heteroatoms such as O, S, or N. Preferably, heteroaryl refers to a five or six-membered aromatic monocyclic ring, wherein 1, 2, or 3 carbon atoms are replaced by the same or different heteroatoms of O, N, or 5 or heterogroups such as S(O) or S(O)₂. Preferably, the 1, 2, or 3, carbon atoms are replaced with the same or different heteroatoms consisting of O, 5, and N. Alternatively, the term heteroaryl means an aromatic bicyclic or tricyclic ring system wherein 1, 2, 3, 4, or 5 carbon atoms are replaced with the same or different heteroatoms of O, N, or 5 or heterogroups such as S(O) or S(O)₂. Preferably, the 1, 2, 3, 4, or 5 carbon atoms are replaced with the same or different heteroatoms consisting of O, 5, and N.

In the context of the invention, the term "heterocyclyl" or "heterocyclic ring" refers to a cycloalkyl group as defined above in which 1, 2, 3, or 4 ring carbon atoms in the cycloalkyl group are replaced by heteroatoms (such as O, 5, N, B, Si, and P) or heterogroups such as S(O) or S(O)₂. In one embodiment, the heteroatoms of the heterocyclyl group are selected from the group consisting of O, S, and N.

A "heteroatom" in the context of the invention refers to the replacement of a carbon atom in a group with another atom that is preferably selected from O, S, N, B, Si, and P, preferably selected from O, S, and N.

In the context of the invention, when referring to a "4-membered ring", a "5-membered ring" or a "6-membered ring" (and so on), the respective number refers to the number of ring atoms. In the case that such a ring is a cycloalkyl, then the ring atoms are carbon ring atoms. For example, a phenyl ring that is substituted with a methyl group in this context is a 6-membered ring with 6 carbon ring atoms. In the case that such a ring is a heterocyclyl, a carbon ring atom is replaced by a heteroatom. For example, a 5-membered heterocyclyl contains 5 ring atoms of which at least one ring atom is a heteroatom. For example, such a 5-membered heterocyclyl is pyrrolidine.

In the context of the invention, the symbol 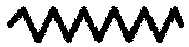 indicates the bond by which a respective group is bound to the remainder of the compound.

Preferably, the halogen in the context of R⁹ is -F, -Cl, or -Br. In preferred embodiments, R⁹ is selected from the group of -F, -Cl, -Br, and -CH₃₋ᵥFᵥ, wherein v = 0-3, preferably 3. In preferred embodiments, R⁹ is selected from the group of -F, -Cl, -Br, and -CF₃.

In preferred embodiments, R⁸ is selected from the group of -H, -Cl, -CH₃, -Br, -CF₃ and -OMe.

In preferred embodiments of the invention, R⁸ and R⁹ are H.

In preferred embodiments, R¹ is a C_{b} cycloalkyl group with b = 3-5, wherein optionally a hydrogen atom in R¹ is replaced with deuterium, fluor or a trifluormethyl group. In preferred embodiments, R¹ is selected from the group of cyclopropyl, cyclopentyl, and a bicyclo[1.1.1]pentan group, wherein optionally a hydrogen atom in the cyclopropyl, cyclopentyl, and the bicyclo[1.1.1]pentan group is replaced with a fluor or a trifluormethyl.

In preferred embodiments, a hydrogen atom in R¹ is replaced by deuterium. In preferred embodiments, all hydrogen atoms in R¹ are replaced by deuterium. In preferred embodiments, R¹ is a linear Cₙ alkyl group with n = 1-5, and all hydrogen atoms in R¹ are replaced by deuterium. In preferred embodiments, n = 4.

In preferred embodiments of the invention, the compound comprises the formula (I-a) 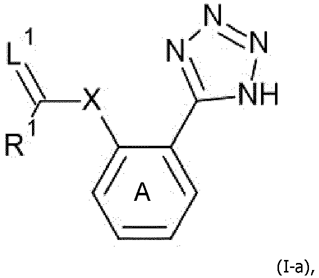
wherein the compound comprises a phenyl group A, wherein the phenyl group A is substituted with a tetrazole group and with a spiro group X,
wherein X comprises two aliphatic heterocycles B¹ and B² that are connected by a carbon spiro atom and each of B¹ and B² comprises one nitrogen heteroatom,
wherein B¹ is connected to A via the nitrogen heteroatom of B¹,
wherein B² is connected to an R¹(C=L¹) group via the nitrogen heteroatom of B²,
wherein the amount of ring atoms m¹ in B¹ is 6 or fewer,
wherein the amount of ring atoms m² in B² is 6 or fewer,
wherein R¹ is a Cₙ alkyl group with n = 1-5, a Cⱼ alkenyl group with j = 2-5, or a C_{d} alkyne group with d = 2-5, wherein optionally a hydrogen in R¹ is replaced with a fluor atom,
wherein L¹ is =O, =S, =C(CN)₂, =N-OH, or =N-OMe,
or a tautomer, isomer, diastereomer, enantiomer, hydrate, mixture or salt thereof.

In preferred embodiments of the invention, the compound comprises the formula (I-b) 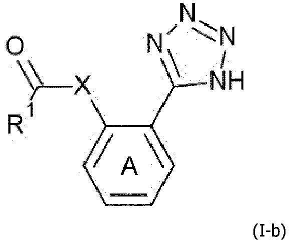
wherein the compound comprises a phenyl group A, wherein the phenyl group A is substituted with a tetrazole group and with a spiro group X,
wherein X comprises two aliphatic heterocycles B¹ and B² that are connected by a carbon spiro atom and each of B¹ and B² comprises one nitrogen heteroatom,
wherein B¹ is connected to A via the nitrogen heteroatom of B¹,
wherein B² is connected to an alkanoyl group via the nitrogen heteroatom of B², wherein the alkanoyl group comprises a R¹, wherein R¹ is a linear Cₙ alkyl group with n = 1-4,
wherein the amount of ring atoms m¹ in B¹ is 6 or fewer,
wherein the amount of ring atoms m² in B² is 6 or fewer,
or a tautomer, isomer, diastereomer, enantiomer, hydrate, mixture or salt thereof.

In preferred embodiments of the invention, X is a diazaspiro group. In preferred embodiments of the invention, X comprises two heteroatoms. Preferably, X is a diazaspiro group that comprises two heteroatoms.

In preferred embodiments of the invention, there is no nitrogen heteroatom next to the carbon spiro atom in the ring B¹ and/or in the ring B².

In preferred embodiments, in the case that B¹ comprises an even number of "m¹" ring atoms, the nitrogen heteroatom is at the position (m')/2 from the carbon spiro atom. In preferred embodiments, in the case that B² comprises an even number of "m²" ring atoms, the nitrogen heteroatom is at the position (m²)/2 from the carbon spiro atom. In this context, "m¹" refers to the number of ring atoms in B¹ and "m²" refers to the number of ring atoms in B². In preferred embodiments, in the case that B¹ and B² each comprises an even number of "m¹" and "m²" ring atoms, the nitrogen heteroatom is at the position (m')/2 and (m²)/2 from the carbon spiro atom respectively. For example, in an embodiment in which the spiro group is a 3,9-diazaspiro[5.5] undecane group, both B¹ and B² comprise an even number of 6 ring atoms and the nitrogen heteroatoms are at the positions (m')/2 (= 6/2, therefore 3) and (m²)/2 (= 6/2, therefore 3), as indicated below.

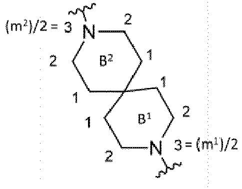

In other preferred embodiments, in the case that B¹ and/or B² comprise an even number of "m¹" and/or "m²" ring atoms, the nitrogen heteroatom is not at the position of (m')/2 and/or (m²)/2 from the carbon spiro atom respectively.

In preferred embodiments of the invention, there is no nitrogen heteroatom next to the carbon spiro atom in B¹ and B² and in the case that B¹ and B² each comprises an even number of "m¹" and "m²" ring atoms, the nitrogen heteroatom is at the position (m')/2 and (m²)/2 from the carbon spiro atom respectively.

In preferred embodiments, the spiro group is a spiro[y.z] group with y ≤ 5 and z ≤ 5, such as a diazaspiro[y.z] group with y ≤ 5 and z ≤ 5. In this context, the spiro[y.z] group refers to a spiro[y.z] C_{(y+z+1)}alkane group. For example, a spiro[y.z] group with y = 4 and z = 5 refers to a spiro[4.5]decane group. While according to the spiro-nomenclature, such compounds are called a spiro[y.z]"alkane", this does not indicate that there are no heteroatoms in the rings of the spiro-compounds.

In preferred embodiments of the invention y = z. In preferred embodiments, the number of ring atoms in B¹ is equal to the number or ring atoms in B². In preferred embodiments of the invention, the number of ring atoms in B¹ and B² is each 4 or each 5, more preferably wherein the number of ring atoms in B¹ and B² is 4. In embodiments, in which B¹ comprises 4 or 5 ring atoms, the compounds of the invention yielded a particular high efficacy in BLT2 activation. The spiro compounds of the invention exhibit the lowest BLT2 EC₅₀ values and the highest efficacies with B¹ and B² both having 4 ring atoms.

In other embodiments, y ≠ z. In preferred embodiments of the invention, B¹ comprises more ring atoms than B². In preferred embodiments of the invention, B¹ comprises fewer ring atoms than B².

In particular preferred embodiments of the invention, B¹ does not comprise 6 ring atoms. In particularly preferred embodiments, B¹ does comprise fewer than 6 ring atoms. Preferably, B¹ comprises 4 or 5 ring atoms. Preferably, B¹ comprises more than 3 ring atoms. Preferably, B¹ and B² comprises more than 3 ring atoms. In preferred embodiments of the invention, B¹ comprises 4 ring atoms, and B² comprises 4, 5 or 6 ring atoms, preferably wherein B² comprises 4 ring atoms. In the case of B¹ comprising four ring atoms, the efficacy of the compounds of the invention in BLT2 activation increases with decreasing ring size of B² from 6 to 4 ring atoms.

In preferred embodiments of the invention B¹ comprises 5 ring atoms, and B² comprises 4, 5 or 6 ring atoms, preferably wherein B² comprises 6 ring atoms. In preferred embodiments of the invention, B¹ comprises fewer ring atoms than B², preferably wherein B¹ comprises 5 ring atoms. In the case of B¹ comprising 5 ring atoms, the efficacy of the compounds of the invention in BLT2 activation increases with increasing the ring size of B² from 4 to 6 ring atoms.

In preferred embodiments of the invention, y and z is 3. In preferred embodiments, the number of ring atoms in B¹ and B² is 4. Preferably, the spiro group is a 2,6-diazaspiro[3.3]heptane group

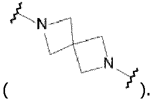

In preferred embodiments of the invention, y and z is 4. In preferred embodiments, the number of ring atoms in B¹ and B² is 5. Preferably, the spiro group is a 2,7-diazaspiro[4.4]nonane group

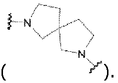

In preferred embodiments of the invention, y is 4, z is 5, and B¹ comprises fewer ring atoms than B². Preferably, the spiro group is a 2,8-diazaspiro[4.5]decane group

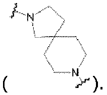

In preferred embodiments of the invention, the spiro group is selected from the group of

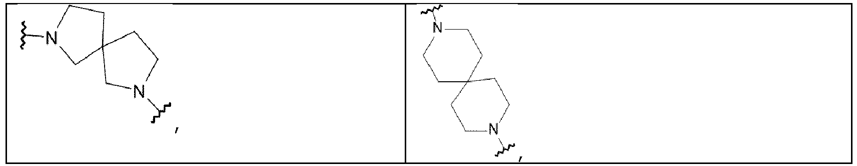

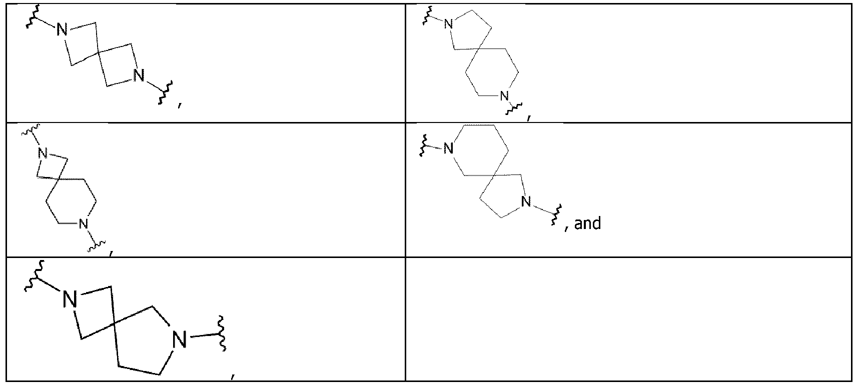

preferably the spiro group is selected from the group of

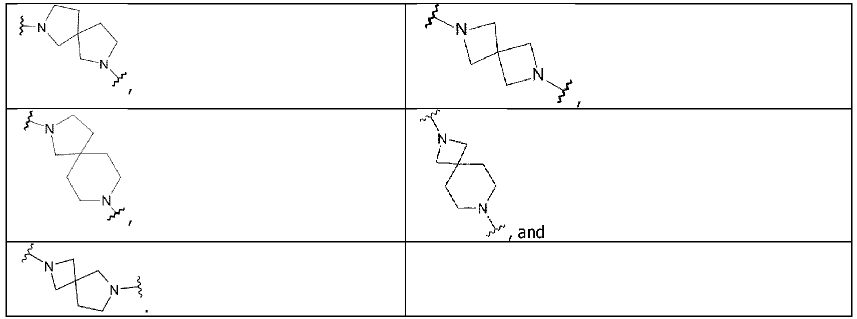

In preferred embodiments of the invention, wherein the spiro group is selected from the group of

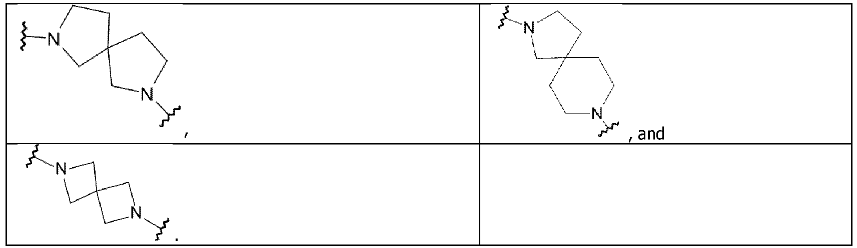

In preferred embodiments of the invention, X is not a diazaspiro[y.z] group with y = 3 and z = 5. In preferred embodiments of the invention, when X is a diazaspiro[y.z] group with y = 3 and z = 5, then B¹ is smaller than B².

In preferred embodiments of the invention, n is 4, j is 4 and/or d is 4. These embodiments refer to the context of R¹.

In preferred embodiments of the invention, R¹ is a linear or branched Cₙ alkyl, preferably wherein R¹ is a linear Cₙ alkyl. In most preferred embodiments of the invention, R¹ is a linear C₄ alkyl.

In preferred embodiments of the invention L¹ is =O or =S. In most preferred embodiments, L¹ is =O.

In preferred embodiments of the invention, the compound comprises a structure that is selected from the group of

| | |
|---|---|
| 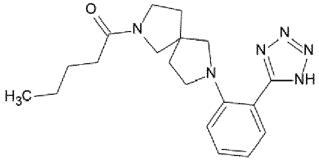 Compound 10: 1-(7-(2-(1H Tetrazol-5-yl)phenyl)-2,7-diazaspiro[4.4]nonan-2-yl)pentan-1-one, | 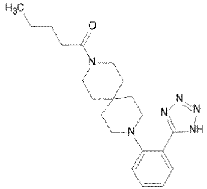 Compound 12: 1-(9-(2-(1H Tetrazol-5-yl)phenyl)-3,9-diazaspiro[5.5]undecan-3-yl)pentan-1-one, |
| 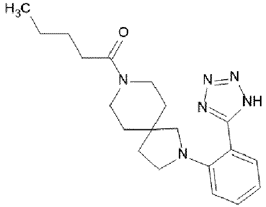 Compound 13: 1-(2-(2-(1H Tetrazol-5-yl)phenyl)-2,8-diazaspiro[4.5]decan-8-yl)pentan-1-one, | 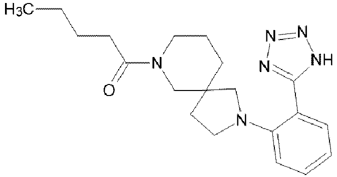 Compound 14: 1-(2-(2-(1H-Tetrazol-5-yl)phenyl)-2,7-diazaspiro[4.5]decan-7-yl)pentan-1-one, |
| 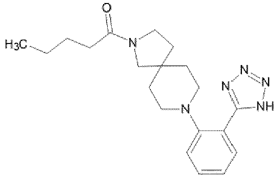 Compound 15: 1-(8-(2-(1H Tetrazol-5-yl)phenyl)-2,8-diazaspiro[4.5]decan-2-yl)pentan-1-one, | 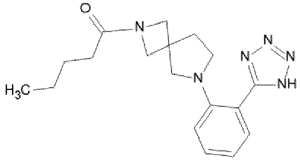 Compound 16: 1-(6-(2-(1H-Tetrazol-5-yl)phenyl)-2,6-diazaspiro[3.4]octan-2-yl)pentan-1-one, |
| 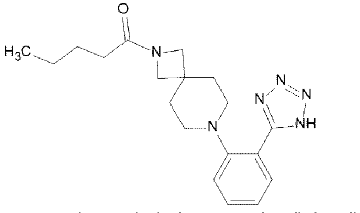 Compound 17: 1-(7-(2-(1H Tetrazol-5-yl)phenyl)-2,7-diazaspiro[3.5]nonan-2-yl)pentan-1-one, | 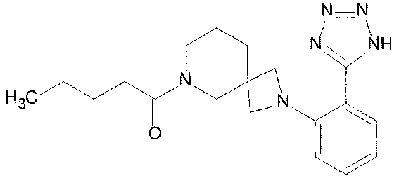 Compound 18: 1-(2-(2-(1H-Tetrazol-5-yl)phenyl)-2,7-diazaspiro[3.5]nonan-7-yl)pentan-1-one, |
| 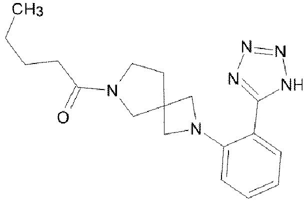 Compound 19: 1-(2-(2-(1H Tetrazol-5-yl)phenyl)-2,6-diazaspiro[3.4]octan-6-yl)pentan-1-one, and | 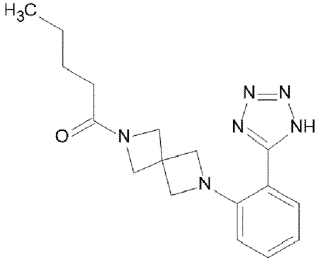 Compound 20: 1-(6-(2-(1H-Tetrazol-5-yl)phenyl)-2,6-diazaspiro[3.3]heptan-2-yl)pentan-1-one. |

In preferred embodiments, the compound comprises a structure that is selected from the group of **Compound 10, Compound 13** and **Compound 20.** In most preferred embodiments, the compound is **Compound 20.**

**In a second aspect, the invention pertains to a compound** that comprises a structure with the formula (II) 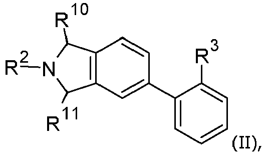
wherein R³ is a 1H-Tetrazol-5-yl group 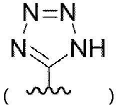 or a carboxyl group,
wherein R¹⁰ and R¹¹ are independently selected from -H, phenyl and a C₁₋₃ alkyl, preferably wherein at least one of R¹⁰ and R¹¹ is -H,
wherein R² is R⁴-(C=L²)-, wherein R⁴ is a Cₚ alkyl group with p = 1-5, a Cₒ alkenyl group with o = 2-5, or a Cε alkyne group with t = 2-5, wherein optionally R⁴ is substituted with a substituent selected from fluor and R⁵,
wherein R⁵ is a methyl group, ethyl group or phenyl group,
wherein L² is =O, =S, =C(CN)₂, =N-OH, =N-OMe, preferably =O or =S,
or wherein R² is a 5-membered heteroaryl with at least one nitrogen heteroatom, wherein the 5-membered heteroaryl with at least one nitrogen heteroatom comprises one substituent R⁶ and/or one substituent R⁷,
wherein R⁶ and R⁷ are independently selected from -H and a C_{q} alkyl-group with q = 1 to 3, or wherein R⁶ and R⁷ form a 6-membered ring together with the atoms that they are attached to that comprises at least 5 carbon ring atoms,
or a tautomer, isomer, diastereomer, enantiomer, hydrate, mixture or salt thereof.

In preferred embodiments of the invention, the 5-membered heteroaryl comprises one or two, preferably two nitrogen heteroatoms. In preferred embodiments of the invention, the 5-membered heteroaryl additionally comprises one oxygen heteroatom or one sulfur heteroatom. In preferred embodiments of the invention, the 5-membered heteroaryl comprises two nitrogen heteroatoms and one oxygen heteroatom. In preferred embodiments of the invention, the 5-membered heteroaryl comprises one nitrogen heteroatom and one heteroatom selected from oxygen and sulfur. In preferred embodiments of the invention, q is 2.

In preferred embodiments of the invention, L² is =O or =S, preferably L² is =O. In preferred embodiments of the invention, R⁴ is a branched or linear Cₚ alkyl group with p = 1-4. Most preferably, R⁴ is a linear Cₚ alkyl group with p = 4. In preferred embodiments, in the context of R⁴, p is 4, o is 4, and/or t is 4.

If not indicated otherwise, a moity in the context of the invention is considered un-substituted. Therefore, in preferred embodiments of the invention, R⁴ is unsubstituted. Therefore, in preferred embodiments of the invention, p is 4 and R⁴ is a unsubstituted linear Cₚ alkyl group with p = 4.

In preferred embodiments of the invention, the compound comprises a structure with the formula (II) 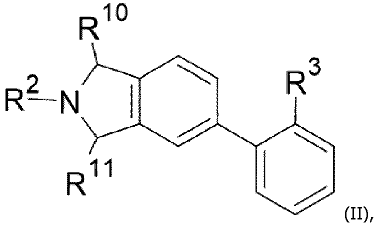
wherein R³ is a 1H-Tetrazol-5-yl group 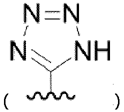 or a carboxyl group,
wherein R¹⁰ and R¹¹ are independently selected from -H, phenyl and methyl, wherein at least one of R¹⁰ and R¹¹ is -H,
wherein R² is R⁴-(C=O)-, wherein R⁴ is a linear Cₚ alkyl group with p = 1-4, optionally wherein R⁴ is substituted with a substituent selected from fluor and one R⁵,
wherein R⁵ is a methyl group, ethyl group or phenyl group,
or wherein R² is a 5-membered heteroaryl with at least one nitrogen heteroatom, wherein the 5-membered heteroaryl with at least one nitrogen heteroatom comprises one substituent R⁶ and/or one substituent R⁷,
wherein R⁶ and R⁷ are independently selected from -H and a C_{q} alkyl-group with q = 1 to 3,
or a tautomer, isomer, diastereomer, enantiomer, hydrate, mixture or salt thereof.

In preferred embodiments of the invention, the compound comprises a structure with the formula (II) 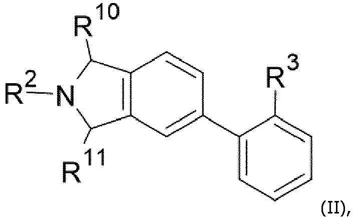
wherein R³ is a 1H-Tetrazol-5-yl group 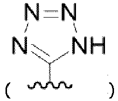 or a carboxyl group,
wherein R¹⁰ and R¹¹ are independently selected from -H, and methyl, preferably wherein at least one of R¹⁰ and R¹¹ is -H,
wherein R² is R⁴-(C=O)-, wherein R⁴ is a linear Cₚ alkyl group with p = 1-4, optionally, wherein R⁴ is substituted with a substituent selected from fluor and one R⁵,
wherein R⁵ is a methyl group, ethyl group or phenyl group,
or a tautomer, isomer, diastereomer, enantiomer, hydrate, mixture or salt thereof.

In preferred embodiments of the invention, the compound comprises a structure with the formula (II) 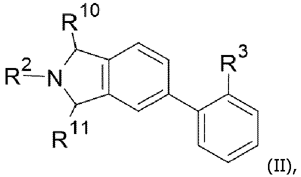
wherein R³ is a 1H-Tetrazol-5-yl group 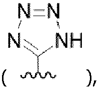
wherein R¹⁰ and R¹¹ are -H,
wherein R² is R⁴-(C=O)-, wherein R⁴ is a linear Cₚ alkyl group with p = 1-4,
or wherein R² comprises the structure 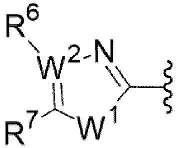
with W¹ is C, O, or S; and W² is C, N, or O, wherein
R⁶ and R⁷ is independently selected from -H and a C_{q} alkyl-group with q = 1 to 3,
or a tautomer, isomer, diastereomer, enantiomer, hydrate, mixture or salt thereof.

In preferred embodiments of the invention, p is 3 or 4. In particular preferred embodiments, p is 4. In preferred embodiments, R⁴ is a linear Cₚ alkyl group with p = 1-4.

In preferred embodiments of the invention, wherein R² is R⁴-(C=O)-, R⁴ is a linear Cₚ alkyl group with p = 1-4.

In preferred embodiments of the invention, R³ is a 1H-Tetrazol-5-yl group 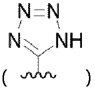 or a carboxyl group (-C(=O)OH). In most preferred embodiments of the invention, R³ is a 1H-Tetrazol-5-yl group. By introducing to the position R³ a 1H-Tetrazol-5-yl group, the potency of the compounds of the invention (for example as seen in the low EC₅₀ values for BLT2 binding and a high efficacy in BLT2 activation) is enhanced significantly.

In preferred embodiments of the invention, R⁴ is mono-substituted with an R⁵ at the carbon atom that is in α-position or at a carbon-atom that is in β-position of the (C=L²) group of the R⁴-(C=L²)-, wherein R⁵ is a phenyl group, methyl group or ethyl group. Preferably, R⁴ is mono-substituted with an R⁵ at the carbon atom that is in α-position or at a carbon-atom that is in β-position of the carbonyl group of the R⁴-(C=O)-, wherein R⁵ is selected from a phenyl group, methyl group or ethyl group.

In this context, the position designations α, β, γ, and δ are used as follows:

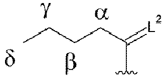

In preferred embodiments of the invention, p is 3 and R⁴ is mono-substituted with an R⁵ at the carbon atom that is in α-position or at a carbon-atom that is in β-position of the carbon atom of the (C=L²) group of the R⁴-(C=L²)-, wherein R⁵ is a phenyl group, methyl group or ethyl group. Preferably, p is 3 and R⁴ is mono-substituted with an R⁵ at the carbon atom that is in α-position or at a carbon-atom that is in β-position of the carbon atom of the carbonyl group of the R⁴-(C=O)-, wherein R⁵ is selected from a phenyl group, methyl group and ethyl group.

In preferred embodiments of the invention, R⁴ is substituted with a R⁵. In preferred embodiments of the invention, R⁴ is substituted with one R⁵. In preferred embodiments of the invention, R⁵ is a phenyl group, methyl group or ethyl group.

In preferred embodiments of the invention, p is 3 and R⁴ is mono-substituted with an ethyl group that is connected to the carbon atom in α-position of the (C=L²) group of the R⁴-(C=L²)-. In preferred embodiments, p is 3 and R⁴ is mono-substituted with an ethyl group that is connected to the carbon atom in α-position of the carbonyl group of R⁴-(C=O)-.

In preferred embodiments of the invention, p is 3 and R⁴ is mono-substituted with a phenyl group that is connected to the carbon atom in α-position of the (C=L²) group of the R⁴-(C=L²)-. In preferred embodiments, p is 3 and R⁴ is mono-substituted with a phenyl group that is connected to the carbon atom in α-position of the carbonyl group of R⁴-(C=O)-.

In preferred embodiments of the invention, wherein p is 3 and R⁴ is mono-substituted with a methyl group that is connected to the carbon atom in α-position of the (C=L²) group of the R⁴-(C=L²)-. In preferred embodiments, p is 3 and R⁴ is mono-substituted with a methyl group that is connected to the carbon atom in α-position of the carbonyl group of R⁴-(C=O)-.

In preferred embodiments, the carbon atom to which R⁵ is connected to is a chiral center that is in *R* or Sconfiguration. In preferred embodiments of the invention, R⁵ is a methyl group. In most preferred embodiments of the invention, R⁵ is a methyl group and the carbon atom to which R⁵ is connected to is a chiral center that is in 5 configuration. Such a substitution resulted in a particularly low BLT2 EC₅₀ and a high efficacy in BLT2 activation compared to CAY.

In preferred embodiment of the invention, R¹⁰ and R¹¹ are independently selected from -H, a phenyl and a methyl, preferably wherein at least one of R¹⁰ and R¹¹ is -H. In preferred embodiments of the invention, R¹⁰ is selected from -H, phenyl or methyl. In preferred embodiments of the invention, R¹¹ is selected from -H and phenyl. In preferred embodiments of the invention at least one of R¹⁰ and R¹¹ is -H. In preferred embodiments of the invention, not both R¹⁰ and R¹¹ are -H.

In most preferred embodiments of the invention R¹⁰ and R¹¹ are -H. In this embodiment, the compounds of the invention exhibited the lowest BLT2 EC₅₀ values as well as the highest efficacies.

In preferred embodiments of the invention, R² is a 5-membered heteroaryl with at least one nitrogen heteroatom,
wherein the 5-membered heteroaryl with at least one nitrogen heteroatom comprises one substituent R⁶ and/or one substituent R⁷,
wherein R⁶ and R⁷ are independently selected from -H and a C_{q} alkyl-group with q = 1 to 3, or wherein R⁶ and R⁷ form a 6-membered ring together with the atoms that they are attached to that comprises at least 5 carbon ring atoms.

In preferred embodiments of the invention, R⁶ and R⁷ are each attached to a carbon atom and the carbon atom that R⁶ and R⁷ is attached to, is not the same carbon atom. Preferably, R⁶ and R⁷ form a 6-membered ring comprising 6 carbon ring atoms together with the atoms that they are attached to.

In preferred embodiments of the invention, R² comprises the structure 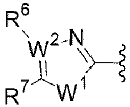
wherein W¹ is C, O, or S; and W² is C, N, or O, wherein
R⁶ and R⁷ is independently selected from -H and a C_{q} alkyl-group with q = 1 to 3, or wherein R⁶ and R⁷ form a 6-membered ring together with the atoms that they are attached to that comprises at least 5 carbon ring atoms.

In preferred embodiments of the invention, q is 2.

In preferred embodiments of the invention R⁶ is -H. In preferred embodiments of the invention, R⁶ is -H and R⁷ is a C_{q} alkyl-group with q = 1 to 3, preferably, wherein R⁷ is ethyl. In preferred embodiments of the invention, R⁶ is -H and R⁷ is an ethyl group. With this selection of substituents, the lowest BLT2 EC₅₀ values were achieved for the isoindoline derivatives and the efficacy of the compounds of the invention in BLT2 activation was increased.

In preferred embodiments of the invention, W² is C and W¹ is S, preferably wherein R⁷ is ethyl and R⁶ is -H. In preferred embodiments of the invention, W² is O and W¹ is C, preferably wherein R⁷ is ethyl and R⁶ is -H.

In preferred embodiments of the invention, W² is N and W¹ is O, preferably wherein R⁷ is ethyl and R⁶ is -H. With this selection of substituents, the BLT2 EC₅₀ values were minimized and the efficacy of the compounds of the invention in BLT2 activation was maximized compared to CAY.

In preferred embodiments of the invention, R² is selected from, 5-ethylisoxazole, 1,3,4,-oxadiazole, 5-ethylthiazole, 4,5,6,7,tethrahydrobenzo[d]thiazole, 4-ethylthiazole, and 1-ethyl-1H-pyrazol-3-yl. In more preferred embodiments of the invention, R² is selected from 5-ethylisoxazole, 1,3,4,-oxadiazole, and 5-ethylthiazole.

In preferred embodiments of the invention, R² is selected from

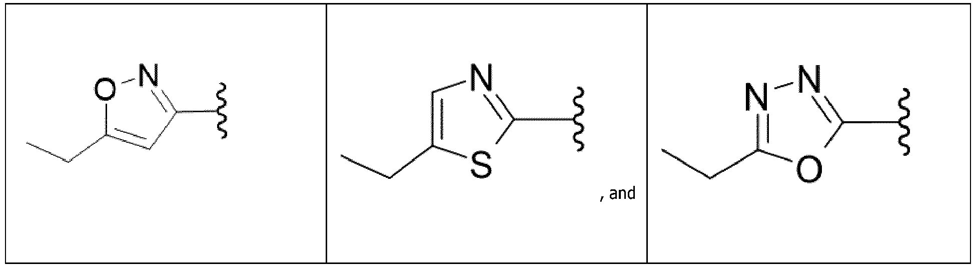

In preferred embodiments of the invention, wherein the compound comprises a structure selected from the group of:

| | |
|---|---|
| 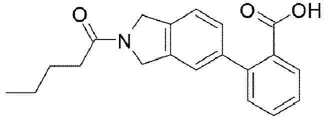 Compound 11: 2-(2-Pentanoylisoindolin-5-yl)benzoic acid, | 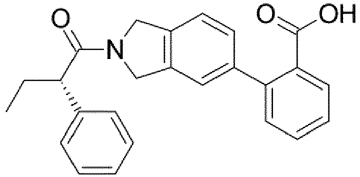 Compound 21: (S)-2-(2-(2-Phenylbutanoyl)isoindolin-5-yl)benzoic acid, |
| 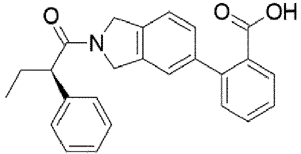 Compound 22: (R)-2-(2-(2-Phenylbutanoyl)isoindolin-5-yl)benzoic acid, | 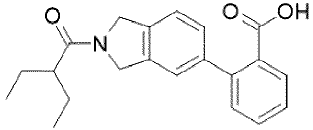 Compound 23: 2-(2-(2-Ethylbutanoyl)isoindolin-5-yl)benzoic acid, |
| 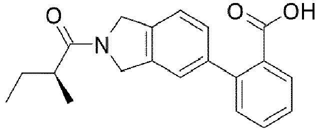 Compound 24: (S)-2-(2-(2-Methylbutanoyl)isoindolin-5-yl)benzoic acid, | 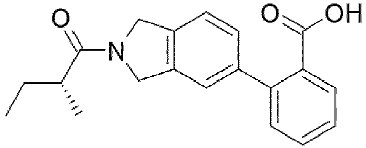 Compound 25: (R)-2-(2-(2-Methylbutanoyl)isoindolin-5-yl)benzoic acid, |
| 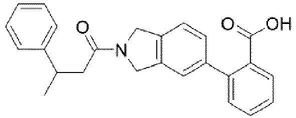 Compound 26: 2-(2-(3-Phenylbutanoyl)isoindolin-5-yl)benzoic acid, | 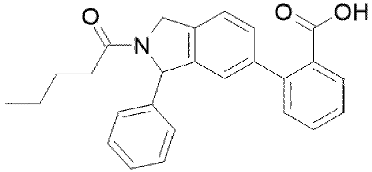 Compound 27: 2-(2-Pentanoyl-3-phenylisoindolin-5-yl)benzoic acid, |
| 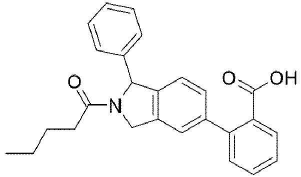 Compound 28: 2-(2-Pentanoyl-1-phenylisoindolin-5-yl)benzoic acid, | 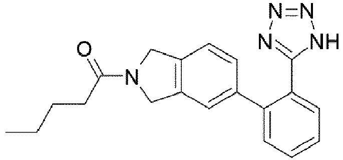 Compound 29: 1-(5-(2-(1H-Tetrazol-5-yl)phenyl)isoindolin-2-yl)pentan-1-one, |
| 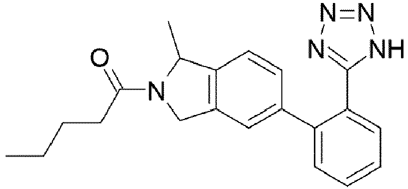 Compound 30: 1-(5-(2-(1H-Tetrazol-5-yl)phenyl)-1-methylisoindolin-2-yl)pentan-1-one, | 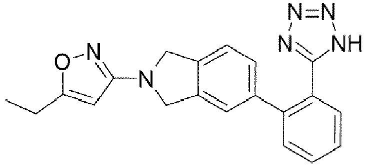 Compound 31: 3-(5-(2-(1H-Tetrazol-5-yl)phenyl)isoindolin-2-yl)-5-ethylisoxazole, |
| 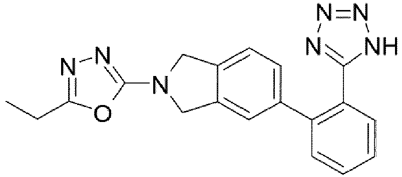 Compound 32: 2-(5-(2-(1H-Tetrazol-5-yl)phenyl)isoindolin-2-yl)-5-ethyl-1,3,4-oxadiazole, | 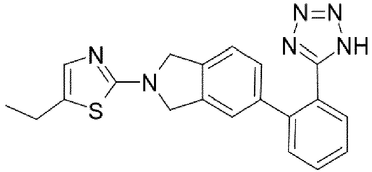 Compound 33: 2-(5-(2-(1H-Tetrazol-5-yl)phenyl)isoindolin-2-yl)-5-ethylthiazole, |
| 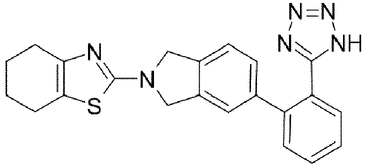 Compound 34: 2-(5-(2-(1H-Tetrazol-5-yl)phenyl)isoindolin-2-yl)-4,5,6,7-tetrahydrobenzo[d]thiazole, | 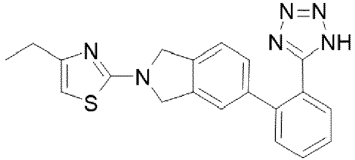 Compound 35: 2-(5-(2-(1H-Tetrazol-5-yl)phenyl)isoindolin-2-yl)-4-ethylthiazole, and |
| 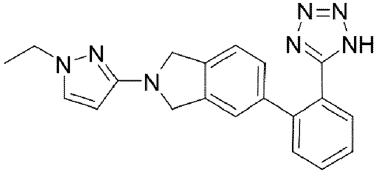 Compound 36: 5-(2-(1H-Tetrazol-5-yl)phenyl)-2-(1-ethyl-1H-pyrazol-3-yl)isoindoline. | |

In preferred embodiments of the invention, wherein the compound is selected from the group of **Compound 11, Compound 24, Compound 29, Compound 30, Compound 31, Compound 32** or **Compound 33.**

In preferred embodiments of the invention, wherein the compound is selected from the group of **Compound 11, Compound 29, Compound 30, Compound 31, Compound 32** or **Compound 33.**

In preferred embodiments of the invention, wherein the compound is selected from the group of **Compound 11, Compound 31, Compound 32** and **Compound 33,** preferably **Compound 31, Compound 32** and **Compound 33.**

In most preferred embodiments, the compound is **Compound 32.**

**In a third aspect, the invention pertains to a method of synthesizing a compound** according to the first aspect of the invention. Preferably, the compound comprises the formula (I), (I-a) or (I-b).

In preferred embodiments, L¹ in the compound that comprises the formula (I) is O.

In preferred embodiments of the invention, the synthesis comprises a reaction step a: 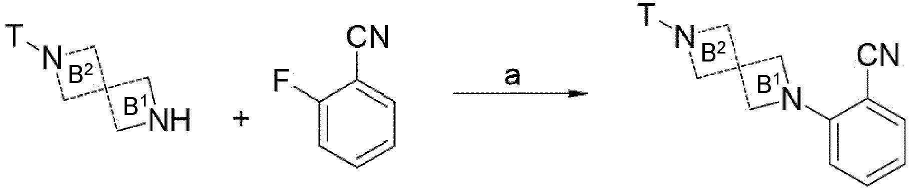
wherein T is an amine protection group such as Boc, Fmoc or trityl,
wherein the method comprises a step of reacting a spiro compound in a nucleophilic aromatic substitution with 2-fluorobenzonitrile,
wherein B¹ and B² are two aliphatic heterocycles that are connected by a carbon spiro atom and together form the spiro compound, wherein each B¹ and B² comprises a nitrogen heteroatom. Preferably, the amine protection group is Boc.

In this context, Boc refers to a tert-butyloxycarbonyl group, Fmoc refers to a fluorenylmethoxycarbonyl group, and trityl refers to a triphenylmethyl group.

In this context, 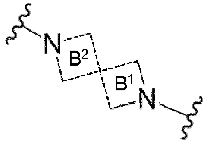 as in 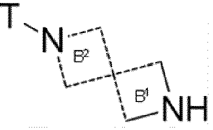 refers to a spirocyclic group that comprises two rings, namely B² and B', as defined herein.

In preferred embodiments of the invention, B¹ and B² are defined according to another aspect of the invention.

In preferred embodiments of the invention, the reaction step a generates a compound selected from **Compounds 70 to 79.**

In preferred embodiments of the invention, the method comprises a reaction step b of removing the amine protection group, thus generating a secondary amine group, preferably wherein the reaction step b is

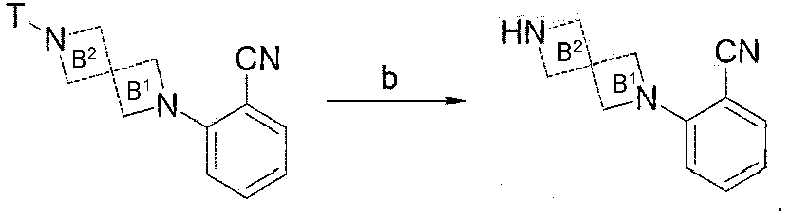

In preferred embodiments, the amine protection group is Boc. Preferably, the Boc is removed with trifluoroacetic acid.

In preferred embodiments of the invention, the reaction step b generates a compound selected from **Compounds 80 to 89.**

In preferred embodiments of the invention, the method comprises a step c of acylating the secondary amine group of B², preferably wherein reaction step c is

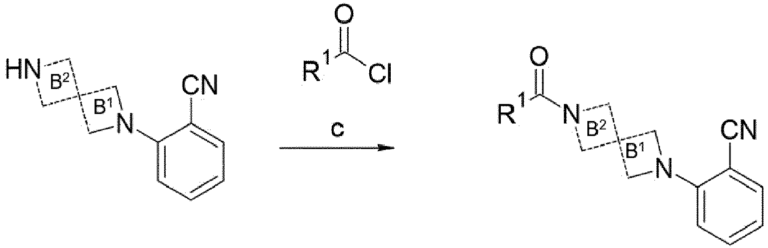

In preferred embodiments of the invention, the reaction step c is conducted in the presence of a weak base such as triethylamine.

In preferred embodiments of the invention, reaction step c generates a compound selected from the **Compounds 90 to 99.**

In preferred embodiments of the invention, the method comprises a reaction step d of replacing a nitrile group with a tetrazole group, preferably wherein reaction step d is 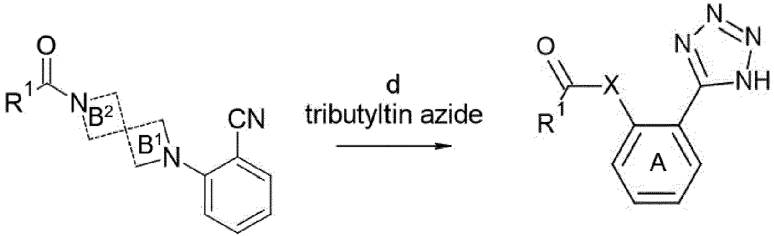 thus generating a compound according to the first aspect of the invention, wherein L¹ of compound with is O.

In preferred embodiment, the reaction step d is conducted in p-xylene, preferably at reflux temperature.

In preferred embodiments of the invention, the method comprises sequentially conducting the reaction steps a-d in the sequence a → b → c → d.

In preferred embodiments of the invention, wherein the method comprises sequentially conducting the reaction steps a-d in the sequence a → d → b → c.

**In a fourth aspect, the invention pertains to an intermediate,** wherein the intermediate is selected from

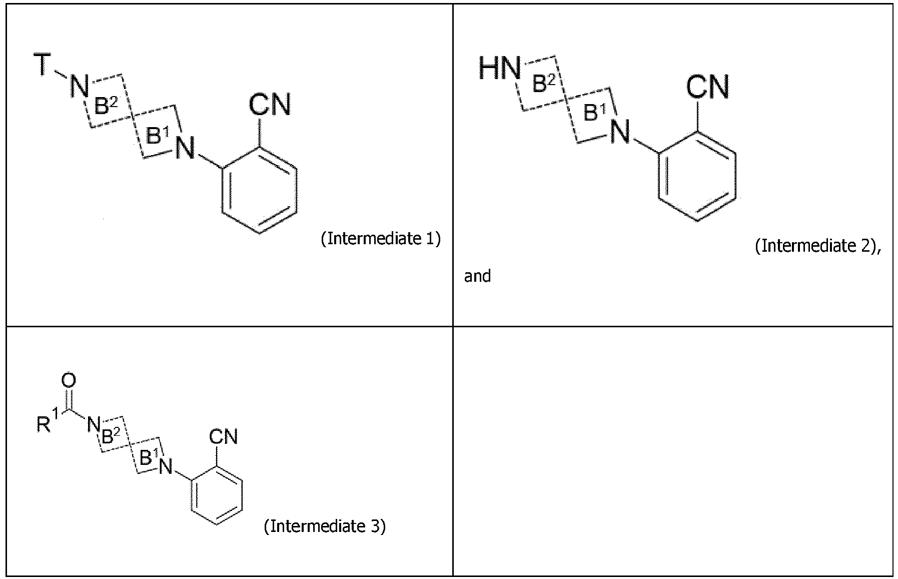

wherein, 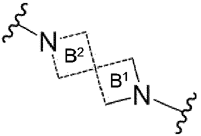 refers to a spirocyclic group that comprises two rings, namely B² and B¹, as defined according to the first aspect of the invention, R¹ is defined according to the first aspect of the invention and T is defined as in the third aspect of the invention.

In preferred embodiments of the invention, **Intermediate 1** is selected from **Compounds 70 to 79.** In preferred embodiments of the invention, **Intermediate 2** is selected from **Compounds 80 to 89.** In preferred embodiments of the invention, **Intermediate 3** is selected from **Compounds 90 to 99.**

**In a fifth aspect, the invention pertains to a method of synthesizing a compound** according to the second aspect of the invention. Preferably, the compound is a compound comprising the formula (II).

In preferred embodiments of the invention, the synthesis comprises a reaction step e₁, e₂, or e₃: 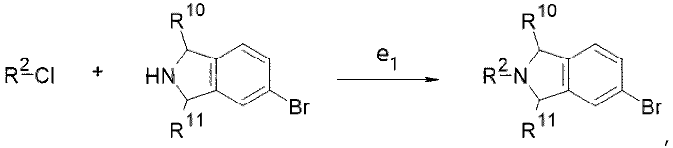 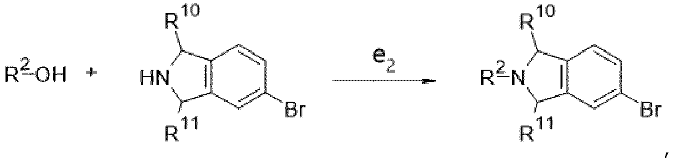 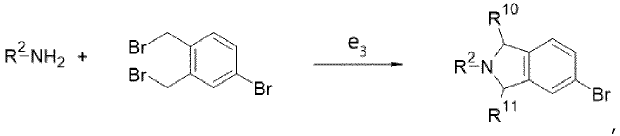 wherein R², R¹⁰ and R¹¹ are defined according to another aspect of the invention.
Preferably, reaction step e₁ is conducted in the presence of triethylamine, wherein reaction step e₂ is preferably conducted in the presence of HBTU, methylmorpholine and EDCL, and/or reaction step e₃ is preferably conducted in the presence of sodium hydride.

In preferred embodiments of the invention, the reaction step e₁ generates a compound selected from **Compounds 100, 107-109.**

In preferred embodiments of the invention, the reaction step e₂ generates a compound selected from **Compounds 101-106.**

In preferred embodiments of the invention, the reaction step e₃ generates a compound selected from **Compounds 110-115.**

In preferred embodiments of the invention, the method comprises a reaction step f, 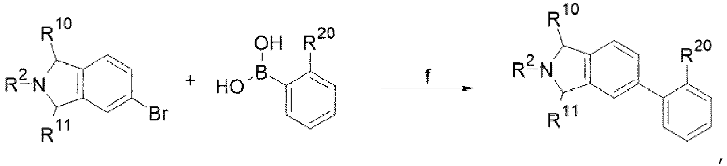 wherein B is boron, R²⁰ is -CO₂Me (thus reaction step f comprises a reaction with 2-methoxycarbonylphenylboronic acid) or R²⁰ is -CN (thus reaction step f comprises a reaction with 2-cyanophenylboronic acid. Preferably the reaction is conducted in the presence of Pd(PPh₃) and potassium carbonate or cesium carbonate.

In preferred embodiments of the invention, the reaction step f with 2-methoxycarbonylboronic acid generates a compound selected from the **Compounds 116-125.**

In preferred embodiments of the invention, the reaction step f with 2-cyanophenylboronic acid generates a compound selected from the **Compounds 125-132.**

In preferred embodiments of the invention, the method comprises a reaction step g, 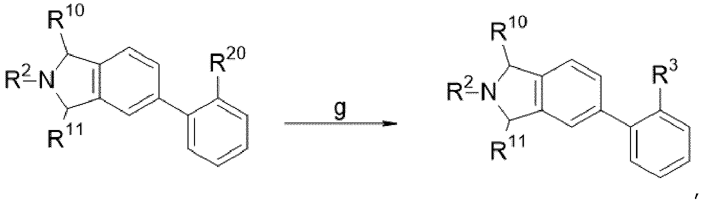
wherein in the case that R²⁰ is -CO₂Me, the reaction step g is conducted in presence of LiOH, or
wherein in the case that R²⁰ is -CN, the reaction step g is conducted in the presence of tributyltin azide,
thus generating a compound according to the second aspect of the invention. In this context, R³ is defined as in the second aspect of the invention.

In preferred aspects of the invention, reaction step g is conducted in the presence of LiOH, and the reaction step g generates a compound selected from the **Compounds 11, 21-28.**

In preferred embodiments of the invention, the reaction step g is conducted in the presence of tributyltin azide, and the reaction step g generates the **Compounds 29-36.**

In preferred embodiments of the invention, the method comprises an additional reaction step h1 of reacting 4-bromophtalimide with phenyl magnesium bromide in presence of DMPU, wherein reaction step h1 is

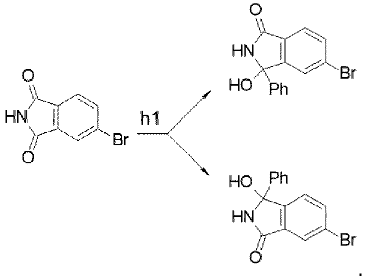

In preferred embodiments of the invention, wherein the reaction step h1 generates **Compounds 133 and/or 134.**

In preferred embodiments of the invention, a reaction product of reaction step h1 that comprises a hydroxyl group is treated in a reaction step h2 with triethylsilane and trifluoroacetic acid, thus reducing said hydroxyl group, wherein reaction step h2 is 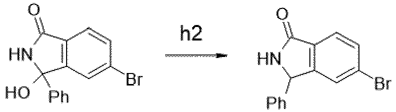 and/or

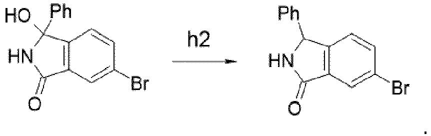

In preferred embodiments of the invention, the reaction step h2 generates **Compounds 135 and/or 136.**

In preferred embodiments of the invention, the method comprises an additional reaction step h3, wherein carbonyl group is reduced by reacting a reaction product of reaction step h2 with a BH3.THF complex, wherein reaction step h3 is 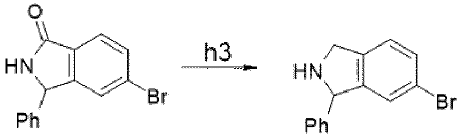 and/or

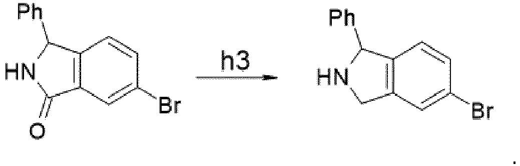

In preferred embodiments of the invention, the reaction step h3 generates the **Compounds 137 and/or 138.**

In preferred embodiments of the invention, the reaction steps h1, h2 and/or h3 are conducted prior to reaction step e1, e2 or e3, preferably prior to reaction step e1.

**In a sixth aspect, the invention pertains to an intermediate,** wherein the intermediate is selected from

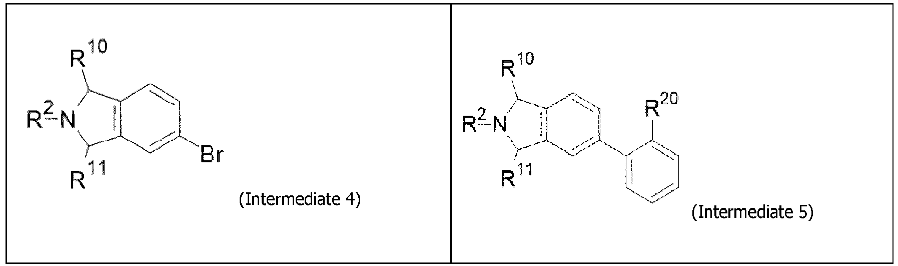

wherein R², R¹⁰, and R¹¹ are defined as in the second aspect of the invention and R²⁰ is -CN or -CO₂Me.

In preferred embodiments of the invention, **Intermediate** 4 is selected from **Compounds 100** to **115.**

In preferred embodiments of the invention, wherein the **Intermediate** 5 is selected from **Compounds 116** to **132.**

**In a seventh aspect, the invention pertains to a compound for use** in the treatment of a disease or condition in a subject, wherein the compound is a compound according to any other aspect of the invention.

In this context the term "treatment" includes the application or administration of a therapeutic agent (such as a compound of the invention) or procedure to a patient in need thereof, with the purpose to cure, heal, alleviate, relieve, alter, remedy, ameliorate, improve, affect or prevent the condition or disease, the symptoms of the condition or disease, or the predisposition toward the condition or disease. Hence, the term "treatment" can include prophylactic treatment of a condition or disease or of the symptoms of a condition or disease.

Preferably, the subject of the invention is an animal. In this context, the term "animal" includes humans. Preferably, the subject is a mammal or bird, most preferably a mammal. Preferably, the mammal is a human. Preferably, the mammal is a non-human primate, dog, cat, sheep, cattle, goat, pig, mouse, rat, rabbit or guinea pig.

In preferred embodiments, the disease or condition is a skin disease, inflammatory disease, a tissue lesion, and/or pain. In preferred embodiments of the invention, the disease is pain or a tissue lesion.

In preferred embodiments of the invention, the disease is a disease associated with a reduced activity of Leukotriene B4 receptor 2 (BLT2). The compounds of the invention do not activate BLT1. For this reason, they are especially useful in treatments that require a selective activation of the BLT2 receptor.
In preferred embodiments of the invention, the compound of the invention acts as a BLT2 agonist. As used herein, the term "BLT2 agonist" means a substance that affects an increase in the amount or rate of BLT2 activity. Preferably, a substance of the invention can act directly, for example, by binding to BLT2 and increasing the amount or rate of BLT2 activity. A BLT2 agonist can also increase the amount or rate of BLT2 activity, for example, by binding to BLT2 in such a way as to enhance or promote interaction of BLT2 with a BLT2 ligand; BLT2 activation may be affected by binding to BLT2 and modifying it, such as inducing a conformational change, or removal or addition of a moiety; and by binding to BLT2 and enhancing its stability.

In preferred embodiments of the invention, the disease or condition is pain. In preferred embodiments of the invention the pain is neuropathic pain such as a chemotherapy induced pain.

The term "pain" as used herein refers to an unpleasant sensation. For example, the subject experiences discomfort, distress or suffering. Various painful conditions are known by the skilled person in the art and are encompassed by the term "pain" as used herein. Such examples include nociceptive pain, non-nociceptive pain, acute pain, chronic pain, neuropathic pain and phantom pain. Furthermore, pain in the context of the invention can be acute pain or chronic pain. In particular preferred embodiments of the invention, the pain is a neuropathic pain.

The term "nociception" as used herein refers to the transduction of noxious or potentially injurious stimuli into a sensation. The term "nociceptive pain" as used herein refers to pain caused by activity in primary sensory pain fibers in the peripheral nervous system. Neurons in the peripheral nervous system that typically respond to noxious or painful stimuli are commonly referred to as nociceptors or nociceptive neurons. Yet further, the nociceptive pathways extend to the somatosensory cortex.

The term "non-nociceptive pain" as used herein refers to pain caused by activity in neurons in the central nervous system. Examples of neurons in the central nervous system that may cause non-nociceptive pain include neurons in the dorsal horn of the spinal cord such as interneurons and projection neurons, or neurons in parts of the brain known to be involved in pain sensation such as the rostral ventromedial medulla (RVM) and the periaqueductal grey (PAG).

The term "acute pain" as used herein refers to pain that is transient in nature or lasting less than 1 month. Acute pain is typically associated with an immediate injurious process such as soft tissue damage, infection, or inflammation, and serves the purpose of notifying the animal of the injurious condition, thus allowing for treatment and prevention of further injury.

The term "chronic pain" as used herein refers to pain that lasts longer than 1 month or beyond the resolution of an acute tissue injury or is recurring or is associated with tissue injury and/or chronic diseases that are expected to continue or progress. Examples of chronic diseases that are expected to continue or progress may include cancer, arthritis, inflammatory disease, chronic wounds, cardiovascular accidents, spinal cord disorders, central nervous system disorder or recovery from surgery.

The term "neuropathy" as used herein refers to any condition or disease that adversely affects the normal functioning of the nervous system. Neuropathies can originate anywhere in the central or peripheral nervous system, but only in some cases does this produce neuropathic pain. The term "neuropathic pain" as used herein refers to pain that result from damage to or abnormal function of the nervous system itself. It may exist independently of any form of tissue injury outside of the nervous system. Examples of conditions that may lead to neuropathic pain include disease (e.g., HIV, Herpes, Diabetes, Cancer, autoimmune disorders), acute trauma (surgery, injury, electric shock), and chronic trauma (repetitive motion disorders, chemical toxicity such as alcohol, chemotherapy, or heavy metals).

The term "phantom pain" as used herein refers to a condition whereby the patient senses pain in a part of the body that is either no longer physically present due to amputation, or is known to be completely insensate due to total peripheral nerve destruction.

The term "hyperalgesia" as used herein refers to an increased sensitivity to nociceptive or painful stimuli. The term "allodynia" as used herein describes a condition whereby normally non-noxious stimuli are perceived as painful. Both hyperalgesia and allodynia can be divided into primary and secondary categories or conditions. Primary hyperalgesia/allodynia is an increase in sensitivity to painful and previously non-painful stimuli in a region of the body that has undergone tissue injury. Secondary hyperalgesia/allodynia is an increase in pain sensitivity globally and requires descending input into the periphery from various pain processing centers in the brain.

In preferred embodiments of the invention, the disease is a skin disease. Preferably, the skin disease is an inflammatory skin disease such as atopic dermatitis, psoriasis, hidradenitis suppurativa, rosacea, vitiligo, acne, and/or exfoliative dermatitis. Preferably, the psoriasis is a pustular psoriasis, inverse psoriasis, a napkin psoriasis, guttate psoriasis, erythrodermic psoriasis, psoriasis in the mouth or seborrheic-like psoriasis. Preferably, the skin disease is a chronic inflammatory skin disease.

In preferred embodiments of the invention, the skin disease is a lesion. In particularly preferred embodiments, the subject is afflicted with a diabetes and the condition or disease is a lesion.

In preferred embodiments, the condition or disease is a tissue lesion. In preferred embodiments, the tissue lesion is an ulcer, a diabetes-related lesion, a lesion of the skin, a tissue lesion, a burn, a sunburn, an aging skin lesion, a lesion due to corneal ulceration, a gastro-intestinal lesion (such as a lesion due to inflammatory gastrointestinal disease), a lesion due to intestinal inflammatory diseases, a lesion due to Crohn's disease, ulcerative colitis, a hemorrhoid, a lesion due to epidermolysis bullosa, a skin blistering wound, a lesion due to psoriasis, a lesion due to seborrheic dermatitis, a lesion due to retinopathy, a lesion from the oral cavity (mucositis), a lesion due to vaginal mucositis, a lesion due to periodontal disease, or a lesion due to surgery. Preferably the lesion is a wound of the skin. In preferred embodiments, the tissue lesion is a lesion due to psoriasis. In preferred embodiments of the invention, the tissue lesion is a skin lesion or gastro-intestinal lesion. In preferred embodiments, the tissue lesion is a wound. Wounds are lesions that result from a cut or superficial damage to the skin or mucous membrane.

The process of wound healing comprises overlapping phases (coagulation phase, inflammatory phase and proliferative/remodelling phase). Cells that are involved in the healing process include platelets, keratinocytes/epithelial cells, fibroblasts/myofibroblasts, different immune cells and endothelial cells.

The most studied form of wound healing is in skin healing. Tissue injury initiates the coagulation process and activates the sympathetic nervous system. The platelets forming the blood clots release signals, mainly PDGF (platelet derived growth factor) and TGF (transforming growth factor) changing the local environment. Injured and stressed cells release alarm signals that initiate the recruitment of immune cells such as neutrophils and monocytes. Within the wounded tissue, the immune cells secrete various chemokines, growth factors like VEGF-A, FGF, and EGF (vascular endothelial growth factor A, fibroblast growth factor, epidermal growth factor), ROS (reactive oxygen species) and matrix digestive enzymes, which change the microenvironment and allow the healing process to enter the proliferative phase where failing and dead tissue is removed by macrophages. Cells from the wound edges, such as fibroblasts and keratinocytes, migrate inwards to the wound centre and cover the wound surface with a layer of collagen and extracellular matrix. The fibroblasts within the wound are then transformed into myofibroblasts expressing contractile a-SMA (a-smooth muscle actin) allowing the wound to contract and finally close. Following wound closure, the afflicted site becomes re-epithelialized by keratinocytes/epithelial cells whereby the integrity of the organ barrier is regained.

BLT2 agonists can accelerate keratinocyte migration by stimulating nuclear factor (NF)-κB signaling, which induces the expression of tumor necrosis factors (TNFs) and metalloproteinases (MMPs). They promote diabetic and non-diabetic wound healing by enhancing keratinocyte migration.

In the context of the invention, the BLT2 receptor preferably refers to the receptor isoform described by the uniprot entry Q9NPC1 (https://www.uniprot.org/uniprotkb/Q9NPC1/entry, dated 19 October 2023). In particular preferred embodiments, the BLT2 receptor comprises the amino acid sequence as disclosed in **Table 1.**

**Table 1. Amino acid sequence BLT2**

| |
|---|
| MSVCYRPPGNETLLSWKTSRATGTAFLLLAALLGLPGNGFVVWSLAGWRPARGRPLAATLVLHLALADGAVLLLTPLFVAFLTRQAWPLGQAGCK AVYYVCALSMYASVLLTGLLSLQRCLAVTRPFLAPRLRSPALARRLLLAVWLAALLLAVPAAVYRHLWRDRVCQLCHPSPVHAAAHLSLETLTAFVL PFGLMLGCYSVTLARLRGARWGSGRHGARVGRLVSAIVLAFGLLWAPYHAVNLLQAVAALAPPEGALAKLGGAGQAARAGTTALAFFSSSVNPVL YVFTAGDLLPRAGPRFLTRLFEGSGEARGGGRSREGTMELRTTPQLKVVGQGRGNGDPGGGMEKDGPEWDL |

In the context of the invention, the BLT1 receptor preferably refers to the receptor isoform described by the uniprot entry Q15722-1 (https://www.uniprot.org/uniprotkb/Q15722/entry, on 19 October 2023). In particular preferred embodiments, the BLT1 receptor comprises the amino acid sequence as disclosed in **Table 2.**

**Table 2. Amino acid sequence BLT1**

| |
|---|
| MNTTSSAAPPSLGVEFISLLAIILLSVALAVGLPGNSFVVWSILKRMQKRSVTALMVLNLALADLAVLLTAPFFLHFLAQGTWSFGLAGCRLCHYVCG VSMYASVLLITAMSLDRSLAVARPFVSQKLRTKAMARRVLAGIWVLSFLLATPVLAYRTVVPWKTNMSLCFPRYPSEGHRAFHLIFEAVTGFLLPFLA VVASYSDIGRRLQARRFRRSRRTGRLVVLIILTFAAFWLPYHVVNLAEAGRALAGQAAGLGLVGKRLSLARNVLIALAFLSSSVNPVLYACAGGGLLR SAGVGFVAKLLEGTGSEASSTRRGGSLGQTARSGPAALEPGPSESLTASSPLKLNELN |

The dosage and concentration of a compound of the invention for treatment may be varied as desired, as further described herein. Depending on the subject and/or condition being treated and on the administration route, the compound can generally be administered in dosages of 0.01 mg to 500 mg /kg body weight, preferably 0.1 to 200 mg/kg body weight, more preferably 1 to 100 kg/mg body weight, more preferably about 5 to 50 mg/kg body weight, most preferably about 10 mg/kg body weight. Preferably, the compound is administered in a dosage of 0.01 mg to 500 mg /kg body weight per day, preferably 0.1 to 200 mg/kg body weight per day, more preferably 1 to 100 kg/mg body weight per day, more preferably about 5 to 50 mg/kg body weight per day, most preferably about 10 mg/kg body weight per day. A typical dosage may be one administration daily, or multiple administrations daily. In the case of a prolonged or controlled-release dosage forms (e.g., patches) the unit dose is designed for administration over a defined period of time.

In preferred embodiments of the invention, the administration of the compound of the invention, includes oral, subcutaneous, intravenous, intranasal, transdermal, transmucosal, topical (e.g., via gels, salves, lotions, creams, etc.), intraperitoneal, intramuscular, intrapulmonar, vaginal, parenteral, rectal, intrathecal, peridural, gastric, systemic and/or intraocular administration. Preferably, the administration of the compound of the invention includes oral, intranasal, subcutaneous and/or topical administration. Even more preferably, the administration of the compound of the invention includes oral and/or subcutaneous administration Even more preferably, the administration of the compound of the invention includes oral administration.

**In an eighth aspect, the invention pertains to a therapeutic composition** comprising a compound of the invention and a pharmaceutically acceptable carrier and/or excipient. In a preferred embodiment, the therapeutic composition is for use in the treatment of a condition or disease, as disclosed herein.

To prepare the present therapeutic composition, a compound of the invention can be mixed with a suitable pharmaceutically acceptable carrier. The therapeutic composition comprising a compound of the invention can be a solid, a half-solid, a solution, suspension, or an emulsion. In preferred embodiments of the invention, the therapeutic composition is in one or more dosage forms. Such a therapeutic composition can be prepared according to methods known to those skilled in the art.

In preferred embodiments of the invention, a compound of the invention is formulated in an oral dosage form (e.g., a liquid, tablet, capsule, gel), an inhalation, a nasal spray, a patch, an absorbing gel, a liquid, suppository, injections, or an I.V. drip. Preferably, the compound of the invention is formulated in a liquid, for example as an oral dosage form or as an injection.

In preferred embodiments, the therapeutic composition comprises a pharmaceutical carrier or vehicle suitable for administration of the compounds provided herein. Such a pharmaceutical carrier or vehicle includes all such carriers known to a person skilled in the art to be suitable for the particular mode of administration. In addition, a compound of the invention can also be administered with other components that do not impair the desired action, or with components that supplement the desired action, or have another action.

Preferably, an oral therapeutic composition includes an inert diluent or an edible carrier. Such an oral therapeutic composition can be enclosed in a gelatin capsule or compressed into a tablet. For the purpose of oral therapeutic administration, the compound of the invention can be incorporated with excipients and used in the form of tablets, troches, or capsules. Oral compositions can also be prepared using a fluid carrier, for example in the form of a mouthwash, wherein the compound in the fluid carrier is applied orally and swished and expectorated or swallowed. Pharmaceutically compatible binding agents, and/or adjuvant materials can be included as part of the therapeutic composition.

Tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring.

The composition of the present invention can be in liquid form. A liquid formulation can comprise, for example, the compound of the invention in water-in-solution and/or suspension form; and a vehicle comprising polyethoxylated castor oil, alcohol and/or a polyoxyethylated sorbitan mono-oleate with or without flavoring. Each dosage form comprises an effective amount of a compound of the invention and can optionally comprise a pharmaceutically inert agent, for example a pharmaceutically inert agent that can be included in pharmaceutical dosage forms for oral administration.

Solutions or suspensions used for parenteral, intradermal, or subcutaneous application can include the following components: a sterile diluent such as water for injection, saline solution, fixed oils, polyethylene glycols, glycerine; propylene glycol or other synthetic solvents; anti-bacterial agents such as benzyl alcohol or methyl parabens; antioxidants such as ascorbic acid or sodium bisulfate; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating the compound of the invention in the required amount in an appropriate solvent with one or a combination of ingredients enumerated above, as required, followed by filtered sterilization.

A composition of the present invention can also be administered in injection-ready stable liquids for injection or I.V. drip. For example, saline or other injection-ready liquid can be mixed with a compound of the invention.

For administration by inhalation, a compound is delivered in the form of an aerosol spray from pressured container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of a nasal spray or suppository.

The compositions of the present invention can also be administered topically to the skin of a subject. The agents can be mixed with a pharmaceutically acceptable carrier or a base which is suitable for topical application to skin to form a dermatological composition, wherein the pharmaceutical composition is formulated into an ointment, salve, gel, or cream as generally known in the art. Suitable examples of carrier or base include, but not limited to, water, a glycol, an alcohol, a lotion, cream, gel, emulsion, and spray. A composition for topical administration comprising a compound of the invention can be integrated into a topical dressing, medicated tape, dermal patch absorbing gel and cleansing tissues.

The compositions of the present invention can also be administered in a suppository form, comprising an outer layer containing the composition in a suppository base. The suppository base may, for example, be any conventional suppository base material such as glycogelatin, polyethylene glycol, fractionated palm kernel oil, or one or more natural, synthetic or semisynthetic hard fats such as cocoa butter.

In a further embodiment of the present invention, the agent is in a controlled-release form. Extended or controlled-release formulations can comprise one or more combination of excipients that slow the release of the agents of the invention by coating or temporarily bonding or decreasing their solubility of the active agents. Examples of these excipients include cellulose ethers such as hydroxypropylmethylcellulose (e.g., Methocel K4M), polyvinylacetate-based excipients such as, e.g., Kollidon SR, and polymers and copolymers based on methacrylates and methacrylic acid such as, e.g., Eudragit NE 30D. In one embodiment of the invention, compositions comprise one or more carriers that protect the agents against rapid elimination from the body, such as time-release formulations or coatings. Such carriers include controlled-release formulations, including, for example, microencapsulated delivery systems.

In preferred embodiments, the compound is formulated for oral administration or subcutaneous administration. In preferred embodiments, the compound is formulated for oral administration.

In preferred embodiments, the compound for use in the treatment of a disease is mixed with a suitable pharmaceutically acceptable carrier or excipient.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES

The figures show:
**Figure 1****:** Chemical structures of **(A)** LTB4, **(B)** 12-HHT **(C)** Cay10583 and **(D)** Irbesartan.
**Figure 2****:** Reaction steps of the synthesis of the spiro **Compounds 10** and **12-20.** In the first step (GP1) a nucleophilic aromatic substitution between the corresponding spiroamines and 2-fluorobenzonitrile is condcuted. Next, after Boc deprotection with trifluoroacetic acid (GP2), the other amine group is acylated with valeryl chloride in presence of triethylamine to obtain Compounds 90-99 (GP3). The final tetrazole group is then constructed by reaction with tributyltin azide in p-xylene at reflux temperature (GP4).

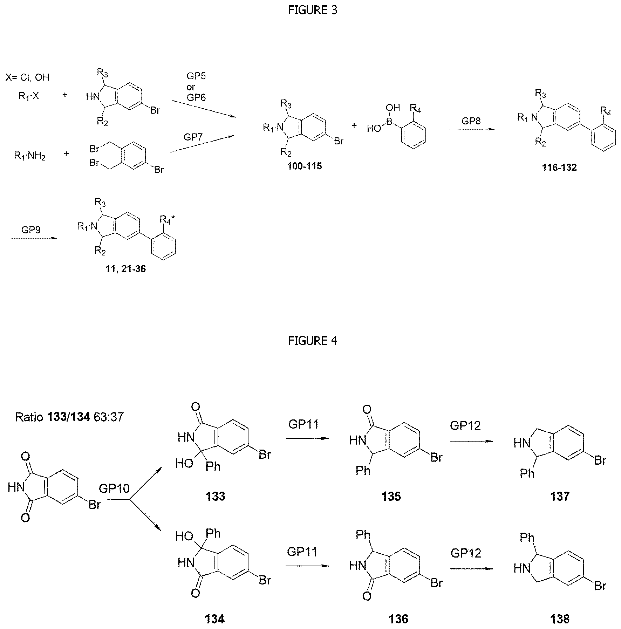

**Figure 3****:** Reaction steps of the synthesis of the isoindoline **Compounds 11** and **21-36.** N-acylated isoindoline intermediates is first synthesized by coupling of the corresponding acid chloride or carboxylic acids and 5-bromoisoindoline (GP5 or GP6). On the other hand, for heterocycle-containing isoindolines, the core scaffold is built by reaction of 4-bromo-1,2-bis(bromomethyl)benzene with the corresponding amino-heterocylcles in presence of sodium hydride (GP7). In the next step intermediates 116-132 are prepared by Suzuki coupling reaction of compounds 100-115 with 2-cyanophenylboronic acid or 2-methoxycarbonylphenylboronic acid catalysed by Pd(PPh3)4 (GP8). The final compounds are then obtained either by hydrolysis of the ester group or by tetrazole ring formation with tributyltin azide (GP9).
**Figure 4****:** Synthesis of precursor compounds for isoindoline derivative synthesis. In a first step, 4-bromophtalimide is reacted with phenyl magnesium bromide in presence of DMPU (GP10). Next, the hydroxyl group is reduced by treatment with triethylsilane and trifluoroacetic acid (GP11). Afterwards, the missing carbonyl group is reduced by reaction of the resulting intermediates **Compound 135** and **Compound 136** with the BH₃.THF complex to yield the desired precursors **Compound 137** and **Compound 138** (GP12).

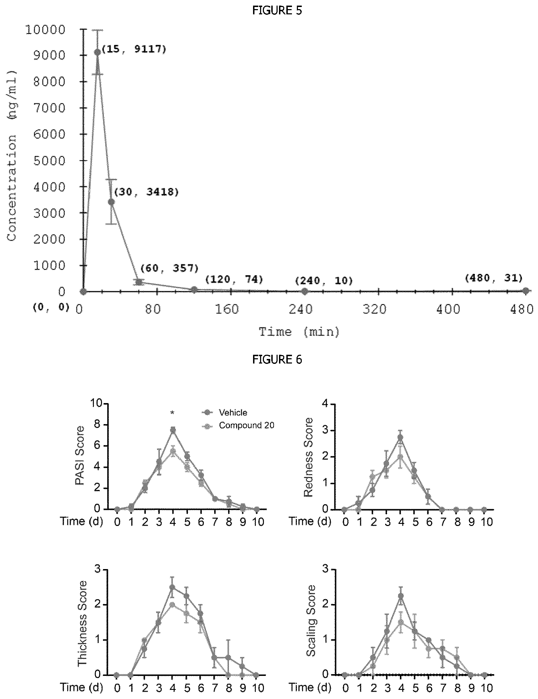

**Figure 5****:** Plasma concentration of **Compound 20** *sc* at 10 mg/kg in dependency of time. (Mean ±SD, n=3).
**Figure 6****: Compound 20** suppresses disease development in the IMQ psoriasiform dermatitis mouse model. Mice were treated daily with 62.5 mg IMQ topically applied on the backskin for six consecutive days (starting at day 1). At day 1, 3 and 5, **Compound 20** (10 mg/kg) or vehicle (PBS) was administered s.c.. Cumulative Psoriasis Area Severity Index (PASI) scores, being the sum of the individual scores for redness, thickness and scaling were analyzed daily until 10 days after initial IMQ application. Data are expressed as mean ± SEM (standard error of the mean). *p ≤ 0.05 versus IMQ control for four individual animals. Multiple t-tests with FDR correction.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Synthesis of Compounds of the invention

Chemicals for the synthesis of the compounds of the present invention were purchased from Acros Organics (Geel, Belgium), Alfa Aesar GmbH & Co KG (Karlsruhe, Germany), BLD Pharmatech GmbH (Kaiserslautern, Germany), Sigma-Aldrich Chemie GmbH (Steinheim, Germany) and TCI Deutschland (Eschborn, Germany).

Reactions were monitored via thin layer chromatography (TLC) using ALUGRAM from Merck (Darmstadt, Germany). To record NMR-spectra, compounds were dissolved in DMSO*-d*₆ or CDCl₃ and measured on DPX250, Avance 300 and Avance 400 from Bruker Corporation (Massachusetts, USA) using tetramethylsilane as an internal standard. All chemical shift values are reported in ppm, the multiplicity of the signals assigned as follows: s (singlet), d (duplet), t (triplet) and m (multiplet). Mass spectrometry analysis was performed in positive ion mode by electrospray-ionization (ESI) on a LCMS-2020 single quadrupole MS from Shimadzu (Duisburg, Deutschland). Precision mass was measured using MALDI Orbitrap XL from Life Technologies GmbH (Darmstadt, Germany). For purity estimation of the synthesized compounds, a reverse phase high-performance liquid chromatography (RP-HPLC) was performed using the Luna 10 µm C18(2) 100 Å, LC Column 250 x 4.6 mm from Phenomenex LTD (Aschaffenburg, Germany) and the analysis was conducted using the Shimadzu prominence module from Shimadzu. Acetonitrile and aqueous formic acid 0.1% were used as eluents. The established method for purity determination was initiated with 90% water (0.1% formic acid), then a linear gradient from 90% to 5% water (0.1% formic acid) for 13 min was chosen, finally additional 7 min 5% water (0.1% formic acid). The flow rate was adjusted to 1.0 mL/min and the UV-vis detection occurred at 254 nm and 280 nm, respectively. Purity of all tested compounds was determined higher than 95%.

Pure compounds (≥95% purity) were obtained after purification by reverse phase HPLC or recrystallization. The structures of all final products were confirmed by ¹H, ¹³C NMR spectroscopy and HPLC analysis coupled to electrospray ionization mass spectrometry (HPLC/ESI-MS) which was also used to determine the purity. Some amide products presented a mixture of conformers in the NMR. In these cases, below only the ¹H NMR of the most abundant conformer is described. Additionally, HRMS was also determined.

### Synthesis of spiro Compounds 10 and 12-20

The synthesis of spiro-compounds **10** and **12-20** was accomplished in a 4-step synthesis as shown in **FIG. 2****.**

**GP1. Nucleophilic aromatic substitution:** The corresponding spiro-amine (1.5 eq), 2-fluorobenzonitrile (1 eq) and potassium carbonate (2 eq) were dissolved in DMF and stirred at 90 °C overnight. After this time, solvents were evaporated at reduced pressure and the residue taken up in water/EtOAc. The aqueous phase was extracted with EtOAc (3x). The combined organic layers were dried over magnesium sulfate, filtered and evaporated. The crude product was purified by flash chromatography.

**GP2. BOC deprotection:** Trifluoroacetic acid was added to a solution of the substrate in dichloromethane (TFA/DCM 1:2) at 0°C. The resulting solution was allowed to stir at rt for 1h. Then, the solvents were directly evaporated under reduced pressure and the residue used in the next step without further purification.

**GP3. Acylation reaction:** Triethylamine (3.0 eq) and the corresponding acid chloride (1.5 eq) were added to the corresponding amine as trifluoroacetic acid salt (1.0 eq) in dichloromethane at 0°C under Ar. The reaction mixture was then allowed to stir at rt for 3h. The reaction mixture was diluted with dichloromethane and washed with a saturated aqueous ammonium chloride solution and brine. The organic phase was dried over magnesium sulfate, filtered and evaporated. The crude product was purified by flash chromatography.

**GP4. Tetrazole ring formation:** To a solution of the substrate (1 eq) in p-xylene was added tributyltin azide (6.0 eq) and the resulting solution was heated at 150 °C overnight. Solvents were evaporated and the residue taken up in dichloromethane. The organic phase was extracted with aqueous NaOH 2M (2x). The aqueous phases were collected and acidified with hydrochloric acid until pH 3-4. Then, the aqueous phase was extracted with ethyl acetate (3x). The combined organic layers were dried over magnesium sulfate, filtered and evaporated. The crude compound was purified by HPLC.

### In these reactions, the following compounds were synthesized.

**tert-Butyl 7-(2-cyanophenyl)-2,7-diazaspiro[4.4]nonane-2-carboxylate (Compound 70):** Synthesized according to GP1 from *tert*-butyl 2,7-diazaspiro[4,4]nonane-2-carboxylate (340 mg, 1.5 mmol), 2-fluorobenzonitrile (109 µL, 1.0 mmol) and potassium carbonate (279 mg, 2.0 mmol) in DMF (6.0 mL). Purification by flash chromatography (hexane:EtOAc 8:2 to 7:3) yielded (326 mg, 99%) of **Compound 70.** ¹H NMR (250 MHz, CDCl₃) δ 7.45 (dd, *J* = 7.8, 1.7 Hz, 1H), 7.38-7.30 (m, 1H), 6.73-6.67 (m, 1H), 6.64 (d, *J* = 8.6 Hz, 1H), 3.73 (t, *J* = 6.7 Hz, 2H), 3.54 (s, 2H), 3.50-3.43 (m, 2H), 3.36 (d, *J* = 10.9 Hz, 1H), 3.30 (d, *J=* 10.8 Hz, 1H), 2.05-1.85 (m, 4H), 1.47 (s, 9H).

***tert*-Butyl 9-(2-cyanophenyl)-3,9-diazaspiro[5.5]undecane-3-carboxylate (Compound 71):** Synthesized according to GP1 from *tert*-butyl 3,9-diazaspiro[5,5]undecane-3-carboxylate (350 mg, 1.37 mmol), 2-fluorobenzonitrile (99.7 µL, 0.913 mmol) and potassium carbonate (256 mg, 1.83 mmol) in DMF (6.0 mL). Purification by flash chromatography (hexane:EtOAc 8:2 to 7:3) yielded (250 mg, 77%) of **Compound 71.** ¹H NMR (250 MHz, CDCl₃) δ 7.54 (dd, *J* = 7.8, 1.7 Hz, 1H), 7.49-7.42 (m, 1H), 7.03-6.93 (m, 2H), 3.44-3.39 (m, 4H), 3.21-3.16 (m, 4H), 1.75-1.71 (m, 4H), 1.54-1.49 (m, 4H), 1.46 (s, 9H).

***tert*-Butyl 2-(2-cyanophenyl)-2,8-diazaspiro[4.5]decane-8-carboxylate (Compound 72):** Synthesized according to GP1 from 8-Boc-2,8-diazaspiro[4,5]decane (250 mg, 1.04 mmol), 2-fluorobenzonitrile (75.3 µL, 0.693 mmol) and potassium carbonate (194 mg, 1.39 mmol) in DMF (6.0 mL). Purification by flash chromatography (hexane:EtOAc 8:2 to 7:3) yielded (186 mg, 79%) of **Compound 72.** ¹H NMR (250 MHz, CDCl₃) δ 7.44 (dd, *J* = 7.8, 1.7 Hz, 1H), 7.36-7.29 (m, 1H), 6.70-6.60 (m, 2H), 3.70 (t, *J=* 7.0 Hz, 2H), 3.53-3.34 (m, 6H), 1.89 (t, *J=* 7.0 Hz, 2H), 1.64-1.55 (m, 4H), 1.46 (s, 9H).

***tert*-Butyl 2-(2-cyanophenyl)-2,7-diazaspiro[4.5]decane-7-carboxylate (Compound 73):** Synthesized according to GP1 from *tert-*butyl 2,7-diazaspiro[4,5]decane-7-carboxylate (250 mg, 1.04 mmol), 2-fluorobenzonitrile (75.3 µL, 0.693 mmol) and potassium carbonate (194 mg, 1.39 mmol) in DMF (6.0 mL). Purification by flash chromatography (hexane:EtOAc 9:1 to 7:3) yielded (164 mg, 70%) of **Compound 73.** ¹H NMR (250 MHz, CDCl₃) δ 7.43 (dd, *J* = 7.8, 1.7 Hz, 1H), 7.35-7.28 (m, 1H), 6.69-6.58 (m, 2H), 3.85-3.70 (m, 1H), 3.67-3.58 (m, 1H), 3.51-3.20 (m, 6H), 1.99-1.90 (m, 1H), 1.79-1.54 (m, 6H), 1.37 (br s, 9H).

***tert*-Butyl 8-(2-cyanophenyl)-2,8-diazaspiro[4.5]decane-2-carboxylate (Compound 74):** Synthesized according to GP1 from *tert*-butyl 2,8-diazaspiro[4,5]decane-2-carboxylate (250 mg, 1.04 mmol), 2-fluorobenzonitrile (75.3 µL, 0.693 mmol) and potassium carbonate (194 mg, 1.39 mmol) in DMF (6.0 mL). Purification by flash chromatography (hexane:EtOAc 8:2 to 6:4) yielded (125 mg, 53%) of **Compound 74.** ¹H NMR (250 MHz, CDCl₃) δ 7.55 (dd, *J* = 7.9, 1.5 Hz, 1H), 7.50-7.43 (m, 1H), 7.02-6.95 (m, 2H), 3.48-3.36 (m, 2H), 3.32-3.00 (m, 6H), 1.81-1.75 (m, 6H), 1.47 (br s, 9H).

***tert*-Butyl 6-(2-cyanophenyl)-2,6-diazaspiro[3.4]octane-2-carboxylate (Compound 75):** Synthesized according to GP1 from *tert*-butyl 2,6-diazaspiro[3,4]octane-2-carboxylate (250 mg, 1.18 mmol), 2-fluorobenzonitrile (86.4 µL, 0.787 mmol) and potassium carbonate (219 mg, 1.57 mmol) in DMF (6.0 mL). Purification by flash chromatography (hexane:EtOAc 7:3) yielded (224 mg, 91%) of **Compound 75.** ¹H NMR (250 MHz, CDCl₃) δ 7.44 (dd, *J* = 7.9, 1.5 Hz, 1H), 7.37-7.30 (m, 1H), 6.73-6.67 (m, 1H), 6.61 (d, *J* = 8.6 Hz, 1H), 3.94 (d, *J* = 8.7 Hz, 2H), 3.86 (d, *J* = 8.7 Hz, 2H), 3.74 (s, 2H), 3.65 (t, *J* = 6.8 Hz, 2H), 2.18 (t, *J* = 6.8 Hz, 2H), 1.44 (br s, 9H).

***tert*-Butyl 7-(2-cyanophenyl)-2,7-diazaspiro[3.5]nonane-2-carboxylate (Compound 76):** Synthesized according to GP1 from *tert*-butyl 2,7-diazaspiro[3,4]nonane-2-carboxylate (250 mg, 1.10 mmol), 2-fluorobenzonitrile (79.9 µL, 0.733 mmol) and potassium carbonate (205 mg, 1.47 mmol) in DMF (6.0 mL). Purification by flash chromatography (hexane:EtOAc 7:3) yielded (119 mg, 50%) of **Compound 76.** ¹H NMR (250 MHz, CDCl₃) δ 7.55 (dd, *J* = 7.9, 1.8 Hz, 1H), 7.50-7.43 (m, 1H), 7.02-6.96 (m, 2H), 3.69 (s, 4H), 3.11 (t, *J* = 5.5 Hz, 4H), 1.96 (t, *J* = 5.5 Hz, 4H), 1.45 (br s, 9H).

**tert-Butyl 2-(2-cyanophenyl)-2,7-diazaspiro[3.5]nonane-7-carboxylate (Compound 77):** Synthesized according to GP1 from *tert*-butyl 2,7-diazaspiro[3,5]nonane-7-carboxylate (250 mg, 1.10 mmol), 2-fluorobenzonitrile (79.9 µL, 0.733 mmol) and potassium carbonate (205 mg, 1.47 mmol) in DMF (6.0 mL). Purification by flash chromatography (hexane:EtOAc 7:3) yielded (194 mg, 81%) of **Compound 77.** ¹H NMR (250 MHz, CDCl₃) δ 7.38 (dd, *J* = 7.8, 1.6 Hz, 1H), 7.36-7.29 (m, 1H), 6.72-6.66 (m, 1H), 6.41 (d, *J* = 8.4 Hz, 1H), 3.92 (s, 4H), 3.43-3.38 (m, 4H), 1.80-1.76 (m, 4H), 1.46 (br s, 9H).

***tert*-Butyl 2-(2-cyanophenyl)-2,6-diazaspiro[3.4]octane-6-carboxylate (Compound 78):** Synthesized according to GP1 from *tert*-butyl 2,6-diazaspiro[3,4]octane-6-carboxylate (250 mg, 1.18 mmol), 2-fluorobenzonitrile (86.4 µL, 0.787 mmol) and potassium carbonate (219 mg, 1.57 mmol) in DMF (6.0 mL). Purification by flash chromatography (hexane:EtOAc 7:3) yielded (147 mg, 59%) of **Compound 78.** ¹H NMR (250 MHz, CDCl₃) δ 7.42-7.31 (m, 2H), 6.72 (t, *J* = 7.7 Hz, 1H), 6.42 (d, *J* = 8.4 Hz, 1H), 4.12 (d, *J* = 7.8 Hz, 2H), 4.08 (d, *J* = 7.9 Hz, 2H), 3.54 (s, 2H), 3.43 (t, *J=* 7.0 Hz, 2H), 2.13 (t, *J=* 7.0 Hz, 2H), 1.47 (br s, 9H).

**tert-Butyl 6-(2-cyanophenyl)-2,6-diazaspiro[3.3]heptane-2-carboxylate (Compound 79):** Synthesized according to GP1 from *tert*-butyl 2,6-Diazaspiro[3.3]heptane-2-carboxylate (200 mg, 1.01 mmol), 2-fluorobenzonitrile (73.1 µL, 0.670 mmol) and potassium carbonate (187 mg, 1.34 mmol) in DMF (5.0 mL). Purification by flash chromatography (hexane:EtOAc 7:3) yielded (200 mg, 99%) of **Compound 79.** ¹H NMR (250 MHz, CDCl₃) δ 7.42-7.31 (m, 2H), 6.74 (t, *J* = 7.7 Hz, 1H), 6.42 (d, *J* = 8.4 Hz, 1H), 4.28 (s, 4H), 4.11 (s, 4H), 1.45 (br s, 9H).

**2-(2,7-Diazaspiro[4.4]nonan-2-yl)benzonitrile (Compound 80):** Synthesized according to GP2 from **Compound 70** (326 mg, 1.0 mmol) and trifluoroacetic acid (2.0 mL) in dichloromethane (4.0 mL). The crude product (341 mg, quantitative) was obtained as a trifluroroacetic acid salt. ¹H NMR (250 MHz, CDCl₃) δ 9.32 (br s, 1H), 7.45 (dd, *J* = 7.8, 1.7 Hz, 1H), 7.41-7.34 (m, 1H), 7.11 (br s, 1H), 6.75 (t, *J* =7.8 Hz, 1H), 6.64 (d, *J* = 8.6 Hz, 1H), 3.75 (t, *J* = 6.7 Hz, 2H), 3.64 (d, *J* = 10.0 Hz, 1H), 3.60 (d, *J* = 10.0 Hz, 1H), 3.55-3.46 (m, 2H), 3.39-3.32 (m, 2H), 2.24-2.02 (m, 4H).

**2-(3,9-Diazaspiro[5.5]undecan-3-yl)benzonitrile (Compound 81):** Synthesized according to GP2 from **Compound 71** (250 mg, 0.703 mmol) and trifluoroacetic acid (2.0 mL) in dichloromethane (4.0 mL). The crude product (280 mg, quantitative) was obtained as a trifluroroacetic acid salt. ¹H NMR (250 MHz, CDCl₃) δ 10.25 (br s, 1H), 8.22 (br s, 1H), 7.58 (d, *J* = 7.8 Hz, 1H), 7.51 (td, *J* = 8.1, 1.3 Hz, 1H), 7.08-7.02 (m, 2H), 3.24-3.20 (m, 8H), 1.85-1.81 (m, 8H).

**2-(2,8-Diazaspiro[4.5]decan-2-yl)benzonitrile (Compound 82):** Synthesized according to GP2 from **Compound 72** (186 mg, 0.545 mmol) and trifluoroacetic acid (2.0 mL) in dichloromethane (4.0 mL). The crude product (194 mg, quantitative) was obtained as a trifluroroacetic acid salt. ¹H NMR (250 MHz, CDCl₃) δ 10.00 (br s, 1H), 8.08 (br s, 1H), 7.47 (dd, *J* = 7.8, 1.7 Hz, 1H), 7.42-7.35 (m, 1H), 6.76 (td, *J* = 7.8, 0.8 Hz, 1H), 6.67 (d, *J* = 8.5 Hz, 1H), 3.74 (t, *J* = 7.0 Hz, 2H), 3.54 (s, 2H), 3.32 (br s, 4H), 2.03-1.92 (m, 6H).

**2-(2,7-Diazaspiro[4.5]decan-2-yl)benzonitrile (Compound 83):** Synthesized according to GP2 from **Compound 73** (164 mg, 0.480 mmol) and trifluoroacetic acid (2.0 mL) in dichloromethane (4.0 mL). The crude product (171 mg, quantitative) was obtained as a trifluroroacetic acid salt. ¹H NMR (250 MHz, CDCl₃) δ 8.15 (br s, 2H), 7.47 (dd, *J* = 7.8, 1.7 Hz, 1H), 7.42-7.35 (m, 1H), 6.76 (td, *J* = 7.8, 0.8 Hz, 1H), 6.66 (d, *J* = 8.5 Hz, 1H), 3.71 (t, *J* = 7.1 Hz, 2H), 3.62 (d, *J* = 10.3 Hz, 1H), 3.56 (d, *J* = 10.3 Hz, 1H), 3.29-3.20 (m, 4H), 2.11-1.90 (m, 4H), 1.85-1.68 (m, 2H).

**2-(2,8-Diazaspiro[4.5]decan-8-yl)benzonitrile (Compound 84):** Synthesized according to GP2 from **Compound 74** (125 mg, 0.366 mmol) and trifluoroacetic acid (2.0 mL) in dichloromethane (4.0 mL). The crude product (130 mg, quantitative) was obtained as a trifluroroacetic acid salt. ¹H NMR (250 MHz, CDCl₃) δ 8.59 (br s, 1H), 8.37 (br s, 1H), 7.59 (dd, *J* = 7.8, 1.7 Hz, 1H), 7.52 (td, *J* = 8.2, 1.7 Hz, 1H), 7.10-7.04 (m, 1H), 3.57-3.46 (m, 2H), 3.32-3.10 (m, 6H), 2.05 (t, *J* = 7.5 Hz, 2H), 1.92 (t, *J* = 5.5 Hz, 4H).

**2-(2,6-Diazaspiro[3.4]octan-6-yl)benzonitrile (Compound 85):** Synthesized according to GP2 from **Compound 75** (224 mg, 0.715 mmol) and trifluoroacetic acid (2.5 mL) in dichloromethane (5.0 mL). The crude product (265 mg, quantitative) was obtained as a trifluroroacetic acid salt. ¹H NMR (250 MHz, DMSO-*d*₆) δ 8.69 (br s, 1H), 8.54 (br s, 1H), 7.51 (dd, *J* = 7.9, 1.5 Hz, 1H), 7.47-7.40 (m, 1H), 6.77-6.72 (m, 2H), 4.10-3.89 (m, 4H), 3.77 (s, 2H), 3.54 (t, *J* = 6.8 Hz, 2H), 2.24 (t, *J* = 6.8 Hz, 2H).

**2-(2,7-Diazaspiro[3.5]nonan-7-yl)benzonitrile (Compound 86):** Synthesized according to GP2 from **Compound 76** (119 mg, 0.363 mmol) and trifluoroacetic acid (2.0 mL) in dichloromethane (4.0 mL). The crude product (123 mg, quantitative) was obtained as a trifluroroacetic acid salt. ¹H NMR (250 MHz, DMSO-*d*₆) δ 8.58 (br s, 2H), 7.67 (dd, *J* = 7.7, 1.6 Hz, 1H), 7.61-7.54 (m, 1H), 7.14 (d, *J* = 8.2 Hz, 1H), 7.07 (td, *J* = 7.5, 0.7 Hz, 1H), 3.77 (t, *J* = 6.3 Hz, 4H), 3.06 (t, *J* = 5.5 Hz, 4H), 1.93 (t, *J* = 5.5 Hz, 4H).

**2-(2,7-Diazaspiro[3.5]nonan-2-yl)benzonitrile (Compound 87):** Synthesized according to GP2 from **Compound 77** (194 mg, 0.592 mmol) and trifluoroacetic acid (2.0 mL) in dichloromethane (4.0 mL). The crude product was taken up in a saturated sodium bicarbonate solution and extracted with dichloromethane (3x). The combined organic layers were dried over magnesium sulfate, filtered and evaporated to yield 130 mg (97 %) of **Compound 87** obtained as a free base. ¹H NMR (250 MHz, CDCl₃) δ 7.38 (dd, *J* = 7.8, 1.6 Hz, 1H), 7.36-7.29 (m, 1H), 6.68 (t, *J* = 7.7 Hz, 1H), 6.40 (d, *J* = 8.4 Hz, 1H), 3.92 (s, 4H), 3.43 (br s, 1H), 2.91-2.87 (m, 4H), 1.89-1.84 (m, 4H).

**2-(2,6-Diazaspiro[3.4]octan-2-yl)benzonitrile (Compound 88):** Synthesized according to GP2 from **Compound 78** (144 mg, 0.459 mmol) and trifluoroacetic acid (2.0 mL) in dichloromethane (4.0 mL). The crude product (150 mg, quantitative) was obtained as a trifluroroacetic acid salt. ¹H NMR (250 MHz, CDCl₃) δ 10.03 (br s, 2H), 7.33-7.25 (m, 2H), 6.69 (td, *J* = 7.6, 0.9 Hz, 1H), 6.38 (d, *J* = 8.2 Hz, 1H), 4.13 (d, *J* = 8.1 Hz, 2H), 4.07 (d, *J* = 8.1 Hz, 2H), 3.42 (br s, 2H), 3.31 (br s, 2H), 2.24 (t, *J* = 7.4 Hz, 2H).

**2-(2,6-Diazaspiro[3.3]heptan-2-yl)benzonitrile (Compound 89):** A solution of **Compound 79** (200 mg, 0.670 mmol) and formic acid (2.5 mL) was stirred at rt overnight. The resulting solution was evaporated and the crude product (165 mg, quantitative) was obtained as formic acid salt. ¹H NMR (250 MHz, CDCl₃) δ 8.88 (br s, 2H), 8.33 (m, 1H), 7-39-7.32 (m, 2H), 6.75 (td, *J* = 7.6, 0.9 Hz, 1H), 6.43 (d, *J* = 8.2 Hz, 1H), 4.33 (s, 4H), 4.31 (s, 4H).

**2-(7-Pentanoyl-2,7-diazaspiro[4.4]nonan-2-yl)benzonitrile (Compound 90):** Synthesized according to GP3 from **Compound 80** (341 mg, 1.0 mmol), valeryl chloride (183 µL, 1.5 mmol) and triethylamine (422 µL, 3.0 mmol) in dichloromethane (5.0 mL). The crude product was purified by flash chromatography (hexane:EtOAc 6:4 to 4:6) yielded (311 mg, 99%) of **Compound 90.** ¹H NMR (250 MHz, CDCl₃) δ 9.32 (br s, 1H), 7.45 (dd, *J* = 7.8, 1.4 Hz, 1H), 7.38-7.31 (m, 1H), 6.71 (t, *J* =7.8 Hz, 1H), 6.62 (d, *J=* 8.6 Hz, 1H), 3.79-3.67 (m, 2H), 3.62-3.40 (m, 6H), 2.27 (t, *J* = 7.8 Hz, 2H), 2.06-1.96 (m, 4H), 1.70-1.57 (m, 2H), 1.44-1.29 (m, 2H), 0.92 (t, *J* = 7.3 Hz, 3H).

**2-(9-Pentanoyl-3,9-diazaspiro[5.5]undecan-3-yl)benzonitrile (Compound 91):** Synthesized according to GP3 from **Compound 81** (280 mg, 0.703 mmol), valeryl chloride (128 µL, 1.05 mmol) and triethylamine (295 µL, 2.10 mmol) in dichloromethane (4.0 mL). The crude product was purified by flash chromatography (hexane:EtOAc 6:4 to 4:6) yielded (142 mg, 60%) of **Compound 91.** ¹H NMR (250 MHz, CDCl₃) δ 7.55 (dd, *J* = 7.8, 1.4 Hz, 1H), 7.50-7.43 (m, 1H), 7.01 (d, *J* = 8.3 Hz, 1H), 6.98 (td, *J* = 7.5, 0.9 Hz, 1H), 3.60 (t, *J* = 5.2 Hz, 2H), 3.44 (t, *J* = 5.8 Hz, 2H), 3.22-3.15 (m, 4H), 2.33 (t, *J* = 8.0 Hz, 2H), 1.75 (t, *J* = 5.7 Hz, 4H), 1.67-1.60 (m, 2H), 1.59-1.52 (m, 4H), 1.45-1.32 (m, 2H), 0.93 (t, *J* = 7.3 Hz, 3H).

**2-(8-Pentanoyl-2,8-diazaspiro[4.5]decan-2-yl)benzonitrile (Compound 92):** Synthesized according to GP3 from **Compound 82** (194 mg, 0.545 mmol), valeryl chloride (99.6 µL, 0.818 mmol) and triethylamine (230 µL, 1.64 mmol) in dichloromethane (4.0 mL). The crude product was purified by flash chromatography (hexane:EtOAc 6:4 to 4:6) yielded (149 mg, 84%) of **Compound 92.** ¹H NMR (250 MHz, CDCl₃) δ 7.45 (dd, *J* = 7.8, 1.7 Hz, 1H), 7.37-7.31 (m, 1H), 6.69 (td, *J* = 7.8, 0.8 Hz, 1H), 6.63 (d, *J* = 8.5 Hz, 1H), 3.71 (t, *J* = 7.0 Hz, 2H), 3.55-3.45 (m, 6H), 2.34 (t, *J* = 7.9 Hz, 2H), 1.91 (t, *J* = 7.0 Hz, 2H). 1.68-1.65 (m, 6H), 1.44-1.30 (m, 2H), 0.93 (t, *J* = 7.3 Hz, 3H).

**2-(7-Pentanoyl-2,7-diazaspiro[4.5]decan-2-yl)benzonitrile (Compound 93):** Synthesized according to GP3 from **Compound 83** (171 mg, 0.480 mmol), valeryl chloride (87.7 µL, 0.720 mmol) and triethylamine (202 µL, 1.44 mmol) in dichloromethane (4.0 mL). The crude product was purified by flash chromatography (hexane:EtOAc 6:4 to 4:6) yielded (151 mg, 97%) of **Compound 93.** ¹H NMR (250 MHz, CDCl₃) δ 7.47-7.26 (m, 2H), 6.74-6.59 (m, 2H), 3.93-3.18 (m, 8H), 2.36 (t, *J* = 7.8 Hz, 2H), 1.99-1.33 (m, 10H), 0.93 (t, *J* = 7.3 Hz, 3H).

**2-(2-Pentanoyl-2,8-diazaspiro[4.5]decan-8-yl)benzonitrile (Compound 94):** Synthesized according to GP3 from **Compound 84** (130 mg, 0.366 mmol), valeryl chloride (58.0 µL, 0.476 mmol) and triethylamine (118 µL, 0.842 mmol) in dichloromethane (4.0 mL). The crude product was purified by flash chromatography (hexane:EtOAc 7:3 to 5:5) yielded (104 mg, 87%) of **94.** ¹H NMR (250 MHz, CDCl₃) δ 7.58-7.53 (m, 1H), 7.51-7.43 (m, 1H), 7.03-6.96 (m, 2H), 3.60-3.50 (m, 2H), 3.43 (s, 1H), 3.36-3.28 (m, 1H), 3.32 (s, 1H), 3.22-3.15 (m, 2H), 3.07-2.97 (m, 1H), 2.27 (t, *J* = 7.5 Hz, 2H), 1.91-1.74 (m, 6H), 1.72-1.58 (m, 2H), 1.45-1.30 (m, 2H), 0.93 (t, *J* = 7.3 Hz, 3H).

**2-(2-Pentanoyl-2,6-diazaspiro[3.4]octan-6-yl)benzonitrile (Compound 95):** Synthesized according to GP3 from **Compound 85** (265 mg, 0.715 mmol), valeryl chloride (113 µL, 0.930 mmol) and triethylamine (230 µL, 1.64 mmol) in dichloromethane (6.0 mL). The crude product was purified by flash chromatography (hexane:EtOAc 5:5 to 2:8) yielded (88.0 mg, 41%) of **Compound 95.** ¹H NMR (250 MHz, CDCl₃) δ 7.46 (dd, *J* = 7.9, 1.5 Hz, 1H), 7.39-7.32 (m, 1H), 6.76-6.69 (m, 1H), 6.63 (d, *J* = 8.6 Hz, 1H), 4.07 (d, *J* = 9.0 Hz, 2H), 4.00 (d, *J* = 9.0 Hz, 2H), 3.77 (s, 2H), 3.68 (t, *J* = 6.8 Hz, 2H), 2.21 (t, *J* = 6.8 Hz, 2H), 2.10 (t, *J* = 7.9 Hz, 2H), 1.67-1.55 (m, 2H), 1.42-1.28 (m, 2H), 0.92 (t, *J* = 7.3 Hz, 3H).

**2-(2-Pentanoyl-2,7-diazaspiro[3.5]nonan-7-yl)benzonitrile (Compound 96):** Synthesized according to GP3 from **Compound 86** (123 mg, 0.363 mmol), valeryl chloride (57.5 µL, 0.472 mmol) and triethylamine (117 µL, 0.835 mmol) in dichloromethane (4.0 mL). The crude product was purified by flash chromatography (hexane:EtOAc 5:5 to 3:7) yielded (62.0 mg, 55%) of **Compound 96.** ¹H NMR (250 MHz, CDCl₃) δ 7.56 (dd, *J* = 7.8, 1.6 Hz, 1H), 7.51-7.44 (m, 1H), 7.04-6.98 (m, 2H), 3.80 (s, 4H), 3.13 (s, 4H), 2.11 (t, *J* = 7.8 Hz, 2H), 1.99 (t, *J* = 5.4 Hz, 4H), 1.67-1.55 (m, 2H), 1.43-1.28 (m, 2H), 0.92 (t, *J* = 7.2 Hz, 3H).

**2-(7-Pentanoyl-2,7-diazaspiro[3.5]nonan-2-yl)benzonitrile (Compound 97):** Synthesized according to GP3 from **Compound 87** (130 mg, 0.572 mmol), valeryl chloride (90.6 µL, 0.744 mmol) and triethylamine (186 µL, 1.32 mmol) in dichloromethane (4.0 mL). The crude product was purified by flash chromatography (hexane:EtOAc 5:5 to 3:7) yielded (123 mg, 69%) of **97.** ¹H NMR (250 MHz, CDCl₃) δ 7.39 (dd, *J* = 7.8, 1.6 Hz, 1H), 7.37-7.30 (m, 1H), 6.74-6.67 (m, 1H), 6.41 (d, *J* = 8.4 Hz, 1H), 3.95 (s, 4H), 3.59 (br s, 2H), 3.45 (br s, 2H), 2.34 (t, *J =* 8.0 Hz, 2H), 1.82 (br s, 4H), 1.68-1.55 (m, 2H), 1.44-1.30 (m, 2H), 0.93 (t, *J* = 7.2 Hz, 3H).

**2-(6-Pentanoyl-2,6-diazaspiro[3.4]octan-2-yl)benzonitrile (Compound 98):** Synthesized according to GP3 from **Compound 88** (150 mg, 0.459 mmol), valeryl chloride (72.3 µL, 0.597 mmol) and triethylamine (149 µL, 1.06 mmol) in dichloromethane (4.0 mL). The crude product was purified by flash chromatography (hexane:EtOAc 8:2 to 5:5) yielded (84.0 mg, 62%) of **Compound 98.** ¹H NMR (250 MHz, CDCl₃) δ 7.43-7.33 (m, 2H), 6.74 (t, *J* = 7.6 Hz, 1H), 6.43 (d, *J* = 8.4 Hz, 1H), 4.12 (s, 4H), 3.68 (s, 2H), 3.56 (t, *J* = 7.0 Hz, 2H), 2.28 (t, *J* = 7.8 Hz, 2H), 2.23 (br s, 2H), 1.71-1.58 (m, 2H), 1.45-1.30 (m, 2H), 0.94 (t, *J* = 7.3 Hz, 3H).

**2-(6-Pentanoyl-2,6-diazaspiro[3.3]heptan-2-yl)benzonitrile (Compound 99):** Synthesized according to GP3 from **Compound 89** (165 mg, 0.670 mmol), valeryl chloride (106 µL, 0.871 mmol) and triethylamine (216 µL, 1.54mmol) in dichloromethane (6.0 mL). The crude product was purified by flash chromatography (hexane:EtOAc 8:2 to 5:5) yielded (62.9 mg, 33%) of **Compound 99.** ¹H NMR (250 MHz, CDCl₃) δ 7.42-7.32 (m, 2H), 6.75 (t, *J* = 7.6 Hz, 1H), 6.43 (d, *J* = 8.4 Hz, 1H), 4.31 (s, 4H), 4.17 (s, 4H), 2.09 (t, *J* = 7.8 Hz, 2H), 1.66-1.53 (m, 2H), 1.42-1.27 (m, 2H), 0.92 (t, *J* = 7.3 Hz, 3H).

**1-(7-(2-(1H-Tetrazol-5-yl)phenyl)-2,7-diazaspiro[4.4]nonan-2-yl)pentan-1-one (Compound 10):** Synthesized according to GP4 from **Compound 90** (311 mg, 1.0 mmol) and tributyltin azide (1.64 mL, 6.0 mmol) in p-xylene (10.0 mL). Purification by preparative HPLC yielded (145 mg, 41%) of **Compound 10.** ¹H NMR (300 MHz, CDCl₃) δ 7.72-7.67 (m, 1H), 7.45-7.39 (m, 1H), 7.13 (d, *J* =8.5 Hz, 1H), 7.05 (t, *J* = 7.3 Hz, 1H), 3.59-3.38 (m, 4H), 3.24-3.15 (m, 2H), 3.10-2.99 (m, 2H), 2.27-2.19 (m, 2H), 2.08-1.81 (m, 4H), 1.61-1.46 (m, 2H), 1.35-1.24 (m, 2H), 0.86 (t, *J* = 7.3 Hz, 3H); R_{f} HPLC: 10.2 Min (13 Min from 10 to 95% MeCN in water (0.1 % formic acid), then 7 min 95% MeCN). 95.0 % purity; HRMS (MALDI): m/z found. 355.2240 [M+H]⁺ (cal. C₁₉H₂₇N₆O⁺ 355.2241).

**1-(9-(2-(1H-Tetrazol-5-yl)phenyl)-3,9-diazaspiro[5.5]undecan-3-yl)pentan-1-one (Compound 12):** Synthesized according to GP4 from **Compound 91** (142 mg, 0.419 mmol) and tributyltin azide (688 µL, 2.51 mmol) in *p*-xylene (6.0 mL). Purification by preparative HPLC yielded (46.5 mg, 29%) of **Compound 12.** ¹H NMR (300 MHz, CDCl₃) δ 8.37 (d, *J* = 7.2 Hz 1H), 7.53 (td, *J* = 7.4, 1.6 Hz, 1H), 7.43 (d, *J* =7.6 Hz, 1H), 7.35 (td, *J* = 7.8, 0.9 Hz, 1H), 3.61 (t, *J* = 5.5 Hz, 2H), 3.46 (t, *J* = 5.7 Hz, 2H), 3.03-2.87 (m, 4H), 2.34 (t, *J* = 7.9 Hz, 2H), 1.82-1.76 (m, 4H), 1.66-1.56 (m, 6H), 1.43-1.30 (m, 2H), 0.92 (t, *J* = 7.3 Hz, 3H); ¹³C NMR (75 MHz, CDCl₃) δ 171.8, 153.8, 151.4, 132.5, 130.1, 126.2, 121.6, 118.8, 49.3, 41.4, 37.1, 36.2, 33.2, 29.6, 27.5, 22.6, 13.9; R_{f} HPLC: 9.2 Min (13 Min from 10 to 95% MeCN in water (0.1 % formic acid), then 7 min 95% MeCN). 99.9 % purity; HRMS (MALDI): m/z found. 383.2545 [M+H]⁺ (cal. C₂₁H₃₁N₆O⁺ 383.2554).

**1-(2-(2-(1H-Tetrazol-5-yl)phenyl)-2,8-diazaspiro[4.5]decan-8-yl)pentan-1-one (Compound 13):** Synthesized according to GP4 from **Compound 92** (149 mg, 0.457 mmol) and tributyltin azide (751 µL, 2.51 mmol) in *p*-xylene (6.0 mL). Purification by preparative HPLC yielded (65.3 mg, 39%) of **Compound 13.** ¹H NMR (300 MHz, CDCl₃) δ 7.57 (d, *J* = 7.7 Hz 1H), 7.40-7.34 (m, 1H), 6.97-6.92 (m, 2H), 3.47 (t, *J* = 5.4 Hz, 2H), 3.42-3.27 (m, 2H), 3.00 (t, *J* = 6.7 Hz, 2H), 2.86 (d, *J* = 9.3 Hz, 1H), 2.79 (d, *J* = 9.4 Hz, 1H), 2.29 (t, *J* = 7.7 Hz, 2H), 1.83-1.67 (m, 2H), 1.59-1.44 (m, 6H), 1.34-1.22 (m, 2H), 0.85 (t, *J* = 7.3 Hz, 3H); ¹³C NMR (75 MHz, CDCl₃) δ 172.2, 155.5, 148.3, 131.70, 131.67, 120.2, 116.2, 113.2, 61.6, 49.8, 43.6, 40.5, 39.3, 36.1, 35.7, 34.8, 33.1, 27.5, 22.5, 13.8; R_{f} HPLC: 10.4 Min (13 Min from 10 to 95% MeCN in water (0.1 % formic acid), then 7 min 95% MeCN). 100.0 % purity; HRMS (MALDI): m/z found. 369.2403 [M+H]⁺ (cal. C₂₀H₂₉N₆O⁺ 369.2397).

**1-(2-(2-(1H-Tetrazol-5-yl)phenyl)-2,7-diazaspiro[4.5]decan-7-yl)pentan-1-one (Compound 14):** Synthesized according to GP4 from **Compound 93** (151 mg, 0.465 mmol) and tributyltin azide (763 µL, 2.79 mmol) in *p*-xylene (6.0 mL). Purification by preparative HPLC yielded (45.1 mg, 26%) of **Compound 14.** ¹H NMR (300 MHz, CDCl₃) δ 7.76 (d, *J* = 7.0 Hz 1H), 7.41 (td, *J=* 7.7, 1.6 Hz, 1H), 7.18 (d, *J* = 8.5 Hz, 1H), 7.13 (t, *J=* 7.5 Hz, 1H), 4.42 (d, *J=* 13.4 Hz, 1H), 3.85 (d, *J=* 13.3 Hz, 1H), 3.21-3.12 (m, 1H), 2.97 (d, *J* = 8.6 Hz, 1H), 2.91 (d, *J* = 8.6 Hz, 1H), 2.84-2.76 (m, 1H), 2.68 (td, *J* = 9.6, 3.9 Hz, 1H), 2.49 (d, *J* = 13.0 Hz, 1H), 2.39-2.33 (m, 2H), 1.79-1.69 (m, 2H), 1.67-1.48 (m, 6H), 1.39-1.25 (m, 2H), 0.89 (t, *J =* 7.3 Hz, 3H); ¹³C NMR (75 MHz, CDCl₃) δ 172.5, 153.8, 148.7, 131.6, 131.3, 123.4, 119.6, 119.4, 56.2, 51.3, 49.8, 46.9, 43.9, 34.8, 34.1, 33.0, 27.6, 24.8, 22.6, 13.7; R_{f} HPLC: 11.8 Min (13 Min from 10 to 95% MeCN in water (0.1 % formic acid), then 7 min 95% MeCN). 98.3 % purity; HRMS (MALDI): m/z found. 369.2395 [M+H]⁺ (cal. C₂₀H₂₉N₆O⁺ 369.2397).

**1-(8-(2-(1H-Tetrazol-5-yl)phenyl)-2,8-diazaspiro[4.5]decan-2-yl)pentan-1-one (Compound 15):** Synthesized according to GP4 from **Compound 94** (104 mg, 0.320 mmol) and tributyltin azide (529 µL, 1.93 mmol) in p-xylene (6.0 mL). Purification by preparative HPLC yielded (30.8 mg, 26%) of **Compound 15.** ¹H NMR (300 MHz, CDCl₃) δ 8.31-8.27 (m, 1H), 7.52 (t, *J* = 7.5 Hz, 1H), 7.39 (t, *J* = 7.5 Hz, 1H), 7.35-7.29 (m, 1H), 3.57-3.52 (m, 2H), 3.47 (s, 2H), 3.00-2.87 (m, 4H), 2.29-2.23 (m, 2H), 1.89-1.79 (m, 6H), 1.66-1.64 (m, 2H), 1.42-1.27 (m, 2H), 0.89 (t, *J* = 7.3 Hz, 3H); R_{f} HPLC: 9.3 Min (13 Min from 10 to 95% MeCN in water (0.1 % formic acid), then 7 min 95% MeCN). 100.0 % purity; HRMS (MALDI): m/z found. 369.2400 [M+H]⁺ (cal. C₂₀H₂₉N₆O⁺ 369.2397).

**1-(6-(2-(1H-Tetrazol-5-yl)phenyl)-2,6-diazaspiro[3.4]octan-2-yl)pentan-1-one (Compound 16):** Synthesized according to GP4 from **Compound 95** (88.0 mg, 0.296 mmol) and tributyltin azide (486 µL, 1.78 mmol) in p-xylene (6.0 mL). Purification by preparative HPLC yielded (25.3 mg, 25%) of **Compound 16.** ³H NMR (300 MHz, CDCl₃) δ 7.47 (d, *J* = 7.3 Hz, 1H), 7.38-7.32 (m, 1H), 6.92-6.85 (m, 2H), 4.00 (s, 2H), 3.75 (d, *J* = 10.1 Hz, 2H), 3.70 (d, *J* = 10.1 Hz, 2H), 3.00-2.92 (m, 2H), 2.07-1.99 (m, 4H), 1.51-1.41 (m, 2H), 1.31-1.18 (m, 2H), 0.82 (t, *J* = 7.3 Hz, 3H); ¹³C NMR (75 MHz, CDCl₃) δ 174.3, 155.1, 147.8, 132.0, 131.7, 119.8, 115.7, 112.6, 60.4, 58.8, 58.6, 49.8, 39.4, 35.9, 31.2, 26.9, 22.4, 13.6; R_{f} HPLC: 10.0 Min (13 Min from 10 to 95% MeCN in water (0.1 % formic acid), then 7 min 95% MeCN). 98.8 % purity; HRMS (MALDI): m/z found. 341.2084 [M+H]⁺ (cal. C₁₈H₂₅N₆O⁺ 341.2084).

**1-(7-(2-(1H-Tetrazol-5-yl)phenyl)-2,7-diazaspiro[3.5]nonan-2-yl)pentan-1-one (Compound 17):** Synthesized according to GP4 from **Compound 96** (62.0 mg, 0.199 mmol) and tributyltin azide (327µL, 1.19 mmol) in *p*-xylene (6.0 mL). Purification by preparative HPLC yielded (17.0 mg, 25%) of **Compound 17.** ¹H NMR (300 MHz, DMSO-*d*₆) δ 7.66 (dd, *J* = 7.6, 1.6 Hz, 1H), 7.54-7.49 (m, 1H), 7.25 (d, *J* = 7.8 Hz, 1H), 7.19 (td, *J* = 7.4, 0.7 Hz, 1H), 3.80 (s, 2H), 3.53 (s, 2H), 2.67 (br s, 4H), 2.02 (t, *J=* 7.6 Hz, 2H), 1.74 (t, *J=* 5.0 Hz, 4H), 1.48-1.38 (m, 2H), 1.34-1.22 (m, 2H), 0.85 (t, *J=* 7.3 Hz, 3H); ¹³C NMR (75 MHz, DMSO-*d*₆) δ 172.4, 153.5, 151.9, 132.0, 130.8, 123.1, 119.7, 118.8, 59.2, 57.0, 49.3, 34.5, 32.3, 30.2, 26.4, 25.5, 21.9, 13.8; R_{f} HPLC: 9.3 Min (13 Min from 10 to 95% MeCN in water (0.1 % formic acid), then 7 min 95% MeCN). 99.0 % purity; HRMS (MALDI): m/z found. 355.2240 [M+H]⁺ (cal. C₁₉H₂₇N₆O⁺ 355.2241).

**1-(2-(2-(1H-Tetrazol-5-yl)phenyl)-2,7-diazaspiro[3.5]nonan-7-yl)pentan-1-one (Compound 18):** Synthesized according to GP4 from **Compound 97** (123 mg, 0.395 mmol) and tributyltin azide (651 µL, 2.37 mmol) in *p*-xylene (6.0 mL). Purification by preparative HPLC yielded (10.8 mg, 7.7%) of **Compound 18.** ¹H NMR (300 MHz, CDCl₃) δ 7.49 (d, *J =* 7.7 Hz, 1H), 7.37 (t, *J=* 7.9 Hz, 1H), 6.89 (t, *J=* 7.4 Hz, 1H), 6.63 (d, *J* = 8.4 Hz, 1H), 3.46-3.31 (m, 8H), 2.31 (t, *J=* 7.9 Hz, 2H), 1.74-1.49 (m, 6H), 1.39-1.27 (m, 2H), 0.88 (t, *J* = 7.3 Hz, 3H); R_{f} HPLC: 10.0 Min (13 Min from 10 to 95% MeCN in water (0.1 % formic acid), then 7 min 95% MeCN). 98.6 % purity; HRMS (MALDI): m/z found. 355.2242 [M+H]⁺ (cal. C₁₉H₂₇N₆O⁺ 355.2241).

**1-(2-(2-(1H-Tetrazol-5-yl)phenyl)-2,6-diazaspiro[3.4]octan-6-yl)pentan-1-one (Compound 19):** Synthesized according to GP4 from **Compound 98** (84.0 mg, 0.282 mmol) and tributyltin azide (464 µL, 1.69 mmol) in *p*-xylene (6.0 mL). Purification by preparative HPLC yielded (33.6 mg, 35%) of **Compound 19.** ¹H NMR (300 MHz, DMSO-*d*₆) δ 7.40 (t, *J* = 8.1 Hz, 1H), 7.29 (d, *J* = 7.4 Hz, 1H), 6.87 (t, *J* = 7.4 Hz, 1H), 6.66 (d, *J* = 8.3 Hz, 1H), 3.52-3.23 (m, 8H), 2.17 (t, *J* = 7.6 Hz, 2H), 1.95 (t, *J* = 6.9 Hz, 2H), 1.49-1.39 (m, 2H), 1.33-1.20 (m, 2H), 0.85 (t, *J =* 7.3 Hz, 3H); R_{f} HPLC: 10.2 Min (13 Min from 10 to 95% MeCN in water (0.1 % formic acid), then 7 min 95% MeCN). 99.1 % purity; HRMS (MALDI): m/z found. 341.2089 [M+H]⁺ (cal. C₁₈H₂₅N₆O⁺ 341.2084).

**1-(6-(2-(1H-Tetrazol-5-yl)phenyl)-2,6-diazaspiro[3.3]heptan-2-yl)pentan-1-one (Compound 20):** Synthesized according to GP4 from **Compound 99** (62.9 mg, 0.222 mmol) and tributyltin azide (365 µL, 1.33 mmol) in *p*-xylene (5.0 mL). Purification by preparative HPLC yielded (5.8 mg, 8.0%) of **Compound 20.** ¹H NMR (300 MHz, DMSO-*d*₆) δ 7.44-7.38 (m, 1H), 7.31 (dd, *J* = 7.7, 1.5 Hz, 1H), 6.88 (td, *J* = 7.5, 0.9 Hz, 1H), 6.67 (d, *J* = 7.8 Hz, 1H), 4.18 (s, 2H), 3.89 (s, 2H), 3.65 (s, 4H), 1.96 (t, *J* = 7.6 Hz, 1H), 1.45-1.35 (m, 2H), 1.30-1.20 (m, 2H), 0.84 (t, *J* = 7.3 Hz, 3H); ¹³C NMR (75 MHz, DMSO-*d*₆) δ 172.0, 149.7, 131.3, 131.2, 117.9, 113.8, 109.1, 62.7, 59.4, 57.3, 32.2, 30.2, 26.3, 21.8, 13.7; R_{f} HPLC: 9.9 Min (13 Min from 10 to 95% MeCN in water (0.1 % formic acid), then 7 min 95% MeCN). 97.8 % purity; HRMS (MALDI): m/z found. 327.1933 [M+H]⁺ (cal. C₁₇H₂₃N₆O⁺ 327.1928).

### Synthesis of isoindolines Compounds 11, 21-36

Most of the isoindoline derivatives were obtained according to FIG. 3

**GP5. Acylation reaction:** Triethylamine (1.3 eq or 2.3 eq in case of amine hydrochlorides) and the corresponding acid chloride (1.3 eq) were added to the corresponding amine (1.0 eq) in dichloromethane at 0°C under Ar. The reaction mixture was then allowed to stir at rt for 3h. The reaction mixture was diluted with dichloromethane and washed with a saturated aqueous ammonium chloride solution and brine. The organic phase was dried over magnesium sulfate, filtered and evaporated. The crude product was purified by flash chromatography.

**GP6. Amide coupling reaction:** The corresponding carboxylic acid (1.0 eq) and HBTU (1.5 eq) were dissolved in DMF under Ar and stirred for 15 min at rt. The solution was then cooled to 0°C and the corresponding amine (1.1 eq) and 4-methylmorpholine (6.0 eq) were added. The resulting solution was stirred at 0°C for 30 min. EDCI (1.5 eq) was then added and the reaction mixture was allowed to stir at rt overnight. The solvents were evaporated at reduced pressure and the residue taken up in a saturated sodium bicarbonate solution and ethyl acetate. The phases were separated and the aqueous phases extracted with ethyl acetate (2x). The combined organic phases were washed with brine, dried over magnesium sulfate, filtered and evaporated. The crude product was purified by flash chromatography.

**GP7. Isoindoline ring formation:** Sodium hydride (4.0 eq) was added to a solution of the corresponding amine (2.0 eq) in THF at 0°C and the resulting solution was stirred at 0°C for 30 min. 4-Bromo-1,2-bis(bromomethyl)benzene (1.0 eq) was then added in THF dropwise and the resulting solution stirred at rt overnight. After that time some water was added to quench the reaction and the solvents were evaporated. The residue was taken up in water/EtOAc. The aqueous phase was extracted with ethyl acetate (3x). The combined organic layers were dried over magnesium sulfate, filtered and evaporated. The residue was purified by flash chromatography.

**GP8. Suzuki coupling: a.** To a solution of the aryl bromide (1eq) in DMF under Ar was added successively 1.3 eq of boronic acid, 3 eq of potassium carbonate and 0.1 eq of Pd(PPh₃)₄. The reaction mixture was heated at 120 °C for 2h. The solvents were evaporated and the residue taken into water and ethyl acetate. The aqueous phase was extracted with ethyl acetate (3x). The combined organic layers were dried over magnesium sulfate, filtered and evaporated. The crude compound was purified by flash chromatography; **b.** To a solution of the aryl bromide (1eq) in Toluene/DME/H₂O under Ar was added successively 1.3 eq of boronic acid, 3.5 eq of cesium carbonate and 0.035 eq of Pd(PPh₃)₄. The reaction mixture was heated at reflux temperature overnight. The solvents were evaporated and the residue taken into water and ethyl acetate. The aqueous phase was extracted with ethyl acetate (3x). The combined organic layers were dried over magnesium sulfate, filtered and evaporated. The crude compound was purified by flash chromatography.

**GP9a. Ester hydrolysis:** LiOH (10 eq) (dissolved in a few uL of water) was added to a solution (0.1 M) of the starting material in THF. A few drops of methanol are added until the resulting solution is monophasic. The resulting mixture was heated at 60 °C overnight. Methanol and THF were evaporated and the residue diluted with water and extracted with ethyl acetate (3x). The combined organic layers were dried over magnesium sulphate, filtered and evaporated. The crude product was purified by HPLC.

**GP9b. Tetrazole ring formation:** To a solution of the substrate (1 eq) in p-xylene was added tributyltin azide (6.0 eq) and the resulting solution was heated at 150 °C overnight. Solvents were evaporated and the residue taken up in dichloromethane. The organic phase was extracted with aqueous NaOH 2M (2x). The aqueous phases were collected and acidified with hydrochloric acid until pH 3-4. Then, the aqueous phase was extracted with ethyl acetate (3x). The combined organic layers were dried over magnesium sulfate, filtered and evaporated. The crude compound was purified by HPLC.

These reaction steps resulted in the following yields.

| Compound | R² | R¹¹ | R¹⁰ | R²⁰/R³ | Yields |
|---|---|---|---|---|---|
| **100**, **116**, **11** | 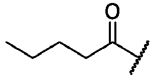 | H | H | CO₂Me/CO₂H | GP5 80% |
| | | | | | GP8 75% |
| | | | | | GP9a/b 49 % |
| **101**, **117**, **21** | 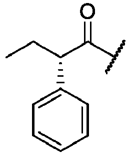 | H | H | CO₂Me/CO₂H | GP6 68 % |
| | | | | | GP8 32 % |
| | | | | | GP9a/b 36 % |
| **102**, **118**, **22** | 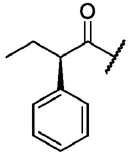 | H | H | CO₂Me/CO₂H | GP6 82 % |
| | | | | | GP8 53 % |
| | | | | | GP9a/b 66 % |
| **103**, **119**, **23** | 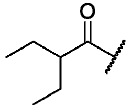 | H | H | CO₂Me/CO₂H | GP6 70 % |
| | | | | | GP8 62 % |
| | | | | | GP9a/b 52 % |
| **104**, **120**, **24** | 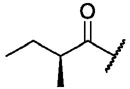 | H | H | CO₂Me/CO₂H | GP6 61 % |
| | | | | | GP8 58 % |
| | | | | | GP9a/b 39 % |
| **105**, **121**, **25** | 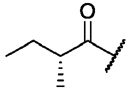 | H | H | CO₂Me/CO₂H | GP6 71 % |
| | | | | | GP8 47 % |
| | | | | | GP9a/b 70 % |
| **106**, **122**, **26** | 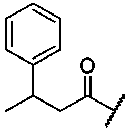 | H | H | CO₂Me/CO₂H | GP6 68 % |
| | | | | | GP8 45 % |
| | | | | | GP9a/b 55 % |
| **107**, **123**, **27** | 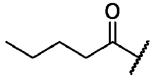 | Ph | H | CO₂Me/CO₂H | GP5 68 % |
| | | | | | GP8 57 % |
| | | | | | GP9a/b 92 % |
| **108**, **124**, **28** | 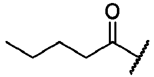 | H | Ph | CO₂Me/CO₂H | GP5 59 % |
| | | | | | GP8 87 % |
| | | | | | GP9a/b 9 % |
| **100**, **125**, **29** | 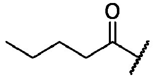 | H | Me | CN/tetrazole | GP5 80 % |
| | | | | | GP8 38 % |
| | | | | | GP9a/b 49 % |
| **109**, **126**, **30** | 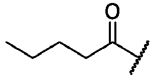 | H | Me | CN/tetrazole | GP5 81 % |
| | | | | | GP8 69 % |
| | | | | | GP9a/b 91 % |
| **110**, **127**, **31** | 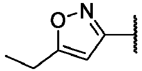 | H | H | CN/tetrazole | GP7 68 % |
| | | | | | GP8 12 % |
| | | | | | GP9a/b 25 % |
| **111**, **128**, **32** | 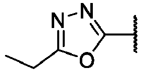 | H | H | CN/tetrazole | GP7 79 % |
| | | | | | GP8 71 % |
| | | | | | GP9a/b 69 % |
| **112**, **129**, **33** | 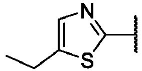 | H | H | CN/tetrazole | GP7 87 % |
| | | | | | GP8 53 % |
| | | | | | GP9a/b 50 % |
| **113**, **130**, **34** | 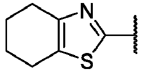 | H | H | CN/tetrazole | GP7 56 % |
| | | | | | GP8 56 % |
| | | | | | GP9a/b 34 % |
| **114, 131, 35** | 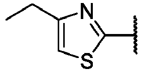 | H | H | CN/tetrazole | GP7 83 % |
| | | | | | GP8 48 % |
| | | | | | GP9a/b 81 % |
| **115**, **132**, **36** | 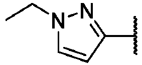 | H | H | CN/tetrazole | GP7 55 % |
| | | | | | GP8 29 % |
| | | | | | GP9a/b 38 % |

In these reactions, the following compounds were synthesized:

**1-(5-Bromoisoindolin-2-yl)pentan-1-one (Compound 100):** Synthesized according to GP1 from 5-bromoisoindoline hydrochloride (400 mg, 1.71 mmol), valeryl chloride (270 µL, 2.22 mmol) and trimethylamine (552 µL, 3.92 mmol) in dichloromethane (15.0 mL). Purification of the crude product by flash chromatography (Hexane/EtOAc 6:4 to 5:5) yielded (386 mg, 80%) of **Compound 100.** ¹H NMR (250 MHz, CDCl₃) δ 7.45-7.40 (m, 2H), 7.13 (d, *J =* 8.2 Hz, 1H), 7.21-7.05 (m, 1H), 4.78-4.73 (m, 4H), 2.36 (t, *J=* 7.5 Hz, 2H), 1.76-1.64 (m, 2H), 1.49-1.34 (m, 2H), 0.95 (t, *J=* 7.3 Hz, 3H).

**(S)-1-(5-Bromoisoindolin-2-yl)-2-phenylbutan-1-one (Compound 101):** Synthesized according to GP2 from (5)-(+)-2-phenylbutyric acid (200 mg, 1.22 mmol), HBTU (709 mg, 1.85 mmol), 5-bromoindoline hydrochloride (315 mg, 1.34 mmol), 4-methylmorpholine (810 µL, 7.37 mmol) and EDCI (355 mg, 1.85 mmol) in DMF (15.0 mL). Purification of the crude product by flash chromatography (Hexane/EtOAc 9:1 to 8:2) yielded (284 mg, 68%) of **Compound 101.** ¹H NMR (250 MHz, CDCl₃) δ 7.41-7.20 (m, 7H), 7.05 (d, *J=* 8.1 Hz, 1H), 4.90-4.48 (m, 4H), 3.51 (t, *J=* 7.4 Hz, 1H), 2.26-2.09 (m, 1H), 1.89-1.71 (m, 1H), 0.91 (t, *J* = 7.4 Hz, 3H).

**(*R*)-1-(5-Bromoisoindolin-2-yl)-2-phenylbutan-1-one (Compound 102):** Synthesized according to GP2 from (*S*)-(+)-2-phenylbutyric acid (200 mg, 1.22 mmol), HBTU (709 mg, 1.85 mmol), 5-bromoindoline hydrochloride (315 mg, 1.34 mmol), 4-methylmorpholine (810 µL, 7.37 mmol) and EDCI (355 mg, 1.85 mmol) in DMF (15.0 mL). Purification of the crude product by flash chromatography (Hexane/EtOAc 9:1 to 8:2) yielded (343 mg, 82%) of **Compound 102.** ¹H NMR (250 MHz, CDCl₃) δ 7.41-7.20 (m, 7H), 7.05 (d, *J* = 8.1 Hz, 1H), 4.90-4.48 (m, 4H), 3.51 (t, *J* = 7.4 Hz, 1H), 2.26-2.09 (m, 1H), 1.89-1.71 (m, 1H), 0.91 (t, *J* = 7.4 Hz, 3H).

**1-(5-Bromoisoindolin-2-yl)-2-ethylbutan-1-one (Compound 103):** Synthesized according to GP2 from 2-ethylbutyric acid (142 mg, 1.22 mmol), HBTU (709 mg, 1.85 mmol), 5-bromoindoline hydrochloride (315 mg, 1.34 mmol), 4-methylmorpholine (810 µL, 7.37 mmol) and EDCI (355 mg, 1.85 mmol) in DMF (15.0 mL). Purification of the crude product by flash chromatography (Hexane/EtOAc 9:1 to 8:2) yielded (253 mg, 70%) of **Compound 103.** ¹H NMR (250 MHz, CDCl₃) δ 7.46-7.40 (m, 2H), 7.13 (d, *J* = 8.1 Hz, 1H), 4.86-4.78 (m, 4H), 2.49-2.37 (m, 1H), 1.79-1.66 (m, 2H), 1.60-1.48 (m, 2H), 0.92 (t, *J* = 7.5 Hz, 6H).

**(.5)-1-(5-Bromoisoindolin-2-yl)-2-methylbutan-1-one (Compound 104):** Synthesized according to GP2 from (*S*)-(+)-2-methylbutyric acid (125 mg, 1.22 mmol), HBTU (709 mg, 1.85 mmol), 5-bromoindoline hydrochloride (315 mg, 1.34 mmol), 4-methylmorpholine (810 µL, 7.37 mmol) and EDCI (355 mg, 1.85 mmol) in DMF (15.0 mL). Purification of the crude product by flash chromatography (Hexane/EtOAc 8:2 to 7:3) yielded (209 mg, 61%) of **Compound 104.** ¹H NMR (250 MHz, CDCl₃) δ 7.45-7.40 (m, 2H), 7.13 (d, *J* = 8.1 Hz, 1H), 4.85-4.75 (m, 4H), 2.61-2.47 (m, 1H), 1.86-1.68 (m, 1H), 1.56-1.40 (m, 1H), 1.17 (d, *J* = 6.8 Hz, 3H), 0.93 (t, *J* = 7.4 Hz, 3H).

**(*R*)-1-(5-Bromoisoindolin-2-yl)-2-methylbutan-1-one (Compound 105):** Synthesized according to GP2 from (R)-2-methylbutyric acid (125 mg, 1.22 mmol), HBTU (709 mg, 1.85 mmol), 5-bromoindoline hydrochloride (315 mg, 1.34 mmol), 4-methylmorpholine (810 µL, 7.37 mmol) and EDCI (355 mg, 1.85 mmol) in DMF (15.0 mL). Purification of the crude product by flash chromatography (Hexane/EtOAc 8:1 to 8:3) yielded (246 mg, 71%) of **Compound 105.** ¹H NMR (250 MHz, CDCl₃) δ 7.45-7.40 (m, 2H), 7.13 (d, *J* = 8.1 Hz, 1H), 4.85-4.75 (m, 4H), 2.61-2.47 (m, 1H), 1.86-1.68 (m, 1H), 1.56-1.40 (m, 1H), 1.17 (d, *J* = 6.8 Hz, 3H), 0.93 (t, *J* = 7.4 Hz, 3H).

**1-(5-Bromoisoindolin-2-yl)-3-phenylbutan-1-one (Compound 106):** Synthesized according to GP2 from 3-phenylbutyric acid (200 mg, 1.22 mmol), HBTU (709 mg, 1.85 mmol), 5-bromoindoline hydrochloride (315 mg, 1.34 mmol), 4-methylmorpholine (810 µL, 7.37 mmol) and EDCI (355 mg, 1.85 mmol) in DMF (15.0 mL). Purification of the crude product by flash chromatography (Hexane/EtOAc 8:2 to 7:3) yielded (286 mg, 68%) of **Compound 106.** ¹H NMR (250 MHz, CDCl₃) δ 7.42-7.26 (m, 6H), 7.24-7.12 (m, 1H), 7.06 (d, *J* = 8.1 Hz, 1H), 4.81-4.62 (m, 4H), 3.53-3.39 (m, 1H), 2.70-2.52 (m, 2H), 1.38 (d, *J* = 7.0 Hz, 3H).

**1-(6-Bromo-1-phenylisoindolin-2-yl)pentan-1-one (Compound 107):** Synthesized according to GP1 from **137** (90.0 mg, 0.328 mmol), valeryl chloride (52.0 µL, 0.427 mmol) and triethylamine (60.1 µL, 0.427 mmol in dichloromethane (4.0 mL). Purification of the crude product by flash chromatography (Hexane/EtOAc 9:1 to 7:3) yielded (80.0 mg, 68%) of **Compound 107.** ¹H NMR (250 MHz, CDCl₃) δ 7.43-7.15 (m, 8H), 5.95 (s, 1H), 5.06-4.88 (m, 2H), 2.41-1.90 (m, 2H), 1.70-1.08 (m, 4H), 0.75 (t, *J* = 7.3 Hz, 3H).

**1-(5-Bromo-1-phenylisoindolin-2-yl)pentan-1-one (Compound 108):** Synthesized according to GP1 from **138** (26.1 mg, 0.0952 mmol), valeryl chloride (15.1 µL, 0.124 mmol) and triethylamine (17.4 µL, 0.124 mmol in dichloromethane (2.0 mL). Purification of the crude product by flash chromatography (Hexane/EtOAc 9:1 to 7:3) yielded (20.0 mg, 59%) of **Compound 108.** ¹H NMR (250 MHz, CDCl₃) δ 7.49 (s, 1H), 7.38-7.18 (m, 6H), 6.88 (d, *J* = 8.1 Hz, 1H), 5.93 (s, 1H), 5.10-4.93 (m, 2H), 2.41-1.91 (m, 2H), 1.70-1.10 (m, 4H), 0.75 (t, *J* = 7.3 Hz, 3H).

**1-(5-Bromo-1-methylisoindolin-2-yl)pentan-1-one (Compound 109):** Synthesized according to GP1 from 5-bromo-1-methylisoindoline hydrochloride (250 mg, 1.01 mmol), valeryl chloride (160 µL, 1.31 mmol) and triethylamine (326 µL, 2.32 mmol in dichloromethane (10.0 mL). Purification of the crude product by flash chromatography (Hexane/EtOAc 9:1 to 7:3) yielded (243 mg, 81%) of **Compound 109.** ¹H NMR (250 MHz, CDCl₃) δ 7.44-7.38 (m, 2H), 7.12-7.05 (m, 1H), 5.27 (q, *J* = 6.5 Hz, 1H), 4.76 (s, 2H), 2.34 (t, *J* = 7.9 Hz, 2H), 1.77-1.60 (m, 2H), 1.49 (d, *J* = 6.4 Hz, 3H), 1.47-1.33 (m, 2H), 0.95 (t, *J* = 7.5 Hz, 3H).

**3-(5-Bromoisoindolin-2-yl)-5-ethylisoxazole (Compound 110):** Synthesized according to GP3 from 4-bromo-1,2-bis(bromomethyl)benzene (386 mg, 1.12 mmol), 5-ethylisoxazol-3-amine (250 mg, 2.23 mmol) and sodium hydride (179 mg, 4.48 mmol) in THF (10.0 mL). Purification of the crude product by flash chromatography (Hexane/EtOAc 9:1 to 7:3) yielded (223 mg, 68%) of 110. ¹H NMR (250 MHz, CDCl₃) δ 7.45-7.39 (m, 2H), 7.17 (d, *J* = 8.0 Hz, 1H), 5.61 (s, 1H), 4.67-4.61 (m, 4H), 2.69 (qd, *J* = 7.6, 0.7 Hz, 2H), 1.29 (t, *J* = 7.6 Hz, 3H).

**2-(5-Bromoisoindolin-2-yl)-5-ethyl-1,3,4-oxadiazole (Compound 111):** Synthesized according to GP3 from 4-bromo-1,2-bis(bromomethyl)benzene (379 mg, 1.10 mmol), 5-ethyl-1,3,4-oxadiazol-2-ylamine (250 mg, 2.21 mmol) and sodium hydride (177 mg, 4.42 mmol) in THF (10.0 mL). Purification of the crude product by flash chromatography (Hexane/EtOAc 5:5 to 0:10) yielded (257 mg, 79%) of **Compound 111.** ¹H NMR (250 MHz, CDCl₃) δ 7.47-7.43 (m, 2H), 7.19 (d, *J* = 8.5 Hz, 1H), 4.83-4.79 (m, 4H), 2.77 (q, *J* = 7.6 Hz, 2H), 1.34 (t, *J* = 7.6 Hz, 3H).

**2-(5-Bromoisoindolin-2-yl)-5-ethylthiazole (Compound 112):** Synthesized according to GP3 from 4-bromo-1,2-bis(bromomethyl)benzene (167 mg, 0.487 mmol), 5-ethylthiazol-2-amine (125 mg, 0.975 mmol) and sodium hydride (78.0 mg, 1.95 mmol) in THF (6.0 mL). Purification of the crude product by flash chromatography (Hexane/EtOAc 8:2 to 6:4) yielded (131 mg, 87%) of **Compound 112.** ¹H NMR (250 MHz, CDCl₃) δ 7.46-7.41 (m, 2H), 7.19 (d, *J* = 8.0 Hz, 1H), 6.91 (t, *J* = 1.2 Hz, 1H), 4.75-4.71 (m, 4H), 2.72 (qd, *J* = 7.5, 1.2 Hz, 2H), 1.27 (t, *J* = 7.5 Hz, 3H).

**2-(5-Bromoisoindolin-2-yl)-4,5,6,7-tetrahydrobenzo[d]thiazole (Compound 113):** Synthesized according to GP3 from 4-bromo-1,2-bis(bromomethyl)benzene (327 mg, 0.955 mmol), 2-amino-4,5,6,7-tetrahydrobenzothiazole (295 mg, 1.91 mmol) and sodium hydride (153 mg, 3.82 mmol) in THF (10.0 mL). Purification of the crude product by flash chromatography (Hexane/EtOAc 9:1 to 7:3) yielded (180 mg, 56%) of **Compound 113.** ¹H NMR (250 MHz, CDCl₃) δ 7.45-7.41 (m, 2H), 7.18 (d, *J* = 7.8 Hz, 1H), 4.75-4.69 (m, 4H), 2.65-2.62 (m, 4H), 1.86-1.81 (m, 4H).

**2-(5-Bromoisoindolin-2-yl)-4-ethylthiazole (Compound 114):** Synthesized according to GP3 from 4-bromo-1,2-bis(bromomethyl)benzene (261 mg, 0.760 mmol), 4-ethyl-1,3-thaizol-2-amine hydrochloride (250 mg, 1.52 mmol) and sodium hydride (153 mg, 3.82 mmol) in THF (10.0 mL). Purification of the crude product by flash chromatography (Hexane/EtOAc 8:2 to 7:3) yielded (194 mg, 83%) of **Compound 114.** ¹H NMR (250 MHz, CDCl₃) δ 7.45-7.41 (m, 2H), 7.18 (d, *J* = 7.8 Hz, 1H), 6.14 (s, 1H), 4.78-4.73 (m, 4H), 2.67 (qd, *J* = 7.5, 1.0 Hz, 2H), 1.27 (t, *J* = 7.5 Hz, 3H).

**5-Bromo-2-(1-ethyl-1H-pyrazol-3-yl)isoindoline (Compound 115):** Synthesized according to GP3 from 4-bromo-1,2-bis(bromomethyl)benzene (386 mg, 1.13 mmol), 1-ethyl-1H-pyrazol-3-amine (250 mg, 2.25 mmol) and sodium hydride (180 mg, 4.50 mmol) in THF (10.0 mL). Purification of the crude product by flash chromatography (Hexane/EtOAc 8:2 to 7:3) yielded (181 mg, 55%) of **Compound 115.** ¹H NMR (250 MHz, CDCl₃) δ 7.43 (s, 1H), 7.39 (dd, *J* = 8.1, 1.7 Hz, , 1H) 7.24 (d, *J* = 2.4 Hz, 1H), 7.17 (d, *J* = 8.1 Hz, 1H,), 5.57 (d, *J* = 2.3 Hz, 1H), 4.64-4.60 (m, 4H), 4.08 (q, *J* = 7.3 Hz, 2H), 1.46 (t, *J* = 7.3 Hz, 3H).

**Methyl 2-(2-pentanoylisoindolin-5-yl)benzoate (Compound 116):** Synthesized according to GP4a from **Compound 100** (80 mg, 0.284 mmol), 2-methoxycarbonylphenylboronic acid (66.3 mg, 0.369 mmol), potassium carbonate (118 mg, 0.851 mmol) and Pd(PPh₃)₄ (32.8 mg, 0.0284 mmol) in DMF (4.0 mL). Purification of the crude product by flash chromatography (Hexane/EtOAc 9:1 to 8:2) yielded (71.7 mg, 75%) of **Compound 116.** ¹H NMR (250 MHz, CDCl₃) δ 7.85 (d, *J* = 7.7 Hz, 1H), 7.72-7.62 (m, 2H), 7.55-7.39 (m, 3H), 7.23-7.17 (m, 1H), 5.07-4.71 (m, 4H), 3.68 (s, 3H), 2.38 (t, *J* = 7.6 Hz, 2H), 1.81-1.62 (m, 2H), 1.51-1.32 (m, 2H), 0.96 (t, *J* = 6.2 Hz, 3H).

**Methyl (*S*)-2-(2-(2-phenylbutanoyl)isoindolin-5-yl)benzoate (Compound 117):** Synthesized according to GP4a from **Compound 101** (284 mg, 0.826 mmol), 2-methoxycarbonylphenylboronic acid (193 mg, 1.07 mmol), potassium carbonate (346 mg, 2.48 mmol) and Pd(PPh₃)₄ (98.4 mg, 0.0826 mmol) in DMF (8.0 mL). Purification of the crude product by flash chromatography (Hexane/EtOAc 8:2 to 7:3) yielded (104 mg, 32%) of **Compound 117.** ¹H NMR (250 MHz, CDCl₃) δ 7.82 (d, *J* = 7.5 Hz, 1H), 7.50 (t, *J* = 7.5 Hz, 1H), 7.42-7.11 (m, 10H), 4.95-4.58 (m, 4H), 3.64 (s, 3H), 3.55 (t, *J* = 7.5 Hz, 1H), 2.27-2.10 (m, 1H), 1.85-1.74 (m, 1H), 0.91 (t, *J* = 7.4 Hz, 3H).

**Methyl (*R*)-2-(2-(2-phenylbutanoyl)isoindolin-5-yl)benzoate (Compound 118):** Synthesized according to GP4a from **Compound 102** (343 mg, 0.996 mmol), 2-methoxycarbonylphenylboronic acid (233 mg, 1.30 mmol), potassium carbonate (417 mg, 2.99 mmol) and Pd(PPh₃)₄ (119 mg, 0.0996 mmol) in DMF (10.0 mL). Purification of the crude product by flash chromatography (Hexane/EtOAc 8:2 to 7:3) yielded (209 mg, 53%) of **Compound 118.** ¹H NMR (250 MHz, CDCl₃) δ 7.82 (d, *J* = 7.5 Hz, 1H), 7.50 (t, *J* = 7.5 Hz, 1H), 7.42-7.11 (m, 10H), 4.95-4.58 (m, 4H), 3.64 (s, 3H), 3.55 (t, *J* = 7.5 Hz, 1H), 2.27-2.10 (m, 1H), 1.85-1.74 (m, 1H), 0.91 (t, *J* = 7.4 Hz, 3H).

**Methyl 2-(2-(2-ethylbutanoyl)isoindolin-5-yl)benzoate (Compound 119):** Synthesized according to GP4a from **Compound 103** (253 mg, 0.854 mmol), 2-methoxycarbonylphenylboronic acid (200 mg, 1.11 mmol), potassium carbonate (358 mg, 2.56 mmol) and Pd(PPh₃)₄ (102 mg, 0.0854 mmol) in DMF (8.0 mL). Purification of the crude product by flash chromatography (Hexane/EtOAc 8:2 to 6:4) yielded (186 mg, 62%) of **Compound 119.** ¹H NMR (250 MHz, CDCl₃) δ 7.89-7.84 (m, 1H), 7.57-7.51 (m, 1H), 7.46-7.29 (m, 3H), 7.24-7.21 (m, 2H), 4.89 (s, 4H), 3.71 (s, 3H), 2.54-2.42 (m, 1H), 1.84-1.66 (m, 2H), 1.64-1.47 (m, 2H), 0.94 (t, *J* = 7.4 Hz, 3H).

**Methyl (*S*)-2-(2-(2-methylbutanoyl)isoindolin-5-yl)benzoate (Compound 120):** Synthesized according to GP4a from **Compound 104** (209 mg, 0.744 mmol), 2-methoxycarbonylphenylboronic acid (174 mg, 0.967 mmol), potassium carbonate (308 mg, 2.23 mmol) and Pd(PPh₃)₄ (86.0 mg, 0.0744 mmol) in DMF (8.0 mL). Purification of the crude product by flash chromatography (Hexane/EtOAc 8:2 to 6:4) yielded (145 mg, 58%) of **Compound 120.** ¹H NMR (250 MHz, CDCl₃) δ 7.88-7.84 (m, 1H), 7.58-7.51 (m, 1H), 7.46-7.42 (m, 1H), 7.40-7.30 (m, 2H), 7.26-7.21 (m, 2H), 4.86 (s, 4H), 3.70 (s, 3H), 2.66-2.52 (m, 1H), 1.88-1.71 (m, 1H), 1.59-1.41 (m, 1H), 1.19 (d, *J* = 6.8 Hz, 3H), 0.95 (t, *J* = 7.4 Hz, 3H).

**Methyl (*R*)-2-(2-(2-methylbutanoyl)isoindolin-5-yl)benzoate (Compound 121):** Synthesized according to GP4a from **Compound 105** (245 mg, 0.868 mmol), 2-methoxycarbonylphenylboronic acid (203 mg, 1.13 mmol), potassium carbonate (364 mg, 2.60 mmol) and Pd(PPh₃)₄ (103 mg, 0.0868 mmol) in DMF (10.0 mL). Purification of the crude product by flash chromatography (Hexane/EtOAc 8:2 to 6:4) yielded (138 mg, 47%) of **Compound 121.** ¹H NMR (250 MHz, CDCl₃) δ 7.88-7.84 (m, 1H), 7.58-7.51 (m, 1H), 7.46-7.42 (m, 1H), 7.40-7.30 (m, 2H), 7.26-7.21 (m, 2H), 4.86 (s, 4H), 3.70 (s, 3H), 2.66-2.52 (m, 1H), 1.88-1.71 (m, 1H), 1.59-1.41 (m, 1H), 1.19 (d, *J* = 6.8 Hz, 3H), 0.95 (t, *J* = 7.4 Hz, 3H).

**Methyl 2-(2-(3-phenylbutanoyl)isoindolin-5-yl)benzoate (Compound 122):** Synthesized according to GP4a from **Compound 106** (314 mg, 0.912 mmol), 2-methoxycarbonylphenylboronic acid (213 mg, 1.19 mmol), potassium carbonate (382 mg, 2.74 mmol) and Pd(PPh₃)₄ (105 mg, 0.0912 mmol) in DMF (10.0 mL). Purification of the crude product by flash chromatography (Hexane/EtOAc 7:3 to 5:5) yielded (164 mg, 45%) of **Compound 122.** ¹H NMR (250 MHz, CDCl₃) δ 7.85 (dd, *J* = 7.7, 1.4 Hz, 1H), 7.53 (td, *J* = 7.5, 1.4 Hz, 1H), 7.45-7.38 (m, 1H), 7.35-7.15 (m, 9H), 4.88-4.65 (m, 4H), 3.68 (s, 3H), 3.56-3.41 (m, 1H), 2.74-2.55 (m, 2H), 1.85-1.74 (m, 1H), 1.39 (t, *J* = 7.0 Hz, 3H).

**Methyl 2-(2-pentanoyl-3-phenylisoindolin-5-yl)benzoate (Compound 123):** Synthesized according to GP4a from **Compound 107** (80 mg, 0.223 mmol), 2-methoxycarbonylphenylboronic acid (52.2 mg, 0.29 mmol), potassium carbonate (93.5 mg, 0.67 mmol) and Pd(PPh₃)₄ (25.8 mg, 0.0223 mmol) in DMF (5.0 mL). Purification of the crude product by flash chromatography (Hexane/EtOAc 7:3 to 6:4) yielded (52.5 mg, 57%) of **Compound 123.** ¹H NMR (250 MHz, CDCl₃) δ 7.77 (dd, *J* = 7.6, 1.5 Hz, 1H), 7.48 (td, *J* = 7.5, 1.5 Hz, 1H), 7.40-7.23 (m, 9H), 6.94 (s, 1H), 6.01 (s, 1H), 5.18-5.00 (m, 2H), 3.32 (s, 3H), 2.45-1.93 (m, 2H), 1.70-1.13 (m, 4H), 0.76 (t, *J* = 7.3 Hz, 3H).

**Methyl 2-(2-pentanoyl-1-phenylisoindolin-5-yl)benzoate (Compound 124):** Synthesized according to GP4a from **Compound 108** (20.0 mg, 0.0558 mmol), 2-methoxycarbonylphenylboronic acid (13.0 mg, 0.0629 mmol), potassium carbonate (23.3 mg, 0.167 mmol) and Pd(PPh₃)₄ (6.50 mg, 0.00558 mmol) in DMF (2.0 mL). Purification of the crude product by flash chromatography (Hexane/EtOAc 7:3 to 6:4) yielded (20.0 mg, 87%) of **Compound 124.** ¹H NMR (250 MHz, CDCl₃) δ 7.86-7.82 (m, 1H), 7.57-7.42 (m, 2H), 7.38-7.02 (m, 8H), 6.89 (d, *J* = 8.1 Hz, 1H), 6.03 (s, 1H), 5.10-4.93 (m, 2H), 3.63 (s, 3H), 2.41-1.91 (m, 2H), 1.70-1.10 (m, 4H), 0.75 (t, *J* = 7.3 Hz, 3H).

**2-(2-Pentanoylisoindolin-5-yl)benzonitrile (Compound 125):** Synthesized according to GP4b from **Compound 100** (385 mg, 1.36 mmol), 2-cyanophenylboronic acid (261 mg, 1.77 mmol), cesium carbonate (1.56 g, 4.78 mmol) and Pd(PPh₃)₄ (55.2 mg, 0.0478 mmol) in Toluene/DME/H₂O (7:9:20, 17 mL). Purification of the crude product by flash chromatography (Hexane/EtOAc 6:4 to 3:7) yielded (157 mg, 38%) of **Compound 125.** ¹H NMR (250 MHz, CDCl₃) δ 7.77 (d, *J* = 7.6 Hz, 1H), 7.66 (t, *J* = 7.6 Hz, 1H), 7.52-7.36 (m, 5H), 4.88 (s, 4H), 2.40 (t, *J* = 7.4 Hz, 2H), 1.78-1.66 (m, 2H), 1.50-1.35 (m, 2H), 0.96 (t, *J* = 7.3 Hz, 3H).

**2-(1-Methyl-2-pentanoylisoindolin-5-yl)benzonitrile (Compound 126):** Synthesized according to GP4a from **Compound 109** (243 mg, 0.820 mmol), 2-cyanophenylboronic acid (157 mg, 1.07 mmol), potassium carbonate (401 mg, 2.87 mmol) and Pd(PPh₃)₄ (94.8 mg, 0.082 mmol) in DMF (8.0 mL). Purification of the crude product by flash chromatography (Hexane/EtOAc 7:3 to 6:4) yielded (179 mg, 69%) of **Compound 126.** ¹H NMR (250 MHz, CDCl₃) δ 7.71-7.26 (m, 7H), 5.36 (q, *J* = 6.5 Hz, 1H), 4.78 (s, 2H), 2.33 (t, *J* = 7.9 Hz, 2H), 1.74-1.62 (m, 2H), 1.52 (d, *J* = 6.4 Hz, 3H), 1.47-1.33 (m, 2H), 0.94 (t, *J* = 7.5 Hz, 3H).

**2-(2-(5-Ethylisoxazol-3-yl)isoindolin-5-yl)benzonitrile (Compound 127):** Synthesized according to GP4a from **Compound 110** (220 mg, 0.745 mmol), 2-cyanophenylboronic acid (142 mg, 0.969 mmol), potassium carbonate (364 mg, 2.61 mmol) and Pd(PPh₃)₄ (86.7 mg, 0.0745 mmol) in DMF (8.0 mL). Purification of the crude product by flash chromatography (Hexane/EtOAc 8:2 to 6:4) yielded (28.2 mg, 12%) of **Compound 127.** ¹H NMR (250 MHz, CDCl₃) δ 7.79-7.75 (m, 1H), 7.65 (td, *J* = 7.6, 1.4 Hz, 1H), 7.54-7.41 (m, 5H), 5.65 (s, 1H), 4.75 (s, 4H), 2.70 (qd, *J* = 7.6, 0.7 Hz, 2H), 1.30 (t, *J* = 7.6 Hz, 3H).

**2-(2-(5-Ethyl-1,3,4-oxadiazol-2-yl)isoindolin-5-yl)benzonitrile (Compound 128):** Synthesized according to GP4a from **Compound 111** (250 mg, 0.850 mmol), 2-cyanophenylboronic acid (162 mg, 1.10 mmol), potassium carbonate (416 mg, 2.98 mmol) and Pd(PPh₃)₄ (98.2 mg, 0.0850 mmol) in DMF (10.0 mL). Purification of the crude product by flash chromatography (Hexane/EtOAc 5:5 to 0:10) yielded (191 mg, 71%) of **Compound 128.** ¹H NMR (250 MHz, CDCl₃) δ 7.78 (dd, *J* = 7.7, 1.3 Hz, 1H), 7.67 (td, *J* = 7.6, 1.4 Hz, 1H), 7.53-7.43 (m, 5H), 4.94 (s, 4H), 2.79 (q, *J* = 7.6 Hz, 2H), 1.36 (t, *J* = 7.6 Hz, 3H).

**2-(2-(5-Ethylthiazol-2-yl)isoindolin-5-yl)benzonitrile (Compound 129):** Synthesized according to GP4a from **Compound 112** (131 mg, 0.424 mmol), 2-cyanophenylboronic acid (80.8 mg, 0.550 mmol), potassium carbonate (207 mg, 1.48 mmol) and Pd(PPh₃)₄ (49.0 mg, 0.0424 mmol) in DMF (6.0 mL). Purification of the crude product by flash chromatography (Hexane/EtOAc 8:2 to 5:5) yielded (74.2 mg, 53%) of **Compound 129.** ¹H NMR (250 MHz, CDCl₃) δ 7.79-7.76 (m, 1H), 7.66 (td, *J* = 7.8, 1.4 Hz, 1H), 7.54-7.42 (m, 5H), 6.93 (br s, 1H), 4.85 (s, 4H), 2.73 (qd, *J* = 7.4, 0.8 Hz, 2H), 1.28 (t, *J* = 7.4 Hz, 3H).

**2-(2-(4,5,6,7-Tetrahydrobenzo[d]thiazol-2-yl)isoindolin-5-yl)benzonitrile (Compound 130):** Synthesized according to GP4a from **Compound 113** (179 mg, 0.534 mmol), 2-cyanophenylboronic acid (102 mg, 0.694 mmol), potassium carbonate (261 mg, 1.87 mmol) and Pd(PPh₃)₄ (62.4 mg, 0.0534 mmol) in DMF (6.0 mL). Purification of the crude product by flash chromatography (Hexane/EtOAc 8:2 to 5:5) yielded (106 mg, 56%) of **Compound 130.** ¹H NMR (250 MHz, CDCl₃) δ 7.79-7.75 (m, 1H), 7.66 (td, *J* = 7.6, 1.4 Hz, 1H), 7.54-7.41 (m, 5H), 4.84 (s, 4H), 2.65 (br s, 4H), 1.86-1.81 (m, 4H).

**2-(2-(4-Ethylthiazol-2-yl)isoindolin-5-yl)benzonitrile (Compound 131):** Synthesized according to GP4a from **Compound 114** (194 mg, 0.627 mmol), 2-cyanophenylboronic acid (120 mg, 0.815 mmol), potassium carbonate (306 mg, 2.19 mmol) and Pd(PPh₃)₄ (72.8 mg, 0.0627 mmol) in DMF (8.0 mL). Purification of the crude product by flash chromatography (Hexane/EtOAc 8:2 to 5:5) yielded (99.3 mg, 48%) of **Compound 131.** ¹H NMR (250 MHz, CDCl₃) δ 7.80-7.76 (m, 1H), 7.66 (td, *J* = 7.6, 1.4 Hz, 1H), 7.54-7.43 (m, 5H), 6.15 (t, *J* = 1.1 Hz, 1H), 4.87 (s, 4H), 2.68 (qd, *J* = 7.5, 1.0 Hz, 2H), 1.28 (t, *J* = 7.5 Hz, 3H).

**2-(2-(1-Ethyl-1H-pyrazol-3-yl)isoindolin-5-yl)benzonitrile (Compound 132):** Synthesized according to GP4a from **Compound 115** (181 mg, 0.619 mmol), 2-cyanophenylboronic acid (118 mg, 0.805 mmol), potassium carbonate (303 mg, 2.17 mmol) and Pd(PPh₃)₄ (71.5 mg, 0.0619 mmol) in DMF (8.0 mL). Purification of the crude product by flash chromatography (Hexane/EtOAc 8:2 to 5:5) yielded (56.0 mg, 29%) of **Compound 132.** ¹H NMR (250 MHz, CDCl₃) δ 7.76 (dd, *J* = 7.8, 1.7 Hz, 1H), 7.64 (td, *J* = 7.6, 1.4 Hz, 1H), 7.54-7.40 (m, 5H), 7.26 (d, *J* = 2.4 Hz, 1H), 5.60 (d, *J* = 2.4 Hz, 1H), 4.75 (s, 4H), 4.10 (q, *J* = 7.3 Hz, 2H), 1.48 (t, *J* = 7.3 Hz, 3H).

**2-(2-Pentanoylisoindolin-5-yl)benzoic acid (Compound 11):** Synthesized according to GP5 from **116** (71.7 mg, 0.212 mmol) and lithium hydroxide (51.9 mg, 2.12 mmol). Purification of the crude product by preparative HPLC yielded (33.7 mg, 49%) of **Compound 11.** ¹H NMR (300 MHz, CDCl₃) δ 8.47 (br s, 1H), 7.96-7.81 (m, 1H), 7.71-7.59 (m, 1H), 7.58-7.35 (m, 3H), 7.33-7.27 (m, 1H), 7.25-7.17 (m, 1H), 4.97-4.51 (m, 4H), 2.48-2.20 (m, 2H), 1.83-1.55 (m, 2H), 1.46-1.31 (m, 2H), 0.99-0.87 (m, 3H); R_{f} HPLC: 11.7 Min (13 Min from 10 to 95% MeCN in water (0.1 % formic acid), then 7 min 95% MeCN). 97.4 % purity; HRMS (MALDI): m/z found. 324.1594 [M+H]⁺ (cal. C₂₀H₂₂NO₃⁺ 324.1594).

**(*S*)-2-(2-(2-Phenylbutanoyl)isoindolin-5-yl)benzoic acid (Compound 21):** Synthesized according to GP5 from **Compound 117** (100 mg, 0.250 mmol) and lithium hydroxide (61.1 mg, 2.50 mmol). Purification of the crude product by preparative HPLC yielded (34.6 mg, 36%) of **Compound 21.** ¹H NMR (300 MHz, CDCl₃) δ 9.51 (br s, 1H), 7.97-7.91 (m, 1H), 7.56-7.50 (m, 1H), 7.45-7.39 (m, 1H), 7.38-7.12 (m, 9H), 4.91-4.54 (m, 4H), 3.57-3.49 (m, 1H), 2.24-2.09 (m, 1H), 1.86-1.71 (m, 1H), 0.89 (t, *J* = 7.4 Hz, 3H); R_{f} HPLC: 13.1 Min (13 Min from 10 to 95% MeCN in water (0.1 % formic acid), then 7 min 95% MeCN). 99.3 % purity; HRMS (MALDI): m/z found. 386.1746 [M+H]⁺ (cal. C₂₅H₂₄NO₃⁺ 386.1751).

**(*R*)-2-(2-(2-Phenylbutanoyl)isoindolin-5-yl)benzoic acid (Compound 22):** Synthesized according to GP5 from **Compound 118** (200 mg, 0.501 mmol) and lithium hydroxide (122 mg, 5.01 mmol). Purification of the crude product by preparative HPLC yielded (127 mg, 66%) of **Compound 22.** ¹H NMR (300 MHz, CDCl₃) δ 9.18 (br s, 1H), 7.97-7.91 (m, 1H), 7.56-7.50 (m, 1H), 7.45-7.39 (m, 1H), 7.38-7.12 (m, 9H), 4.91-4.54 (m, 4H), 3.57-3.49 (m, 1H), 2.24-2.09 (m, 1H), 1.86-1.71 (m, 1H), 0.89 (t, *J* = 7.4 Hz, 3H); R_{f} HPLC: 13.0 Min (13 Min from 10 to 95% MeCN in water (0.1 % formic acid), then 7 min 95% MeCN). 99.8 % purity; HRMS (MALDI): m/z found. 386.1749 [M+H]⁺ (cal. C₂₅H₂₄NO₃⁺ 386.1751).

**2-(2-(2-Ethylbutanoyl)isoindolin-5-yl)benzoic acid (Compound 23):** Synthesized according to GP5 from **Compound** 119 (185 mg, 0.526 mmol) and lithium hydroxide (129 mg, 5.26 mmol). Purification of the crude product by preparative HPLC yielded (93.0 mg, 52%) of **Compound 23.** ¹H NMR (300 MHz, CDCl₃) δ 7.91 (dd, *J* = 7.7, 1.4 Hz, 1H), 7.56-7.51 (m, 1H), 7.42 (t, *J* = 7.5 Hz, 1H), 7.34-7.22 (m, 4H), 4.90 (s, 4H), 2.51-2.40 (m, 1H), 1.79-1.64 (m, 2H), 1.60-1.47 (m, 2H), 0.90 (t, *J* = 7.4 Hz, 3H); R_{f} HPLC: 12.0 Min (13 Min from 10 to 95% MeCN in water (0.1 % formic acid), then 7 min 95% MeCN). 100.0 % purity; HRMS (MALDI): m/z found. 338.1754 [M+H]⁺ (cal. C₂₁H₂₄NO₃⁺ 338.1751).

**(*S*)-2-(2-(2-Methylbutanoyl)isoindolin-5-yl)benzoic acid (Compound 24):** Synthesized according to GP5 from **Compound 120** (140 mg, 0.415 mmol) and lithium hydroxide (101 mg, 4.15 mmol). Purification of the crude product by preparative HPLC yielded (52.9 mg, 39%) of **Compound 24.** ¹H NMR (300 MHz, CDCl₃) δ 8.20 (br s, 1H), 7.92 (dd, *J* = 7.7, 1.4 Hz, 1H), 7.56-7.51 (m, 1H), 7.42 (t, *J* = 7.5 Hz, 1H), 7.32 (d, *J* = 7.6 Hz, 1H), 7.30-7.22 (m, 3H), 4.89 (s, 4H), 2.63-2.51 (m, 1H), 1.83-1.69 (m, 1H), 1.54-1.40 (m, 1H), 1.15 (d, *J* = 6.7 Hz, 3H), 0.92 (t, *J* = 7.4 Hz, 3H); R_{f} HPLC: 11.4 Min (13 Min from 10 to 95% MeCN in water (0.1 % formic acid), then 7 min 95% MeCN). 100.0 % purity; HRMS (MALDI): m/z found. 324.1597 [M+H]⁺ (cal. C₂₀H₂₂NO₃⁺ 324.1594).

**(*R*)-2-(2-(2-Methylbutanoyl)isoindolin-5-yl)benzoic acid (Compound 25):** Synthesized according to GP5 from **Compound 121** (140 mg, 0.415 mmol) and lithium hydroxide (101 mg, 4.15 mmol). Purification of the crude product by preparative HPLC yielded (93.5 mg, 70%) of **Compound 25.** ¹H NMR (300 MHz, CDCl₃) δ 9.90 (br s, 1H), 7.92 (dd, *J* = 7.7, 1.4 Hz, 1H), 7.56-7.51 (m, 1H), 7.42 (t, *J* = 7.5 Hz, 1H), 7.32 (d, *J* = 7.6 Hz, 1H), 7.30-7.22 (m, 3H), 4.89 (s, 4H), 2.63-2.51 (m, 1H), 1.83-1.69 (m, 1H), 1.54-1.40 (m, 1H), 1.15 (d, *J* = 6.7 Hz, 3H), 0.92 (t, *J* = 7.4 Hz, 3H); R_{f} HPLC: 11.4 Min (13 Min from 10 to 95% MeCN in water (0.1 % formic acid), then 7 min 95% MeCN). 100.0 % purity; HRMS (MALDI): m/z found. 324.1595 [M+H]⁺ (cal. C₂₀H₂₂NO₃⁺ 324.1594).

**2-(2-(3-Phenylbutanoyl)isoindolin-5-yl)benzoic acid (Compound 26):** Synthesized according to GP5 from **Compound 122** (164 mg, 0.411 mmol) and lithium hydroxide (100 mg, 4.11 mmol). Purification of the crude product by preparative HPLC yielded (86.9 mg, 55%) of **Compound 26.** ¹H NMR (300 MHz, CDCl₃) δ 10.89 (br s, 1H), 7.94-7.89 (m, 1H), 7.51 (t, *J* = 7.5 Hz, 1H), 7.42-7.36 (m, 1H), 7.30-7.10 (m, 9H), 4.74-4.37 (m, 4H), 3.47-3.35 (m, 1H), 2.65-2.47 (m, 2H), 1.33 (d, *J* = 7.0 Hz, 3H); R_{f} HPLC: 12.6 Min (13 Min from 10 to 95% MeCN in water (0.1 % formic acid), then 7 min 95% MeCN). 100.0 % purity; HRMS (MALDI): m/z found. 386.1745 [M+H]⁺ (cal. C₂₅H₂₄NO₃⁺ 386.1751).

**2-(2-Pentanoyl-3-phenylisoindolin-5-yl)benzoic acid (Compound 27):** Synthesized according to GP5 from **Compound 123** (52.5 mg, 0.127 mmol) and lithium hydroxide (30.9 mg, 1.27 mmol). Purification of the crude product by preparative HPLC yielded (46.5 mg, 92%) of **Compound 27.** ¹H NMR (300 MHz, CDCl₃) δ 7.93-7.88 (m, 1H), 7.51-7.46 (m, 1H), 7.42-7.36 (m, 1H), 7.33-7.17 (m, 8H), 7.01 (s, 1H), 6.01 (s, 1H), 5.09-4.95 (m, 2H), 2.42-1.93 (m, 2H), 1.69-1.08 (m, 4H), 0.74 (t, *J* = 7.3 Hz, 3H); R_{f} HPLC: 13.1 Min (13 Min from 10 to 95% MeCN in water (0.1 % formic acid), then 7 min 95% MeCN). 100.0 % purity; HRMS (MALDI): m/z found. 400.1904 [M+H]⁺ (cal. C₂₆H₂₆NO₃⁺ 400.1907).

**2-(2-Pentanoyl-1-phenylisoindolin-5-yl)benzoic acid (Compound 28):** Synthesized according to GP5 from **Compound 124** (20.0 mg, 0.0484 mmol) and lithium hydroxide (11.8 mg, 0.484 mmol). Purification of the crude product by preparative HPLC yielded (1.8 mg, 9%) of **Compound 28.** ¹H NMR (300 MHz, CDCl₃) δ 7.96-7.93 (m, 1H), 7.57-7.52 (m, 1H), 7.47-7.40 (m, 1H), 7.38-7.17 (m, 8H), 7.10-7.03 (s, 1H), 6.03 (s, 1H), 5.14-4.97 (m, 2H), 2.23-2.18 (m, 2H), 1.60-1.10 (m, 4H), 0.76 (t, *J* = 7.3 Hz, 3H); R_{f} HPLC: 13.2 Min (13 Min from 10 to 95% MeCN in water (0.1 % formic acid), then 7 min 95% MeCN). 95.0 % purity; HRMS (MALDI): m/z found. 400.1905 [M+H]⁺ (cal. C₂₆H₂₆NO₃⁺ 400.1907).

**1-(5-(2-(1H-Tetrazol-5-yl)phenyl)isoindolin-2-yl)pentan-1-one (Compound 29):** Synthesized according to GP6 from **Compound 125** (156 mg, 0.0484 mmol) and tributyltin azide (845 µL, 3.08 mmol) in p-xylene (6.0 mL). Purification of the crude product by preparative HPLC yielded (86.9 mg, 49%) of **Compound 29.** ¹H NMR (300 MHz, DMSO-*d*₆) δ 7.71-7.66 (m, 2H), 7.60-7.52 (m, 2H), 7.28-7.11 (m, 2H), 6.97-6.89 (m, 1H), 4.88 (d, *J* = 15.1 Hz, 2H), 4.60 (d, *J* = 10.4 Hz, 2H), 2.34 (t, *J* = 7.5 Hz, 2H), 1.58-1.48 (m, 2H), 1.39-1.27 (m, 2H), 0.89 (t, *J* = 7.3 Hz, 3H); R_{f} HPLC: 11.1 Min (13 Min from 10 to 95% MeCN in water (0.1 % formic acid), then 7 min 95% MeCN). 98.5 % purity; HRMS (MALDI): m/z found. 370.1629 [M+H]⁺ (cal. C₂₀H₂₂N₅O⁺ 370.1638).

**1-(5-(2-(1H-Tetrazol-5-yl)phenyl)-1-methylisoindolin-2-yl)pentan-1-one (Compound 30):** Synthesized according to GP6 from **Compound 126** (179 mg, 0.562 mmol) and tributyltin azide (923 µL, 3.37 mmol) in p-xylene (8.0 mL). Purification of the crude product by preparative HPLC yielded (185 mg, 91%) of **Compound 31.** ¹H NMR (300 MHz, CDCl₃) δ 7.85 (dd, *J* = 7.6, 1.2 Hz, 1H), 7.64-7.57 (m, 1H), 7.54-7.48 (m, 1H), 7.45 (d, *J* = 7.5 Hz, 1H), 7.19 (d, *J* = 8.0 Hz, 1H), 7.08 (d, *J* = 7.9 Hz, 1H), 6.85 (s, 1H), 4.71 (q, *J* = 6.3 Hz, 1H), 4.67 (d, *J* = 15.0 Hz, 1H), 4.60 (d, *J* = 14.3 Hz, 1H), 2.37-2.11 (m, 2H), 1.53-1.38 (m, 2H), 1.41 (d, *J* = 6.4 Hz, 3H), 1.36-1.21 (m, 2H), 0.84 (t, *J* = 7.3 Hz, 3H); R_{f} HPLC: 11.7 Min (13 Min from 10 to 95% MeCN in water (0.1 % formic acid), then 7 min 95% MeCN). 97.3 % purity; HRMS (MALDI): m/z found. 362.1975 [M+H]⁺ (cal. C₂₁H₂₄N₅O⁺ 362.1975).

**3-(5-(2-(1H-Tetrazol-5-yl)phenyl)isoindolin-2-yl)-5-ethylisoxazole (Compound 31):** Synthesized according to GP6 from **Compound 127** (28.2 mg, 0.0894 mmol) and tributyltin azide (147 µL, 0.536 mmol) in p-xylene (2.0 mL). Purification of the crude product by preparative HPLC yielded (8.1 mg, 25%) of **Compound 30.** ¹H NMR (400 MHz, CDCl₃) δ 7.96 (dd, *J* = 7.4, 1.2 Hz, 1H), 7.61 (td, *J* = 7.4, 1.4 Hz, 1H), 7.56 (td, *J* = 7.5, 1.3 Hz, 1H), 7.41 (dd, *J* = 7.6, 1.2 Hz, 1H), 7.05 (d, *J* = 7.8 Hz, 1H), 6.94 (d, *J* = 7.8 Hz, 1H), 6.84 (s, 1H), 5.57 (s, 1H), 4.42 (s, 2H), 4.02 (s, 2H), 2.57 (q, *J* = 7.6 Hz, 2H), 1.20 (t, *J* = 7.6 Hz, 3H); ¹³C NMR (75 MHz, CDCl₃) δ 174.8, 162.4, 154.8, 140.8, 139.1, 136.94, 136.85, 131.2, 131.1, 130.4, 128.6, 128.1, 123.2, 123.1, 122.9, 90.8, 54.0, 53.1, 20.4, 11.6; R_{f} HPLC: 11.9 Min (13 Min from 10 to 95% MeCN in water (0.1 % formic acid), then 7 min 95% MeCN). 100.0 % purity; HRMS (MALDI): m/z found. 359.1615 [M+H]⁺ (cal. C₂₀H₁₉N₆O⁺ 359.1615).

**2-(5-(2-(1H--Tetrazol-5-yl)phenyl)isoindolin-2-yl)-5-ethyl-1,3,4-oxadiazole (Compound 32):** Synthesized according to GP6 from **Compound 128** (180 mg, 0.569 mmol) and tributyltin azide (934 µL, 3.41 mmol) in p-xylene (10.0 mL). Purification of the crude product by preparative HPLC yielded (142 mg, 69%) of **Compound 32.** ¹H NMR (400 MHz, DMSO-*d*₆) δ 7.70-7.67 (m, 2H), 7.60-7.53 (m, 2H), 7.29 (d, *J* = 7.9 Hz, 1H), 7.25 (s, 1H), 6.95 (d, *J* = 7.8 Hz, 1H), 4.76 (s, 2H), 4.72 (s, 2H), 2.72 (q, *J* = 7.6 Hz, 2H), 1.23 (t, *J* = 7.4 Hz, 3H); ¹³C NMR (100 MHz, DMSO-*d*₆) δ 162.1, 161.4, 141.2, 138.8, 136.7, 135.8, 131.0, 130.7, 130.5, 128.2, 127.8, 123.6, 123.3, 122.5, 53.7, 53.2, 18.4, 10.5; R_{f} HPLC: 10.1 Min (13 Min from 10 to 95% MeCN in water (0.1 % formic acid), then 7 min 95% MeCN). 100.0 % purity; HRMS (MALDI): m/z found. 360.1577 [M+H]⁺ (cal. C₁₉H₁₈N₇O⁺ 360.1567).

**2-(5-(2-(1H-Tetrazol-5-yl)phenyl)isoindolin-2-yl)-5-ethylthiazole (Compound 33):** Synthesized according to GP6 from **Compound 129** (70.0 mg, 0.211 mmol) and tributyltin azide (348 µL, 1.27 mmol) in *p*-xylene (6.0 mL). Purification of the crude product by preparative HPLC yielded (39.8 mg, 50%) of **33.** ¹H NMR (300 MHz, DMSO-*d*₆) δ 7.72-7.67 (m, 2H), 7.61-7.54 (m, 2H), 7.29 (d, *J* = 8.5 Hz, 1H), 7.24 (s, 1H), 6.94 (d, *J* = 7.9 Hz, 1H), 6.90 (s, 1H), 4.67 (s, 2H), 4.64 (s, 2H), 2.67 (q, *J* = 7.4 Hz, 2H), 1.19 (t, *J* = 7.4 Hz, 3H); ¹³C NMR (75 MHz, DMSO-*d*₆) δ 165.3, 155.1, 141.3, 138.7, 137.0, 136.1, 135.2, 131.1, 130.7, 30.6, 128.6, 128.1, 127.8, 123.5, 123.4, 122.6, 54.8, 54.7, 20.0, 15.9; R_{f} HPLC: 8.2 Min (13 Min from 10 to 95% MeCN in water (0.1 % formic acid), then 7 min 95% MeCN). 98.9 % purity; HRMS (MALDI): m/z found. 375.1397 [M+H]⁺ (cal. C₂₀H₁₉N₆S⁺ 375.1386).

**2-(5-(2-(1H-Tetrazol-5-yl)phenyl)isoindolin-2-yl)-4,5,6,7-tetrahydrobenzo[d]thiazole (Compound 34):** Synthesized according to GP6 from **Compound 130** (106 mg, 0.297 mmol) and tributyltin azide (490 µL, 1.79 mmol) in p-xylene (6.0 mL). Purification of the crude product by preparative HPLC yielded (39.9 mg, 34%) of **Compound** 34. ¹H NMR (300 MHz, DMSO-*d*₆) δ 7.72-7.67 (m, 2H), 7.61-7.54 (m, 2H), 7.29 (d, *J* = 7.9 Hz, 1H), 7.24 (s, 1H), 6.93 (d, *J* = 8.0, 1.5 Hz, 1H), 4.66 (s, 2H), 4.62 (s, 2H), 3.33 (br s, 4H), 1.75 (br s, 4H); ¹³C NMR (75 MHz, CDCl₃) δ 164.2, 146.9, 141.8, 139.2, 137.6, 136.7, 131.5, 131.2, 131.1, 128.6, 128.3, 124.1, 123.9, 123.1, 116.2, 55.3, 55.1, 27.0, 23.7, 23.2, 23.1; R_{f} HPLC: 8.1 Min (13 Min from 10 to 95% MeCN in water (0.1 % formic acid), then 7 min 95% MeCN). 98.8 % purity; HRMS (MALDI): m/z found. 401.1542 [M+H]⁺ (cal. C₂₂H₂₁N₆S⁺ 401.1543).

**2-(5-(2-(1H-Tetrazol-5-yl)phenyl)isoindolin-2-yl)-4-ethylthiazole (Compound 35):** Synthesized according to GP6 from **Compound 131** (99.3 mg, 0.30 mmol) and tributyltin azide (493 µL, 1.80 mmol) in p-xylene (6.0 mL). Purification of the crude product by preparative HPLC yielded (90.9 mg, 81%) of **Compound 35.** ¹H NMR (300 MHz, DMSO-*d*₆) δ 7.72-7.67 (m, 2H), 7.62-7.54 (m, 2H), 7.30 (d, *J* = 7.9 Hz, 1H), 7.24 (s, 1H), 6.94 (dd, *J* = 7.8, 1.5 Hz, 1H), 6.37 (s, 1H), 4.69 (s, 2H), 4.66 (s, 2H), 2.53 (qd, *J* = 7.5, 0.8 Hz, 2H), 1.18 (t, *J* = 7.5 Hz, 3H); ¹³C NMR (75 MHz, CDCl₃) δ 166.2, 155.4, 155.1, 141.3, 138.7, 137.1, 136.2, 131.1, 130.7, 130.6, 128.1, 127.8, 123.5, 123.4, 122.7, 100.2, 54.9, 54.7, 24.6, 13.0; R_{f} HPLC: 8.0 Min (13 Min from 10 to 95% MeCN in water (0.1 % formic acid), then 7 min 95% MeCN). 100.0 % purity; HRMS (MALDI): m/z found. 375.1386 [M+H]⁺ (cal. C₂₀H₁₉N₆S⁺ 375.1389).

**5-(2-(1H-Tetrazol-5-yl)phenyl)-2-(1-ethyl-1H-pyrazol-3-yl)isoindoline (Compound 36):** Synthesized according to GP6 from **Compound 132** (56.0 mg, 0.178 mmol) and tributyltin azide (293 µL, 1.07 mmol) in *p*-xylene (4.0 mL). Purification of the crude product by preparative HPLC yielded (24.2 mg, 38%) of **Compound 36.** ¹H NMR (300 MHz, DMSO-*d*₆) δ 7.79-7.55 (m, 4H), 7.49 (d, *J* = 2.2 Hz, 1H), 7.26 (d, *J* = 7.9 Hz, 1H), 7.16 (s, 1H), 6.91 (dd, *J* = 7.8, 1.5 Hz, 1H), 5.58 (d, *J* = 2.3 Hz, 1H), 4.49 (s, 2H), 4.45 (s, 2H), 3.96 (q, *J* = 7.2 Hz, 2H), 1.33 (t, *J* = 7.2 Hz, 3H); R_{f} HPLC: 10.4 Min (13 Min from 10 to 95% MeCN in water (0.1 % formic acid), then 7 min 95% MeCN). 97.1 % purity; HRMS (MALDI): m/z found. 358.1774 [M+H]⁺ (cal. C₂₀H₂₀N₇⁺ 358.1775).

### Synthesis of precursor compounds of Compounds 27 and 28:

1-Phenyl and 3-phenyl isoindoline derivatives **Compound 137** and **Compound 138**, precursors of **Compound 27** and **Compound 28,** were prepared by a different three-step synthetic route starting from 4-bromophtalimide as shown in **FIG. 4****.**

In the first step, 4-bromophtalimide reacted with phenyl magnesium bromide in presence of DMPU to give a mixture of separable isomers **Compound 133** (major) and **Compound 134** (minor). Next, the hydroxyl group was reduced by treatment with triethylsilane and trifluoroacetic acid. Afterwards, the missing carbonyl group was reduced by reaction of intermediates **Compound 135** and **Compound 136** with the BH₃.THF complex to yield the desired precursors **Compound 137** and **Compound 138.**

**5-Bromo-3-hydroxy-3-phenylisoindolin-1-one (Compound 133):** Phenylmagnesium bromide solution 1M in THF (49.2 mL, 49.2 mmol) was slowly added to a solution of 4-bromophtalimide (3.0 g, 13.3 mmol) in THF (15.0 mL) at 0°C under Ar. Then DMPU (1.62 mL, 13.4 mmol) was added and the reaction mixture was stirred at 0°C for 2h. The reaction was quenched by addition of saturated aqueous ammonium chloride solution. The aqueous phase was extracted with ethyl acetate (3x). The combined organic layers were washed with water and brine, dried over magnesium sulfate, filtered and evaporated. The crude product was purified by flash chromatography (hexane:EtOAc, 8:2 to 5:5) to yield 1.33 g (33%) of **Compound 133** and 794 mg (20%) of **Compound 134.** ¹H NMR (250 MHz, DMSO-*d*₆) δ 9.36 (s, 1H), 7.68 (dd, *J* = 8.0, 1.9 Hz, 1H), 7.59 (d, *J=* 8.0 Hz, 1H), 7.51-7.45 (m, 3H), 7.40-7.28 (m, 3H), 7.05 (s, 1H).

**6-Bromo-3-hydroxy-3-phenylisoindolin-1-one (Compound 134):** ¹H NMR (250 MHz, DMSO-*d*₆) δ 9.41 (s, 1H), 7.76 (d, *J* = 1.8 Hz, 1H), 7.71 (dd, *J* = 8.0, 1.8 Hz, 1H), 7.49-7.45 (m, 2H), 7.39-7.29 (m, 3H), 7.26 (d, *J* = 8.0 Hz, 1H), 7.02 (s, 1H)

**5-Bromo-3-phenylisoindolin-1-one (Compound 135):** Trifluoroacetic acid (2.30 mL, 30.7 mmol) was added dropwise to a solution **Compound 133** (1.33 g, 4.38 mmol) in dichloromethane (20.0 mL) at rt and the resulting solution was stirred for 5 min. Triethylsilane (1.41 mL, 8.77 mmol) was then added and the reaction mixture was stirred at rt overnight. Water was added and the aqueous phase was extracted with dichloromethane (3x). The combined organic layers were dried over magnesium sulfate, filtered and evaporated. The crude product was purified by flash chromatography (hexane:EtOAc, 6:4 to 0:10) to yield 870 mg (69%) of **135.** ¹H NMR (250 MHz, DMSO-*d*₆) δ 9.15 (s, 1H), 7.70-7.62 (m, 2H), 7.50-7.48 (m, 1H), 7.42-7.27 (m, 5H), 5.75 (s, 1H).

**6-Bromo-3-phenylisoindolin-1-one (Compound 136):** Trifluoroacetic acid (1.23 mL, 16.4 mmol) was added dropwise to a solution **Compound 134** (714 mg, 2.35 mmol) in dichloromethane (15.0 mL) at rt and the resulting solution was stirred for 5 min. Triethylsilane (757 µL, 4.70 mmol) was then added and the reaction mixture was stirred at rt overnight. Water was added and the aqueous phase was extracted with dichloromethane (3x). The combined organic layers were dried over magnesium sulfate, filtered and evaporated. The crude product was purified by flash chromatography (hexane:EtOAc, 7:3 to 2:8) to yield 523 mg (77%) of **Compound 136.** ¹H NMR (250 MHz, DMSO-*d*₆) δ 9.21 (s, 1H), 7.83 (d, *J* = 1.7 Hz, 1H), 7.72 (dd, *J* = 8.1, 1.9 Hz, 1H), 7.41-7.24 (m, 6H), 5.73 (s, 1H).

**6-Bromo-1-phenylisoindoline (Compound 137):** BH₃.THF complex solution 1M in THF (11.1 mL, 11.1 mmol) was added dropwise over 20 min to a solution of **Compound 135** (800 mg, 2.78 mmol) in THF (50.0 mL) at 0°C under Ar. The resulting solution was stirred at reflux temperature for 2 days. Ice water was added to quench the reaction and the solution was basified to pH>12 with aqueous 1M NaOH solution. The aqueous phase was extracted with ethyl acetate (3x). The combined organic layers were wdried over magnesium sulfate, filtered and evaporated. The crude product washed with brine, dried over magnesium sulfate and purified by flash chromatography (hexane:EtOAc, 5:5 to 3:7) to yield 90.0 mg (12%) of **Compound 137.** ¹H NMR (250 MHz, CDCl₃) δ 7.38-7.27 (m, 7H), 7.17 (d, *J* = 8.0 Hz, 1H), 7.11 (s, 1H), 5.42 (s, 1H), 4.40 (d, *J* = 14.3 Hz, 1H), 4.27 (d, *J* = 14.3 Hz, 1H).

**5-Bromo-1-phenylisoindoline (Compound 138):** BH₃.THF complex solution 1M in THF (7.25 mL, 7.25 mmol) was added dropwise over 20 min to a solution of **Compound 136** (522 mg, 1.81 mmol) in THF (35.0 mL) at 0°C under Ar. The resulting solution was stirred at reflux temperature for 2 days. Ice water was added to quench the reaction and the solution was basified to pH>12 with aqueous 1M NaOH solution. The aqueous phase was extracted with ethyl acetate (3x). The combined organic layers were dried over magnesium sulfate, filtered and evaporated. The crude product washed with brine, dried over magnesium sulfate and purified by flash chromatography (hexane:EtOAc, 5:5 to 3:7) to yield 26.1 mg (5%) of **Compound 138.** ¹H NMR (250 MHz, CDCl₃) δ 7.44 (s, 1H), 7.37-7.26 (m, 7H), 6.85 (d, *J* = 8.1 Hz, 1H), 5.39 (s, 1H), 4.43 (d, *J* = 14.0 Hz, 1H), 4.30 (d, *J* = 14.0 Hz, 1H).

### Example 2: IP-1 in-vitro assay showing the potency of the compounds of the present invention.

CHO-K1 cells (DSMZ, ACC 110) were maintained in Ham's F-12 medium (ThermoFisher) supplemented with 10% fetal bovine serum (FBS) and 100 units/ml Penicillin and 100 µg/ml Streptomycin (Gibco).

Stable cell lines that constitutively overexpress either BLT2 (uniprot Q9NPC1, corresponding to the sequence of BLT2 shown in Table 1) or BLT1 (uniprot Q15722-1 corresponding to the sequence of BLT1 shown in Table 2) were generated using the sleeping beauty method.⁴ In brief, the native *homo sapiens* (*h.s*.) cDNA for the respective GPCR was cloned into the plasmid vector pSBbi-Bla (addgene #60526) that procures resistance to Blasticidin. Stable insertion of the expression cassette into the host cell genome was facilitated by the transposase expressed from the co-transfected plasmid pCMV(CAT)T7-SB100 (addgene #34879). Expression of the respective GPCR is governed by the constitutively active EF1a promoter.

Accumulation of inositol monophosphate in response to activation of the Gq pathway can be enhanced by overexpression of a G alpha subunit (GNA) to which the respective GPCR efficiently couples to. This was utilized to increase the assay window of the IP-One assay. The native *h.s.* cDNA sequence of GNAQ, GNA11, GNA14, or GNA15/16 was cloned into pSBbi-GP (addgene #60511), respectively. The expression cassette that in addition procures resistance to Puromycin was introduced into the host cell genome as before. Cell lines stably transfected with only a GNA expression cassette were generated as control cell lines. The combinations of BLT1 (corresponding to the sequence of BLT1 shown in Table 2) + GNA15/16 and BLT2 (corresponding to the sequence of BLT2 shown in Table 1) + GNA14 were identified as the most responsive.

On target activity on BLT2 and off target activity on BLT1 was determined using a cell-based assay in which the accumulation of IP-1 (inositol monophosphate) as a downstream effect of BLT2 or BLT1 activation was detected in a displacement assay based on HTRF (Homogeneous Time-resolved FRET) between FRET acceptor coupled IP-1 and Terbium cryptate coupled anti-IP-1 antibody (IP-One assay kit, Cisbio Bioassays, Codolet, France).

CHO-K1 cells stably overexpressing BLT2 (corresponding to the sequence of BLT2 shown in Table 1) in combination with GNA14, cells stably overexpressing BLT1 (corresponding to the sequence of BLT1 shown in Table 2) with GNA15/16, or as control, cells overexpressing only one of the GNAs were seeded in full growth medium into white tissue culture 384-well plates (Greiner Bio-One, Frickenhausen, Germany) at 15.000 cells/well, respectively. After overnight incubation at 37°C and 5% CO₂ the medium was removed, cells were washed four times with stimulation buffer [146 mM NaCl, 4.2 mM KCl, 1 mM CaCl₂, 0.5 mM MgCl₂, 50 mM LiCl₂, 5.5 mM d-glucose, 0.1%_{(w/v)} fatty acid free bovine serum albumin fraction V (Carl Roth, Karlsruhe, Germany) buffered with 10 mM HEPES at pH 7.4 (NaOH)]. Thereafter, the synthesized end compounds and a total of 0.5% DMSO were added to the cells, the plate was sealed and incubated at 37 ° C for the stimulation period. After 90 minutes the cells were lysed by addition of the detection agents prepared in lysis buffer according to the manufacturer's instructions. The concentration of IP-1 produced by the cells was calculated from a standard curve using dilutions of unlabelled IP-1 in buffer without cells.

The compounds were compared for their potency and their EC₅₀ to the of reference agonist CAY. Relative efficacy was calculated as the activation with 20 µM of compound compared to the activation observed with 20 µM of reference agonist CAY set as 100%. The compounds of the invention were also tested for off-target activity on BLT1 using the same assay principle and LTB4 as reference agonist for BLT1. As the BLT2 agonists based on the spiro derivative **Compound 10** and the isoindoline derivative **Compound 11** showed promising results (see Table 3 and Table 4), these scaffolds were investigated in further studies. As a next set of compounds, a series of spiro derivatives (**Table 3**) and isoindoline was examined (**Table 4**).

Spiro derivatives: All of the compounds presented in **Table 3** showed a significant BLT2 activation. Regarding the second ring B² of the spirocycle, different tendencies were observed depending on the size of the ring B¹ connected to the aromatic. For a 4-member ring connected to the aromatic, a smaller ring was linked to improved EC50 values and efficacies (see **Compound 20** (4:4) **> Compound 19** (5:4) > **Compound 18** (6:4)). However, for a 5-member ring the opposite tendency in potency was observed **(Compound 13** (6:5) > **Compound 10** (5:5) > **Compound 16** (4:5)) although the differences in EC₅₀ were smaller in this case. All in all, several compounds of this family presented an EC₅₀ < 100 nM and especially **Compound 20** (EC₅₀ = 20.3 nM and 117% efficacy), proved to be even more active showing higher efficacy than CAY.

**Table 3. Spiro derivatives and EC₅₀ values for BLT2 activation.**

| **Compd.** | **BLT2 EC₅₀ ± SEM (nM)** | **Efficacy compared to CAY ± SEM** | **off-target test on BLT1** |
|---|---|---|---|
| **CAY** | 28.9 ± 5.70 | 100 % | Inactive |
| **10** | 94 | 65 % | Inactive |
| **13** | 85 | 65 % | Inactive |
| **16** | 106 | 61 % | Inactive |
| **18** | 103 | 47 % | Inactive |
| **19** | 63 | 56 % | Inactive |
| **20** | 20.3 ± 1.0 | 117 ± 13 % | Inactive |

Isoindoline derivatives: A breakthrough on the potency was achieved by the exchange of the carboxylic acid group by tetrazole (**Compound 29**). Therefore, this group was used for most of the next derivatives prepared. To continue with the structure activity relationship (SAR) study, the inventors further synthesized compounds bearing bioisosters of the amide function by the introduction of 5-member ring heterocycles. **Compound 31**, with an isoxazole ring, and **Compound 33** with a thiazole, showed comparable activity to **Compound 29**, while **Compound 32**, with an 1,3,4-oxadiazole, stood out as the most active compound of the series with a EC₅₀ = 12.6 nM.

**Table 4. Isoindolines and related derivatives and EC₅₀ values for BLT2 activation.**

| **Compd.** | **BLT2 EC₅₀ ± SEM (nM)** | **Efficacy compared toCAY ± SEM** | **off-target test on BLT1** |
|---|---|---|---|
| **CAY** | 28.9 ± 5.70 | 100 % | inactive |
| **11** | 638 ± 77.3 | 129 ± 9% | inactive |
| **24** | >100 | 104 % | not tested |
| **29** | 55.0 ± 17.1 | 153 ± 7% | inactive |
| **30** | >100 | 86 % | not tested |
| **31** | 59 ± | 176 % | inactive |
| **32** | 12.6 ± | 140 % | inactive |
| **33** | 68.2 ± | 126 % | inactive |

### Example 3: Receptor binding, aqueous solubility, metabolic stability and cellular toxicity

To achieve sufficient exposure upon oral administration of the compound, aqueous solubility and metabolic stability are two important factors for good absorption and low clearance of the drug. Solubility of the compounds in PBS buffer and in vitro metabolic half-life using rat liver microsomes were determined.

Aqueous solubility: Aqueous solubility was evaluated as described in Blöcher et al.⁵ Final concentrations of 25, 50, 75, 150, 200, 250, 333, 500, 750 and 1000 µM of **Compounds 1**, **13, 20, 32** and **33** were prepared in PBS pH 7.4 solution containing 1% DMSO, in a 96-well transparent flat bottom microtiterplate. Precipitation of the compound was measured at 600 nm and 800 nm after 1 and 24 h at room temperature using a microplate reader (Infinite M200, Tecan Group Ltd., Crailsheim, Germany) and solution clarity was confirmed by eye.

Metabolic Stability: The compounds were tested according to the following method: A solution of the respective compounds (final concentration 1 mM) was prepared in 100% DMSO. 432 µl phosphate buffer (0.1 M, pH 7.4) together with 50 µl NADPH-regenerating system (30 mM glucose-6-phosphate, 4 U/ml glucose-6-phosphate dehydrogenase, 10 mM NADP, 30 mM MgCl₂) and 5 µl of the corresponding test compound were pre-incubated at 37 °C. After 5 min the reaction was started by the addition of 13 µl microsome mix from the liver of Sprague-Dawley rats (Thermo Fisher Scientific, Darmstadt, Germany; 20 mg protein/ml in 0.1 M phosphate buffer). The incubation was performed in a shaking water bath at 37 °C. The reaction was stopped by the addition of 500 µl ice-cold methanol at 0, 30 and 60 min. The samples were centrifuged at 5,000 g for 5 min at 4 °C. The supernatants were analyzed and quantified by HPLC. Control samples were always performed to check the stability of the compounds in the reaction mixture. The first control was without NADPH, which is needed for the enzymatic activity of the microsomes. The second control was with inactivated (microsomes which had been incubated for 20 min at 90 °C). The third control was without test compounds (to determine the baseline). The amounts of the test compounds were quantified by an external calibration curve.

The results of the aqueous solubility and metabolic stability tests are shown in **Table 5.** Selected **Compound 13** and **Compound 20,** as a spiro derivatives examples, and **Compound 32** and **Compound 33** as isoindoline representatives were tested. **Compound 33** showed similar solubility as CAY and improved metabolic stability while **Compound 13**, **20** and **32** showed much improved solubility and stability than **Compound 1.**

**Table 5. Aqueous solubility and in vitro metabolic stability of selected compounds**

| **Cmpd.** | **Aq. Solubility^{a} (µM)** | **Rat liver microsomes^{b} (remaining after 60 min)** |
|---|---|---|
| **CAY** | 250-300 | 66 %^{c} |
| **13** | ≥ 1000 | 99.8 %^{c} |
| **20** | ≥ 1000 | 97.5 %^{c} |
| **32** | 750-1000 | 101 %^{c} |
| **33** | 250-300 | 89 %^{c} |

| | | |
|---|---|---|
| ^{a}Solubility of compound in PBS buffer, pH 7.4, containing 1% DMSO. ^{b}Microsome mix from the liver of Sprague-Dawley rats. ^{c}Percentage of remaining compound after 60 min | | |

### Example 4: In vivo activity of Compound 20

**Compound 20** showed the most promising combination of high in vitro potency and physicochemical properties. It was therefore selected for a pharmacokinetics (PK) study in male C57BL/6J mice following subcutaneous (sc) administration at 10 mg/Kg.

Plasma concentrations were measured at 15 min, 30 min, 1 h, 2 h, 4 h and 8 h. The resulting plasma concentration profile obtained is depicted in **FIG. 5****.** PK parameters are listed in **Table 6.**

**Table 6. PK Parameters of Compound 20**

| Cmpd | Cmax (ng/mL) | Tmax (h) | C_{final} (ng/mL) | AUC_{0→t-240min} (ng.min/mL) |
|---|---|---|---|---|
| **20** (10 mg/Kg *sc*) | 9117 | 15 min | 31 | 242000 |

Given the potential of BLT2 agonism to limit inflammation and promote healing responses, the impact of **Compound 20** on the inflammatory response in a self-resolving model of psoriasiform dermatitis was tested.

These *in two* experiments were performed using 8- to 10-week-old male and female C57BL/6J mice obtained from Charles River Laboratories (Wilmington, Ma, USA). Mice were housed based on a 12 h light/dark cycle with free access to food and water. The IMQ-induced psoriasis mouse model was performed and skin severity was assessed as described in van der Fits et al.⁶ Mouse back skin was shaved one day before starting the experiment. 62.5 mg commercially available cream containing 5% IMQ (Aldara; 3M Pharmaceuticals) was daily applied on the back skin of 8-10 weeks old male or female mice for 6 consecutive days. Skin severity was evaluated according to the PASI scoring system based on the extent of skin thickness, redness and scaling for up to 10 days. Additionally, the mice received s.c. injections of 10 mg/kg **Compound 32** or vehicle (PBS) on day 1, 3, and 5. All mouse experiments were approved by and followed the guidelines of the Hessian animal care and use committee (FU/2064).

To that end, mice were topically treated at their back skin with imiquimod (IMQ), the disease development was followed for up to 10 days. As expected, vehicle treated mice developed self-resolving skin inflammation, which peaked at day 4 (**FIG. 6**). S.c. application of **Compound 20** significantly reduced disease symptoms at the peak of the inflammatory response and showed a tendency for improved disease resolution (**FIG. 6**). These data indicate a clear potential for BLT2 agonism and the compounds of the invention to treat inflammatory skin disease.

### REFERENCES

The references are:
(1) Yokomizo, T.; Kato, K.; Terawaki, K.; Izumi, T.; Shimizu, T. A Second Leukotriene B4 Receptor, Blt2. J. Exp. Med. 2000, 192 (3), 421-432. https://doi.org/10.1084/jem.192.3.421.
(2) Iizuka, Y.; Yokomizo, T.; Terawaki, K.; Komine, M.; Tamaki, K.; Shimizu, T. Characterization of a Mouse Second Leukotriene B4 Receptor, MBLT2. J. Biol. Chem. 2005, 280 (26), 24816-24823. https://doi.org/10.1074/jbc.M413257200.
(3) Hernandez-Olmos, V.; Heering, J.; Bischoff-Kont, I.; Kaps, A.; Rajkumar, R.; Liu, T.; Fürst, R.; Steinhilber, D.; Proschak, E. Discovery of Irbesartan Derivatives as BLT2 Agonists by Virtual Screening. ACS Med. Chem. Lett. 2021, 12 (8), 1261-1266. https://doi.org/10.1021/acsmedchemlett.1c00240.
(4) Kowarz, E.; Löscher, D.; Marschalek, R. Optimized Sleeping Beauty Transposons Rapidly Generate Stable Transgenic Cell Lines. Biotechnol. J. 2015, 10 (4), 647-653. https://doi.org/10.1002/biot.201400821.
(5) Blöcher, R.; Lamers, C.; Wittmann, S. K.; Merk, D.; Hartmann, M.; Weizel, L.; Diehl, O.; Brüggerhoff, A.; Boβ, M.; Kaiser, A.; Schader, T.; Göbel, T.; Grundmann, M.; Angioni, C.; Heering, J.; Geisslinger, G.; Wurglics, M.; Kostenis, E.; Brüne, B.; Steinhilber, D.; Schubert-Zsilavecz, M.; Kahnt, A. S.; Proschak, E. N -Benzylbenzamides: A Novel Merged Scaffold for Orally Available Dual Soluble Epoxide Hydrolase/Peroxisome Proliferator-Activated Receptor γ Modulators. J. Med. Chem. 2016, 59 (1), 61-81. https://doi.org/10.1021/acs.jmedchem.5b01239.
(6) van der Fits, L.; Mourits, S.; Voerman, J. S. A.; Kant, M.; Boon, L.; Laman, J. D.; Cornelissen, F.; Mus, A.; Florencia, E.; Prens, E. P.; Lubberts E. Imiquimod-induced psoriasis-like skin inflammation in mice is mediated via the IL-23/IL-17 axis. J Immunol. 2009, 182 (9), 5836-45. doi: 10.4049/jimmunol.0802999.

## Claims

1. The compound of claim 1, wherein the compound comprises the structure (I-a), 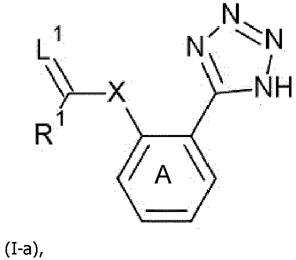
wherein the compound comprises a phenyl group A, wherein the phenyl group A is substituted with a tetrazole group and with a spiro group X,
wherein X comprises two aliphatic heterocycles B¹ and B² that are connected by a carbon spiro atom and each of B¹ and B² comprises one nitrogen heteroatom,
wherein the amount of ring atoms m¹ in B¹ is 6 or fewer,
wherein the amount of ring atoms m² in B² is 6 or fewer,
wherein B¹ is connected to A via the nitrogen heteroatom of B¹,
wherein B² is connected to an R¹(C=L¹) group via the nitrogen heteroatom of B²,
wherein R¹ is a Cₙ alkyl group with n = 1-5, a Cⱼ alkenyl group with j = 2-5, or a C_{d} alkyne group with d = 2-5, wherein optionally a hydrogen in R¹ is replaced with a fluor atom,
wherein L¹ is =O, =S, =C(CN)₂, =N-OH, or =N-OMe, preferably =O or =S,
or a tautomer, isomer, diastereomer, enantiomer, hydrate, mixture or salt thereof.

2. The compound of claim 1, wherein the compound comprises a formula (I-b) 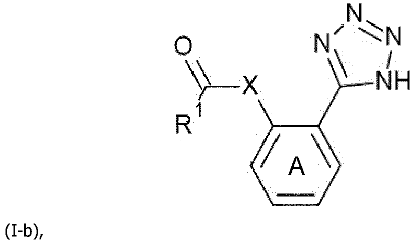
wherein the compound comprises a phenyl group A, wherein the phenyl group A is substituted with a tetrazole group and with a spiro group X,
wherein X comprises two aliphatic heterocycles B¹ and B² that are connected by a carbon spiro atom and each of B¹ and B² comprises one nitrogen heteroatom,
wherein B¹ is connected to A via the nitrogen heteroatom of B¹,
wherein the amount of ring atoms m¹ in B¹ is 6 or fewer,
wherein the amount of ring atoms m² in B² is 6 or fewer,
wherein B² is connected to an alkanoyl group via the nitrogen heteroatom of B², wherein the alkanoyl group comprises a R¹, wherein R¹ is a linear Cₙ alkyl group with n = 1-4,

3. The compound of any one of claims 1 or 2, wherein there is no nitrogen heteroatom next to the carbon spiro atom in the ring B¹ and in the ring B², and/or in the case that B¹ and B² each comprises an even number of "m¹" and "m²" ring atoms, the nitrogen heteroatom is at the position (m¹)/2 and (m²)/2 from the carbon spiro atom respectively.

4. The compound of any one of claims 1 to 3, wherein B¹ and B² comprises more than 3 ring atoms and/or wherein B¹ comprises fewer than 6 ring atoms.

5. The compound of any one of claims 1 to 4, wherein
(i) B¹ comprises fewer ring atoms than B², preferably wherein B¹ comprises 5 ring atoms; or
(ii) the number of ring atoms in B¹ is equal to the number or ring atoms in B², more preferably, wherein the number of ring atoms in B¹ and B² is 4.

6. The compound of any one of claims 1 to 5, wherein the spiro group X is selected from the group of 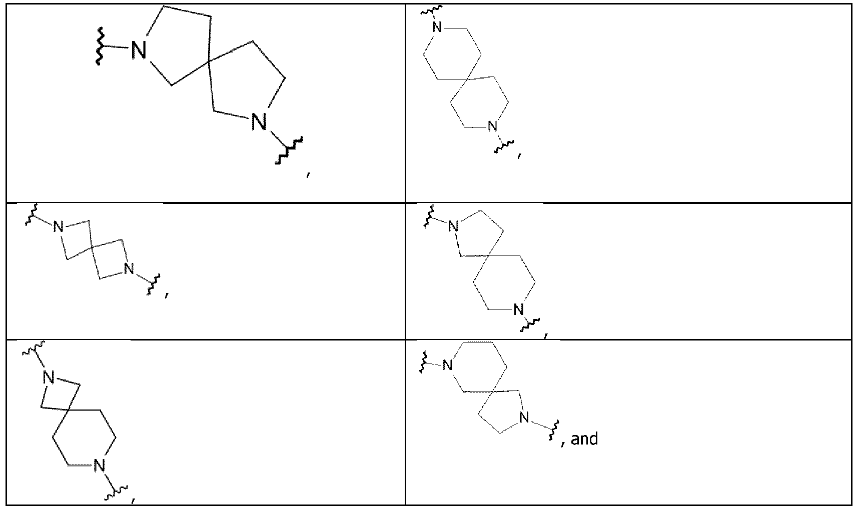 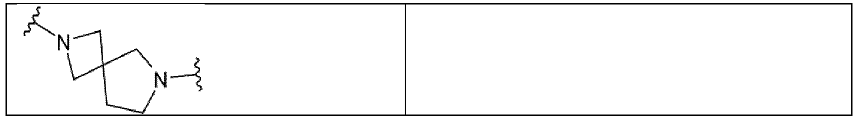 preferably, wherein the spiro group X is selected from the group of

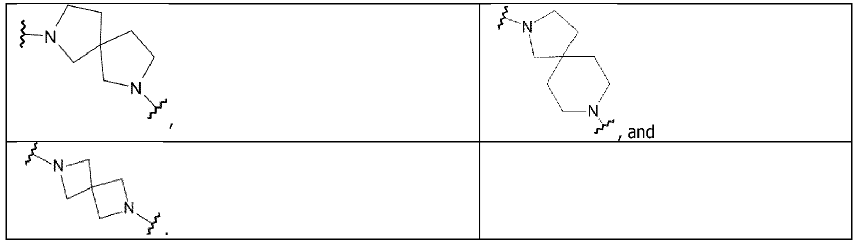

7. The compound of any one of claims 1 to 6, wherein the compound comprises a structure that is selected from the group of
| | |
|---|---|
| 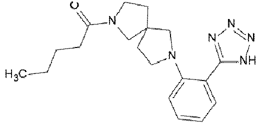 Compound 10: 1-(7-(2-(1H-Tetrazol-5-yl)phenyl)-2,7-diazaspiro[4.4]nonan-2-yl)pentan-1-one, | 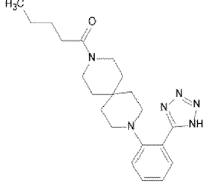 Compound 12: 1-(9-(2-(1H-Tetrazol-5-yl)phenyl)-3,9-diazaspiro[5.5]undecan-3-yl)pentan-1-one. |
| 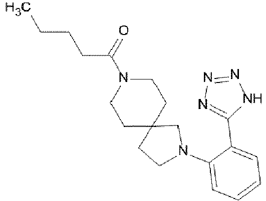 Compound 13: 1-(2-(2-(1H-Tetrazol-5-yl)phenyl)-2,8-diazaspiro[4.5]decan-8-yl)pentan-1-one, | 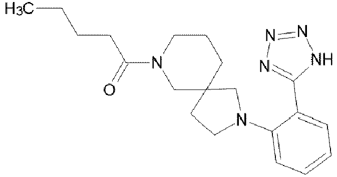 Compound 14: 1-(2-(2-(1H-Tetrazol-5-yl)phenyl)-2,7-diazaspiro[4.5]decan-7-yl)pentan-1-one, |
| 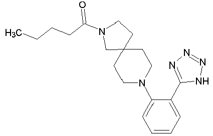 Compound 15: 1-(8-(2-(1H-Tetrazol-5-yl)phenyl)-2,8-diazaspiro[4.5]decan-2-yl)pentan-1-one. | 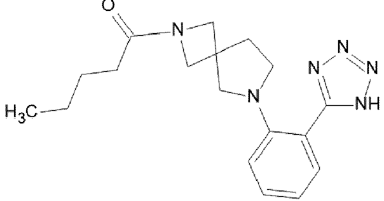 Compound 16: 1-(6-(2-(1H-Tetrazol-5-yl)phenyl)-2,6-diazaspiro[3.4]octan-2-yl)pentan-1-one, |
| 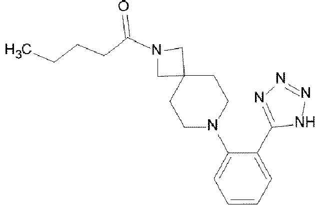 Compound 17: 1-(7-(2-(1H-Tetrazol-5-yl)phenyl)-2,7-diazaspiro[3.5]nonan-2-yl)pentan-1-one, | 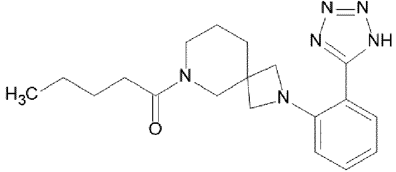 Compound 18: 1-(2-(2-(1H-Tetrazol-5-yl)phenyl)-2,7-diazaspiro[3.5]nonan-7-yl)pentan-1-one, |
| 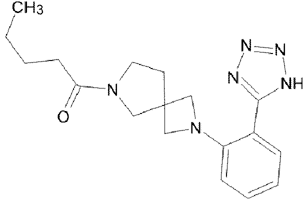 Compound 19: 1-(2-(2-(1H-Tetrazol-5-yl)phenyl)-2,6-diazaspiro[3.4]octan-6-yl)pentan-1-one, and | 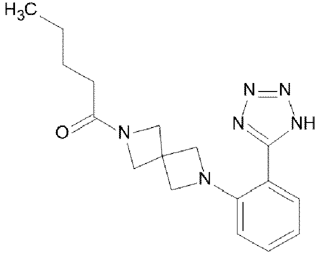 Compound 20: 1-(6-(2-(1H-Tetrazol-5-yl)phenyl)-2,6-diazaspiro[3.3]heptan-2-yl)pentan-1-one. |
preferably, wherein the compound comprises a structure selected from the group of
| | |
|---|---|
| 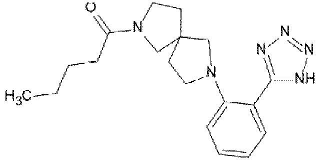 Compound 10: 1-(7-(2-(1H-Tetrazol-5-yl)phenyl)-2,7-diazaspiro[4.4]nonan-2-yl)pentan-1-one, | 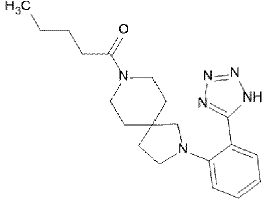 Compound 13: 1-(2-(2-(1H-Tetrazol-5-yl)phenyl)-2,8-diazaspiro[4.5]decan-8-yl)pentan-1-one, and |
| 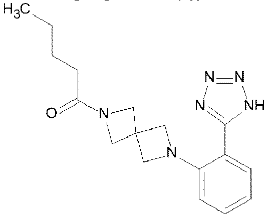 Compound 20: 1-(6-(2-(1H-Tetrazol-5-yl)phenyl)-2,6-diazaspiro[3.3]heptan-2-yl)pentan-1-one. | |

8. **A compound** that comprises a structure with the formula (II) 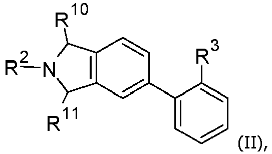
wherein R³ is a 1H-Tetrazol-5-yl group 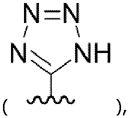 or a carboxyl group,
wherein R¹⁰ and R¹¹ are independently selected from -H, phenyl and a C₁₋₃ alkyl, preferably wherein at least one of R¹⁰ and R¹¹ is -H,
wherein R² is R⁴-(C=L²)-, wherein R⁴ is a Cₚ alkyl group with p = 1-5, a Cₒ alkenyl group with o = 2-5, or a Cₜ alkyne group with t = 2-5, wherein optionally R⁴ is substituted with a substituent selected from fluor and R⁵,
wherein R⁵ is a methyl group, ethyl group or phenyl group,
wherein L² is =O, =S, =C(CN)₂, =N-OH, =N-OMe, preferably =O or =S,
or wherein R² is a 5-membered heteroaryl with at least one nitrogen heteroatom, wherein the 5-membered heteroaryl with at least one nitrogen heteroatom comprises one substituent R⁶ and/or one substituent R⁷,
wherein R⁶ and R⁷ are independently selected from -H and a C_{q} alkyl-group with q = 1 to 3, or wherein R⁶ and R⁷ form a 6-membered ring together with the atoms that they are attached to that comprises at least 5 carbon ring atoms,
or a tautomer, isomer, diastereomer, enantiomer, hydrate, mixture or salt thereof.

9. The compound of claim 8, that comprises a structure with the formula (II) 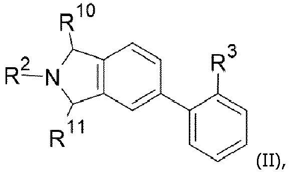
wherein R³ is a 1H-Tetrazol-5-yl group 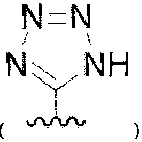 or a carboxyl group,
wherein R¹⁰ and R¹¹ are independently selected from -H, phenyl and methyl, wherein at least one of R¹⁰ and R¹¹ is -H,
wherein R² is R⁴-(C=O)-, wherein R⁴ is a linear Cₚ alkyl group with p = 1-4, optionally wherein R⁴ is substituted with a substituent selected from fluor and one R⁵,
wherein R⁵ is a methyl group, ethyl group or phenyl group,
or wherein R² is a 5-membered heteroaryl with at least one nitrogen heteroatom, wherein the 5-membered heteroaryl with at least one nitrogen heteroatom comprises one substituent R⁶ and/or one substituent R⁷,
wherein R⁶ and R⁷ are independently selected from -H and a C_{q} alkyl-group with q = 1 to 3.

10. The compound of claim 8 or 9, the compound comprises a structure with the formula (II) 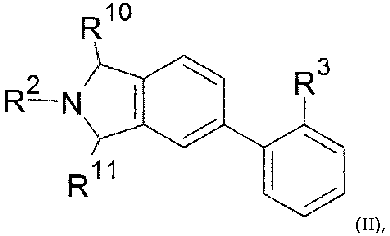
wherein R³ is a 1H-Tetrazol-5-yl group 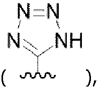
wherein R¹⁰ and R¹¹ are -H,
wherein R² is R⁴-(C=O)-, wherein R⁴ is a linear Cₚ alkyl group with p = 1-4, preferably p = 4
or wherein R² comprises the structure 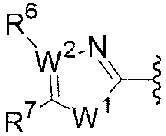
with W¹ = C, O, or S; and W² = C, N, or O, wherein
R⁶ and R⁷ is independently selected from -H and a C_{q} alkyl-group with q = 1 to 3, preferably q = 2.

11. The compound of any one of claims 8 to 10, wherein the compound comprises a structure selected from the group of:
| | |
|---|---|
| 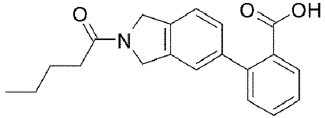 Compound 11: 2-(2-Pentanoylisoindolin-5-yl)benzoic acid, | 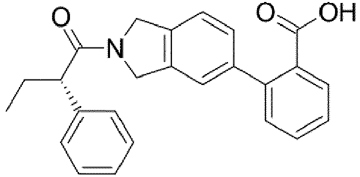 Compound 21: (S)-2-(2-(2-Phenylbutanoyl)isoindolin-5-yl)benzoic acid, |
| 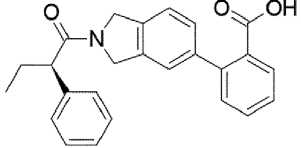 Compound 22: (R)-2-(2-(2-Phenylbutanoyl)isoindolin-5-yl)benzoic acid, | 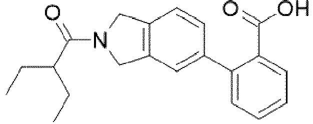 Compound 23: 2-(2-(2-Ethylbutanoyl)isoindolin-5-yl)benzoic acid, |
| 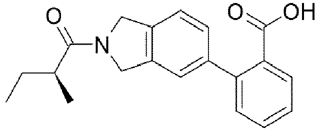 Compound 24: (S)-2-(2-(2-Methylbutanoyl)isoindolin-5-yl)benzoic acid, | 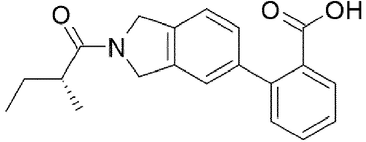 Compound 25: (R)-2-(2-(2-Methylbutanoyl)isoindolin-5-yl)benzoic acid, |
| 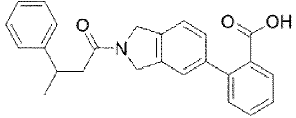 Compound 26: 2-(2-(3-Phenylbutanoyl)isoindolin-5-yl)benzoic acid, | 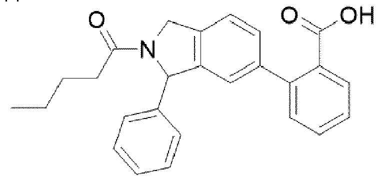 Compound 27: 2-(2-Pentanoyl-3-phenylisoindolin-5-yl)benzoic acid, |
| 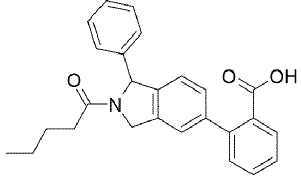 Compound 28: 2-(2-Pentanoyl-1-phenylisoindolin-5-yl)benzoic acid, | 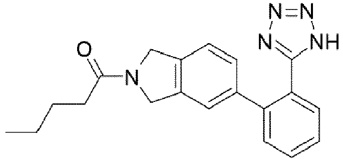 Compound 29: 1-(5-(2-(1H-Tetrazol-5-yl)phenyl)isoindolin-2-yl)pentan-1-one, |
| 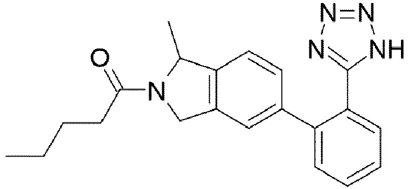 Compound 30: 1-(5-(2-(1H-Tetrazol-5-yl)phenyl)-1-methylisoindolin-2-yl)pentan-1-one, | 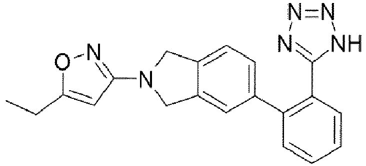 Compound 31: 3-(5-(2-(1H-Tetrazol-5-yl)phenyl)isoindolin-2-yl)-5-ethylisoxazole, |
| 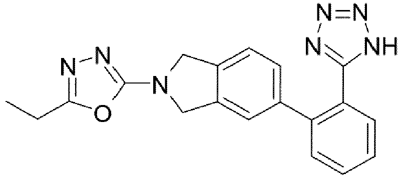 Compound 32: 2-(5-(2-(1H-Tetrazol-5-yl)phenyl)isoindolin-2-yl)-5-ethyl-1,3,4-oxadiazole, | 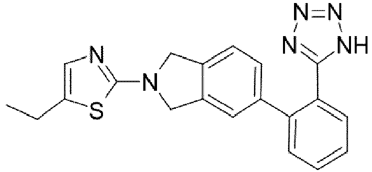 Compound 33: 2-(5-(2-(1H-Tetrazol-5-yl)phenyl)isoindolin-2-yl)-5-ethylthiazole, |
| 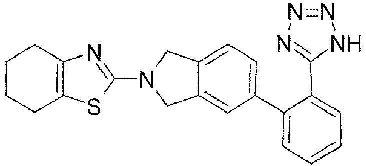 Compound 34: 2-(5-(2-(1H-Tetrazol-5-yl)phenyl)isoindolin-2-yl)-4,5,6,7-tetrahydrobenzor[d]thiazole, | 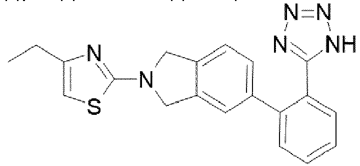 Compound 35: 2-(5-(2-(1H-Tetrazol-5-yl)phenyl)isoindolin-2-yl)-4-ethylthiazole, and |
| 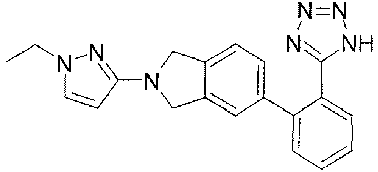 Compound 36: 5-(2-(1H-Tetrazol-5-yl)phenyl)-2-(1-ethyl-1H-pyrazol-3-yl)isoindoline. | |
preferably wherein the compound comprises a structure is selected from the group of
| | |
|---|---|
| 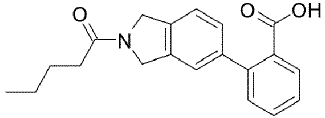 Compound 11: 2-(2-Pentanoylisoindolin-5-yl)benzoic acid, | 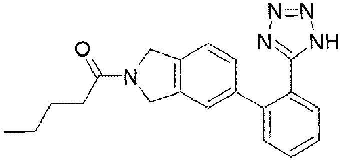 Compound 29: 1-(5-(2-(1H-Tetrazol-5-yl)phenyl)isoindolin-2-yl)pentan-1-one, |
| 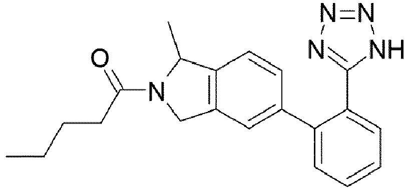 Compound 30: 1-(5-(2-(1H-Tetrazol-5-yl)phenyl)-1-methylisoindolin-2-yl)pentan-1-one, | 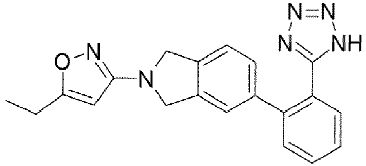 Compound 31: 3-(5-(2-(1H-Tetrazol-5-yl)phenyl)isoindolin-2-yl)-5-ethylisoxazole, |
| 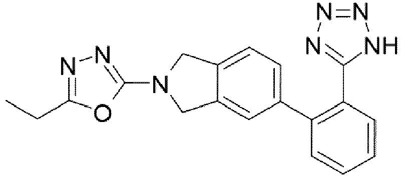 Compound 32: 2-(5-(2-(1H-Tetrazol-5-yl)phenyl)isoindolin-2-yl)-5-ethyl-1,3,4-oxadiazole, and | 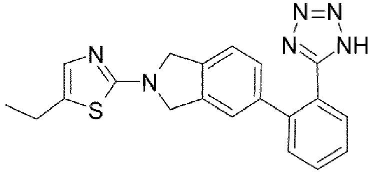 Compound 33: 2-(5-(2-(1H-Tetrazol-5-yl)phenyl)isoindolin-2-yl)-5-ethylthiazole. |
more preferably, wherein the compound is selected from the group of
| | |
|---|---|
| 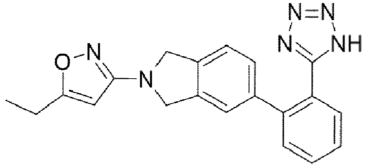 Compound 31: 3-(5-(2-(1H-Tetrazol-5-yl)phenyl)isoindolin-2-yl)-5-ethylisoxazole, | 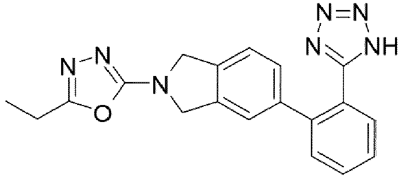 Compound 32: 2-(5-(2-(1H-Tetrazol-5-yl)phenyl)isoindolin-2-yl)-5-ethyl-1,3,4-oxadiazole, and |
| 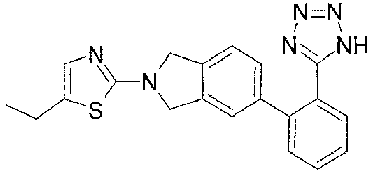 Compound 33: 2-(5-(2-(1H-Tetrazol-5-yl)phenyl)isoindolin-2-yl)-5-ethylthiazole. | |
most preferably, wherein the compound is
| |
|---|
| 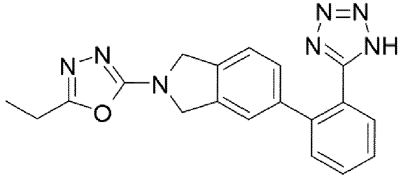 Compound 32: 2-(5-(2-(1H--Tetrazol-5-yl)phenyl)isoindolin-2-yl)-5-ethyl-1,3,4-oxadiazole. |

12. **A therapeutic composition** comprising a compound according to any one of claims 1 to 7 or 8 to 11 and a pharmaceutically acceptable carrier and/or excipient.

13. **A compound or therapeutic composition for use** in the treatment of a disease or condition in a subject, wherein the compound is a compound according to any one of claims 1 to 7 or 8 to 11 and wherein the therapeutic composition is the therapeutic composition according to claim 12.

14. The compound for use of claim 12, wherein the subject is a mammal, preferably a human.

15. The compound for use of claim 12 or 13, wherein the disease or condition is a disease associated with a reduced activity of Leukotriene B4 receptor 2 (BLT2), preferably wherein the condition of disease is a skin disease, an inflammatory disease, a tissue lesion, and/or pain, preferably wherein the disease is pain or a tissue lesion, more preferably wherein
i) the tissue lesion is a skin lesion or gastro-intestinal lesion.
ii) the pain is neuropathic pain such as a chemotherapy induced pain.
iii) the skin disease is an inflammatory skin disease such as psoriasis, atopic dermatitis, hidradenitis suppurativa, rosacea, vitiligo, acne, and/or exfoliative dermatitis.
